(19) Europäisches Patentamt
European Patent Office
Office européen des brevets

(11) EP 0 776 327 B1

(12) **EUROPEAN PATENT SPECIFICATION**

(45) Date of publication and mention of the grant of the patent:
**06.07.2005 Bulletin 2005/27**

(21) Application number: **95930217.5**

(22) Date of filing: **18.08.1995**

(51) Int Cl.[7]: **C07D 471/04**, C07D 471/10, C07D 471/14, C07D 471/20, C07D 471/22, C07D 487/04, C07D 487/10, C07D 487/14, C07D 487/20, C07D 487/22, C07D 211/60, C07D 401/14, C07D 401/12

(86) International application number:
**PCT/US1995/010559**

(87) International publication number:
**WO 1996/006097 (29.02.1996 Gazette 1996/10)**

(54) **NEW MULTIMERIZING AGENTS**

NEUES MULTIMERISIERENDES REAGENZ

NOUVEAUX AGENTS DE MULTIMERISATION

(84) Designated Contracting States:
**AT CH DE ES FR GB LI SE**

(30) Priority: **18.08.1994 US 292598**
**07.06.1995 US 479694**

(43) Date of publication of application:
**04.06.1997 Bulletin 1997/23**

(73) Proprietor: **ARIAD GENE THERAPEUTICS, INC.**
**Cambridge, MA 02139 (US)**

(72) Inventors:
- **HOLT, Dennis, A.**
  **Schwenksville, PA 19473 (US)**
- **LABORDE, Edgardo**
  **Foster City, CA 94404 (US)**
- **KEENAN, Terence**
  **Bay Shore, NY 11706 (US)**
- **GUO, Tao**
  **Dayton, NJ 08810 (US)**

(74) Representative: **Mercer, Christopher Paul**
**Carpmaels & Ransford**
**43, Bloomsbury Square**
**London WC1A 2RA (GB)**

(56) References cited:

**WO-A-94/18317** **WO-A-95/02684**
**US-A- 5 120 727** **US-A- 5 162 333**

- **SCIENCE, Volume 262, issued 21 November 1993, SPENCER et al., "Controlling Signal Transduction with Synthetic Ligands", pages 1019-1024.**
- **PEPTIDE CHEMISTRY, 1993, Volume 31, Number 1, issued 1994, UCHIYAMA et al., "Synthesis of Hybrid Type of Anti-HIV Drugs", pages 89-92.**
- **AMARA ET AL: 'A versatile synthetic dimerizer for the regulation of protein-protein interactions' PROC. NATL ACAD SCI vol. 94, September 1997, pages 10618 - 10623**
- **CLACKSON ET AL: 'Redesigning an FKBP-ligand interface to generate chemical dimerizers with novel specificity' PROC. NATL. ACAD. SCI. vol. 95, September 1998, pages 10437 - 10442**
- **KEENAN ET AL: 'synthesis and activity of bivalent FKBP12 ligands for the regulated dimeration of proteins' BIOORGANIC & MEDICINAL CHEMISTRY vol. 6, 1998, pages 1309 - 1332**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Background of the Invention

Aspects of the design, production and use of biological switches based on ligand-mediated multimerization of immunophilin-based recombinant proteins are disclosed in Spencer et al, 12 Nov 1993, Science **262**:1019-1024 and in International Patent Applications PCT/US94/01660 and PCT/US94/08008, the full contents of each of which are incorporated herein by reference. One class of multimerizing agents is based on dimers of the macrocyclic natural product, FK506, covalently attached to each other via a synthetic linker moiety. The resultant dimers ("FK1012" molecules) are characterized by high binding affinities for immunophilin molecules. However, they are large, complex molecules which can be inconvenient to produce. New methods and materials for multimerizing chimeric proteins containing immunophilin moieties would be desirable, where the methods and materials involve smaller, simpler multimerizing agents which retain a high binding affinity for their coordinate immunophilins, but which are more convenient to produce and are more readily amenable to structural modification.

Peptide Chemistry 1993, vol 31(1) pp. 89-92 discloses anti-HIV activity of protease inhibitors having a Pro (D)Phe derivative as a binding unity to gp 120.

### Description of the Invention

This invention provides a new method and materials for multimerizing immunophilins (including naturally occurring immunophilin proteins as well as chimeric proteins containing immunophilin-derived domains) based on N-oxalyl-pipecolyl and N-oxalyl-prolyl ligand moieties. ("Multimerization" as the term is used herein encompasses dimerization and higher order multimerization.)

The invention relates to immunophilin multimerizing agents of formula I,

$$M^1\text{-}L\text{-}M^2 \quad \textbf{(I)}$$

and pharmaceutically acceptable salts thereof, where $M^1$ and $M^2$ are independently selected from:

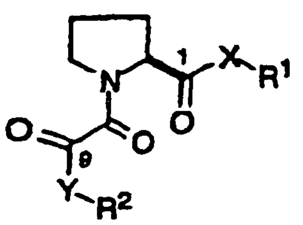

and

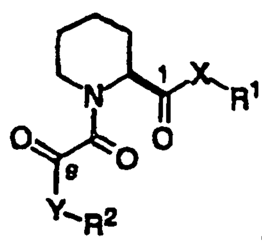

X = O, NH or $CH_2$;
Y = O, NH, $NR^3$, or represents a direct, i.e. covalent, bond from $R^2$ to atom 9;
$R^1$, $R^2$, and $R^3$ are independently $C_1$-$C_{20}$ alkyl or aryl;

wherein alkyl is intended to include both saturated and unsaturated straight chain, branched, cyclic, or polycyclic aliphatic hydrocarbons which may contain oxygen, sulfur, or nitrogen in place of one or more carbon atoms, and which are optionally substituted with one or more functional groups selected from the group consisting of hydroxy, $C_1$-$C_8$ alkoxy, acyloxy, carbamoyl, amino, N-acylamino, ketone, halogen, cyano, carboxyl, and aryl (unless otherwise specified, the alkyl, alkoxy and acyl groups preferably contain 1-6 contiguous aliphatic carbon atoms);

aryl is intended to include stable cyclic, heterocyclic, polycyclic, and polyheterocyclic unsaturated $C_3$-$C_{14}$ moieties, exemplified but not limited to phenyl, biphenyl, naphthyl, pyridyl, furyl, thiophenyl, imidazoyl, pyrimidinyl, and oxazoyl; which may further be substituted with one to five members selected from the group consisting of hydroxy, $C_1$-$C_8$ alkoxy, $C_1$-$C_8$ branched or straight-chain alkyl, acyloxy, carbamoyl, amino, N-acylamino, nitro, halogen, trifluoromethyl, cyano, and carboxyl (see e.g. Katritzky, Handbook of Heterocyclic Chemistry) ;

L is a linker moiety covalently linking monomers $M^1$ and $M^2$ through covalent bonds to either $R^1$ or $R^2$, not necessarily the same in each of $M^1$ and $M^2$; and wherein one of $M_1$ and $M_2$ may optionally be a macrocyclic structure

formed by a covalent link between $R^1$ and $R^2$ provided that said multimerizing agent does not have the formula:

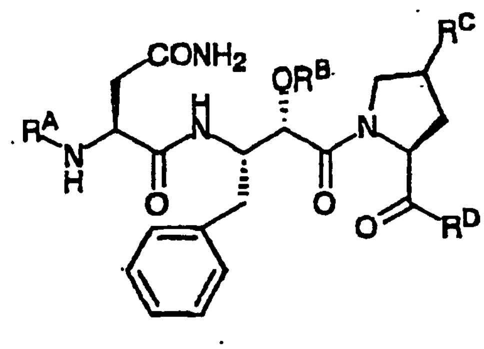

where $R^A$ is carbobenzyloxy, $R^B$ and $R^C$ are each MeOCOCO-Pro-(D)Phe-, and $R^D$ is $-NHC(CH_3)_3$.

Linker moieties (L), need not contain essential elements for binding to the immunophilin proteins, and may be selected from a very broad range of structural types. Preferred moieties include $C_2$-$C_{20}$ alkyl, aryl, or dialkylaryl structures where alkyl and aryl are defined as above. Linker moieties may be conveniently joined to monomers $M^1$ and $M^2$ through functional groups such as ethers, amides, ureas, carbamates, and esters; or through alkyl-alkyl, alkyl-aryl, or aryl-aryl carbon-carbon bonds. Furthermore, linker moieties may be optimized (e.g., by modification of chain length and/or substituents) to enhance pharmacokinetic properties of the formula I multimerizing agent.

In one subset of compounds of this invention, $-YR^2$ is a straight, branched or cyclic aliphatic moiety of 1 to 8 carbon atoms, including for example ethyl, isopropyl, tert-butyl, tert-amyl, and cyclohexyl, and $-XR^1$ is a moiety of the formula

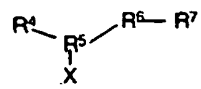

where $R^4$ is an aromatic or heteroaromatic group, e.g. phenyl, substituted phenyl, mdolyl, pyridyl, etc.; $R^5$ is a straight, branched or cyclic aliphatic moiety of 2 to 8 carbon atoms, which may be optionally substituted, including ethyl, propyl, butyl, pentyl moieties and the like; $R^6$ is an aromatic or heteroaromatic moiety bearing a reactive functional group, $R^7$, permitting covalent attachment to a linker moiety. $R^7$ may be -COOH, -CHO, -XH or $XR^8$, where X is O, S or NH (which may bear an optional substituent such as an alkyl group of 1 - 8 carbon atoms) and $R^8$ is $-(CH_2)_z$-COOH where z is an integer from 1 through 4.

The following formulae provide three exemplary (and non-exclusive) classes of compounds of this invention:

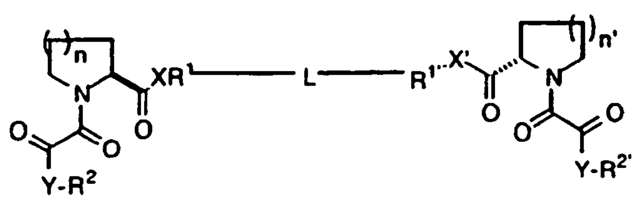

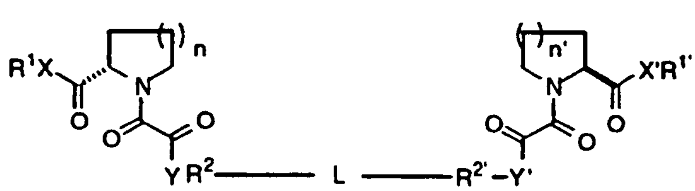

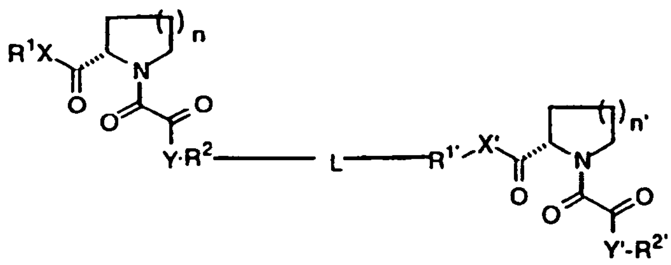

The multimerizing agents of this invention preferably cannot participate in a ternary complex with both immunophilin and calcineurin, or with immunophilin and FRAP (Brown et al., *Nature,* **1994**, *369*, 756-758) , and are therefore not immunosuppressive like FK506 or rapamycin. Additionally, it will often be preferred that the multimerizing agent be physiologically acceptable (i.e., lack undue toxicity toward the cell or organism with which it is to be used), can be taken orally by animals (i.e., is orally active in applications in whole animals, including gene therapy), and/or can cross cellular and other membranes, as necessary for a particular application.

The multimerizing agents can be used as described in PCT/US94/01617 and PCT/US94/08008, e.g. to activate the transcription of a desired gene, actuate apoptosis, or trigger other biological events in engineered cells growing in culture or in whole organisms, including in gene therapy applications. The engineered cells contain and are capable of expressing DNAs encoding proteins containing one or more immunophilin domains, such as an FKBP domain, which are capable of binding to the monomers, M (formula II), or to multimerizing agents comprising such monomers such as depicted in formulas II and in the many examples disclosed herein. In such applications, the multimenzing agent is administered to the cell culture or to the organism containing the cells, as the case may be, in an amount effective to multimerize the proteins containing the corresponding ligand-binding domains (as may be observed by monitoring the transcription, apoptosis or other biological process so triggered). In the case of administration to whole organisms, the multimerizing agent may be administered in a composition containing the multimerizing agent and acceptable veterinary or pharmaceutical diluents and/or excipients. Monomers disclosed herein are also useful, both in the synthesis of dimerizing agents as disclosed in detail herein, and in their own right in view of their binding affinity for immunophilins or modified immunophilins. They may be administered to the engineered cells, or to organisms containing them (preferably in a composition as described above in the case of administration to whole animals), in an amount effective for reversing or blocking the effect of the multimerizing agent, i.e. for preventing, inhibiting or disrupting multimerization.

Compounds of this invention may be prepared by adaptation of known methods for the synthesis of N-oxalyl-pipecolyl, N-oxalyl-prolyl and related monomers. See e.g. Holt, *et al., J. Amer. Chem. Soc.,***1993**, *115*, 9925-9938; Holt, *et al., Biomed. Chem. Lett.,* **1993**, *4*, 315-320; *Luengo, et al., Biomed. Chem. Lett*., **1993,** *4,* 321-324; Yamashita, *et al*., *Biomed. Chem. Lett*., **1993**, *4*, 325-328; Spencer et al, above; PCT/US94/01617; and PCT/US94/08008. See also EP 0 455 427 A1; EP 0 465 426 A1; US 5,023,263 and WO 92/00278.

For example, monomers may be assembled and dimerized via a number of synthetic schemes and in various orders as illustrated in the following reaction schemes.

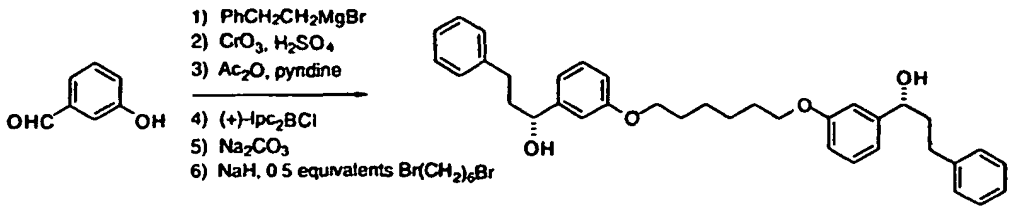


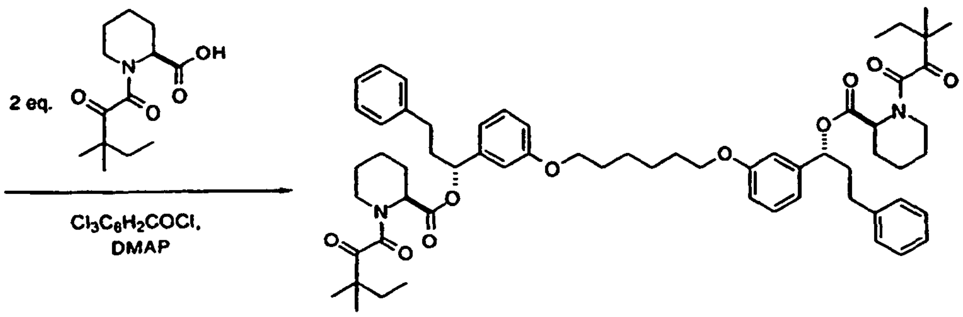


see: Holt, *et al. J. Amer. Chem., Soc,* **1993,** *115*, 9925-9938.

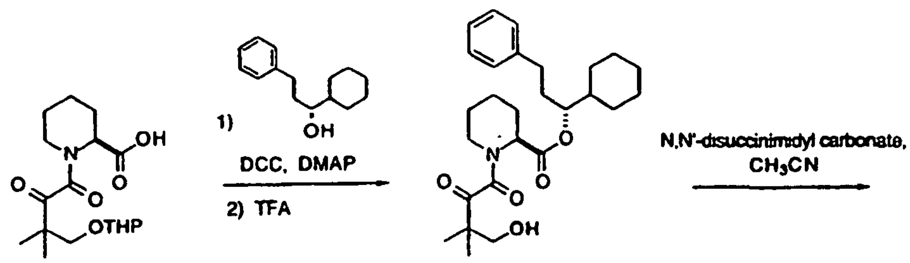


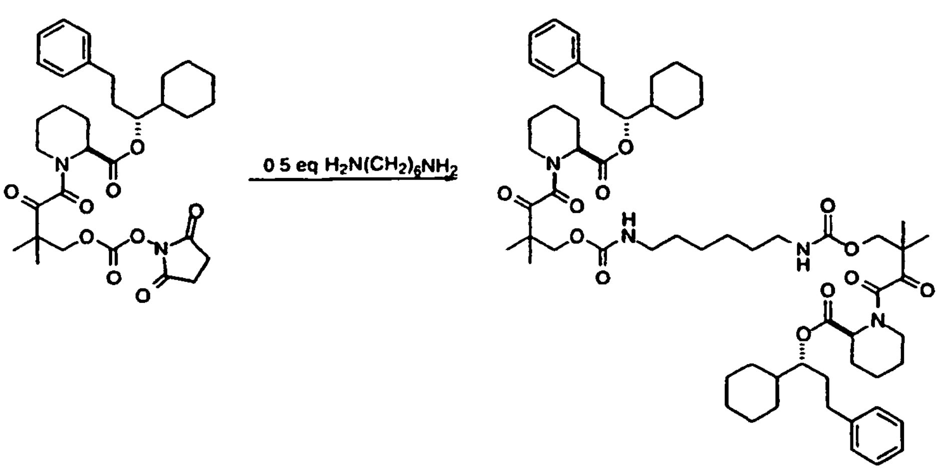


see: Holt, *et al. J. Amer. Chem. Soc.*, **1993**, *115*, 9925-9938.

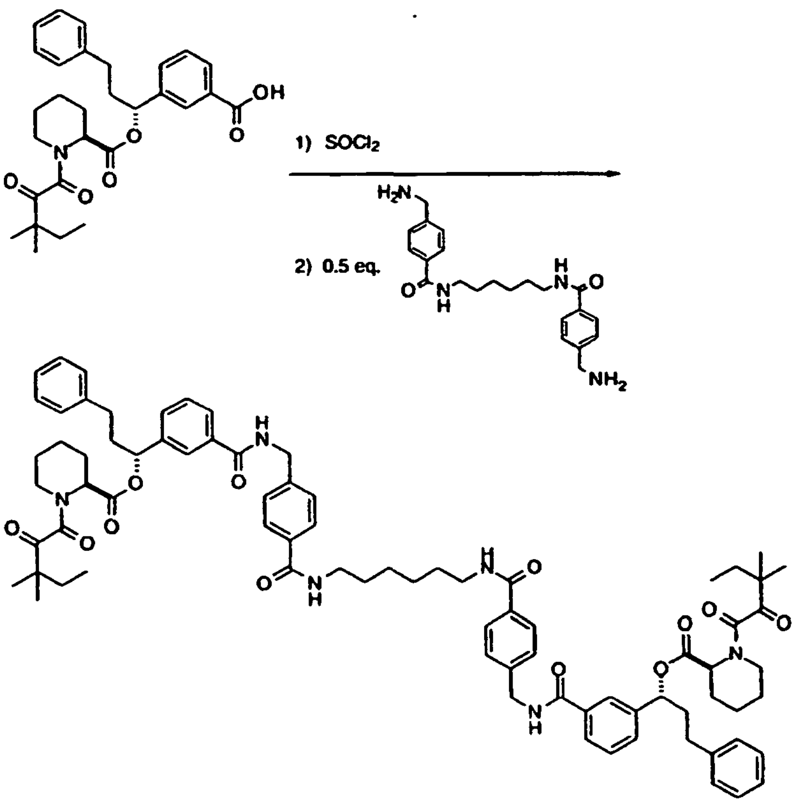


see: Yamashita, et al. *Biomed. Chem. Lett.*, **1993**, *4*, 325-328.

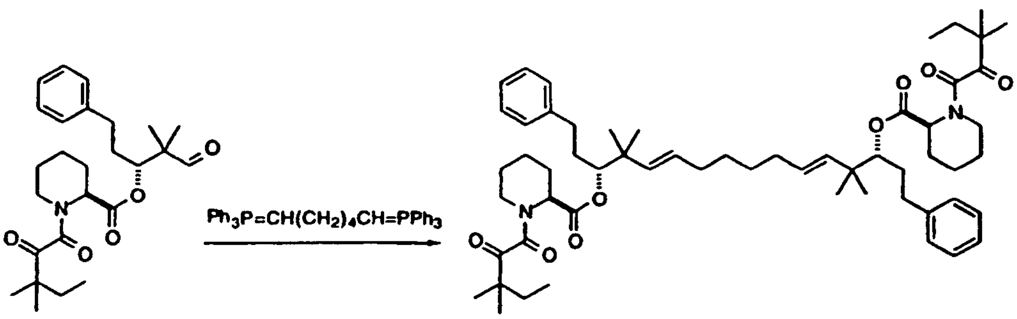


see: Yamashita, et al. *Biomed. Chem. Lett.*, **1993,** *4*, 325-328.

Bis-Wittig reagents are well known in the literature. See e.g., Paquette, *et al., J. Amer. Chem. Soc.*, **1985,** *107*, 6598; Nicolaides, *Synthesis,* **1977,** 127.

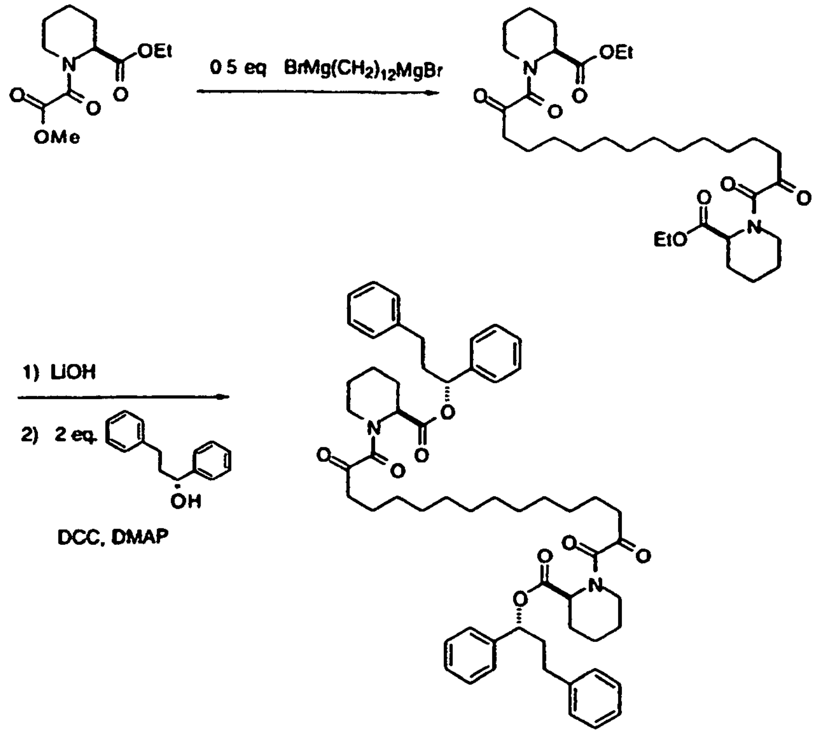


see: Holt, *et al. J. Amer. Chem. Soc.,* **1993,** *115*, 9925-9938.

Bis-Grignard reagents are also well known in the literature. See e.g., Babudri, *et al. J. Orgmet. Chem.,* **1991**, *405, 53-58;* and Fujisawa *et al. Bull. Chem. Soc. Jpn.,* **1983**, *56*, 345.

Heterodimers (e.g., where $M^1 \neq M^2$) may be prepared by stepwise attachment of each monomer to the linker. Attachment methods may be different for each monomer and the linker may be non-symmetrical and/or differentially functionalized to facilitate stepwise attachment of monomers. By way of example, the following reaction schemes illustrate formation of heterodimers.

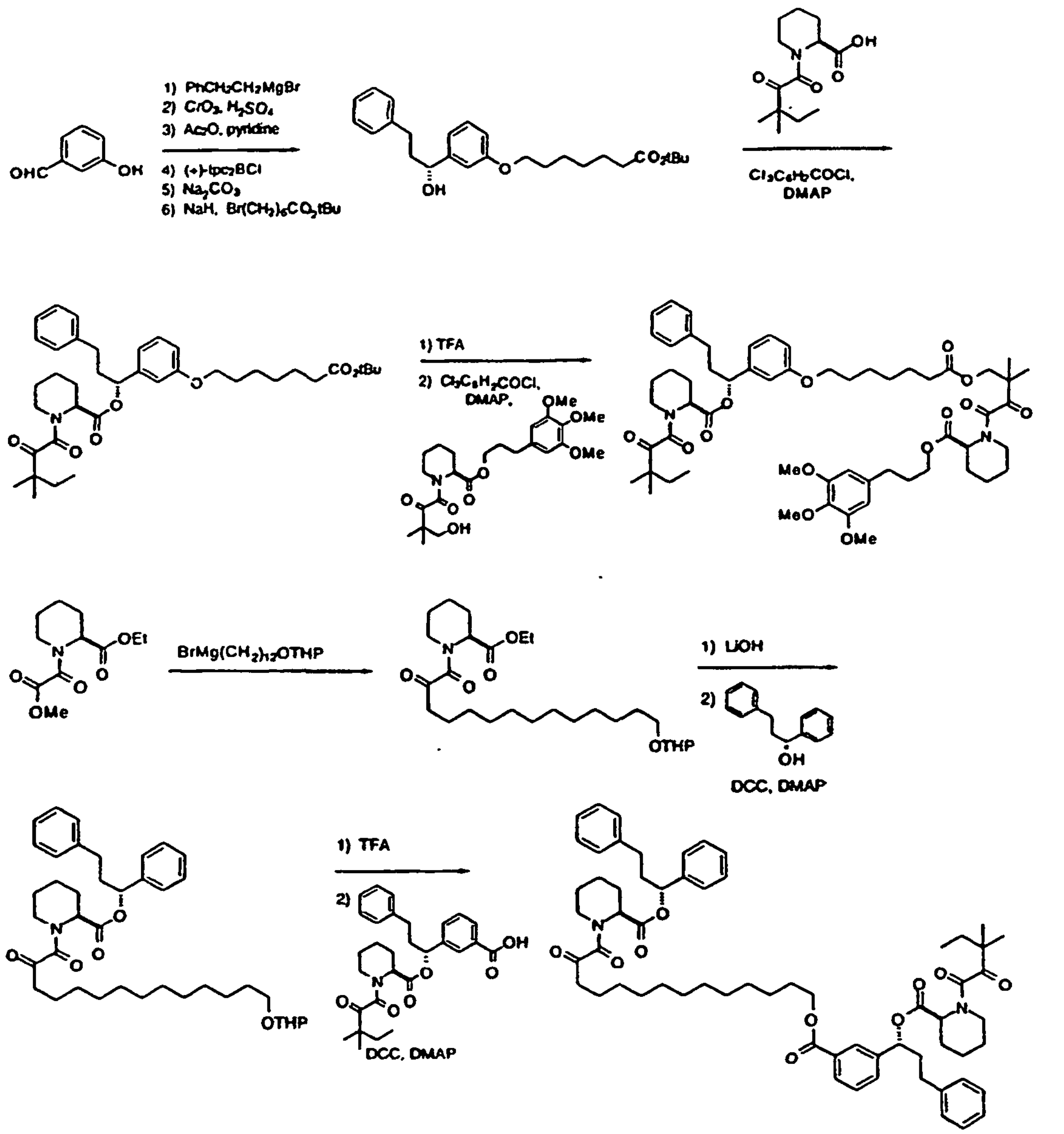


This invention also includes compounds as defined in the claims containing structural elements or substituents which enhance binding to mutant FKBPs relative to indigenous FKBPs. Preferably, such compounds bind to mutant FKBPs at least an order of magnitude better than they bind to FKBP12, and in some cases binding to mutant FKBPs will be greater than 3 orders of magnitude better than to FKBP12. For example, such enhancements in binding to mutant FKBPs can be achieved by compounds containing a carboxylic acid or an amine where the mutant FKBP contains a complementary group (histidine/arginine or aspartate/glutamate) in an appropriate location to form a salt bridge between the compound and the mutant FKBP.

Binding affinities of various multimerizing agents of this invention or their component monomers with respect to FKBP or other immunophilin proteins may be determined by adaptation of conventional methods used in the case of FKBP. For instance, the practitioner may measure the ability of a compound of this invention to compete with the binding of a known ligand to the protein of interest. See e.g. Sierkierka et al, 1989, Nature 341, 755-757 (test compound competes with binding of labeled FK506 derivative to FKBP).

The ability of the multimerizing agents to multimerize chimeric proteins may be measured in cell-based assays by measuring the occurrence of an event triggered by such multimerization. For instance, one may use cells containing and capable of expressing DNAs encoding chimeric proteins compnsing one or more immunophilin-derived ligand binding domains and one or more effector domains capable, upon multimenzation, of actuating a biological response. We prefer to use cells which further contain a reporter gene under the transcriptional control of a regulatory element (i.e., promoter) which is responsive to the multimerization of the chimeric proteins. The design and preparation of

illustrative components and their use in so engineering cells is described in PCT/US94/01617. The cells are grown or maintained in culture. A multimerizing agent is added to the culture medium and the presence of the reporter gene product is measured. Positive results, i.e., multimenzation, correlates with transcription of the reporter gene as observed by the appearance of the reporter gene product.

## Examples

### <u>Synthetic Overview, part I:</u>

The synthesis of functionalized chiral alcohols was carried out as follows. The unsubstituted chiral alcohol 1 was prepared from 3-hydroxybenzaldehyde in five steps following reported procedures by Holt <u>et al</u>. *J. Amer. Chem. Soc.,* **1993,** *115*, 9925-9938.

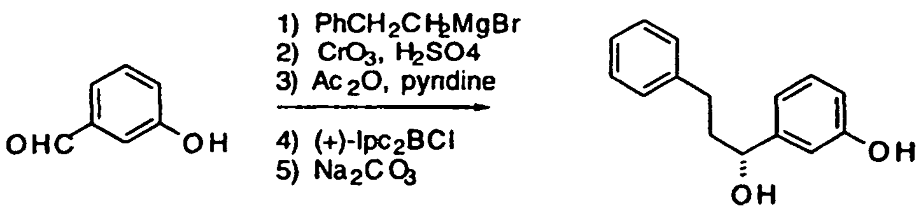


**1**

Alkylation of **1** with 3-*N*-Boc-aminopropylbromide in the presence of one equivalent of NaH gave **2** in good yield. Similarly, alkylation of **1** with *tert*-butyl bromoacetate provided **3.**

**1**

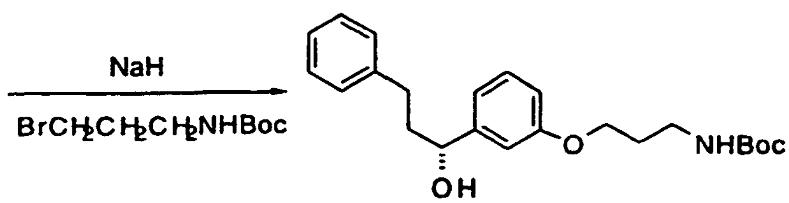


**2**

**1**

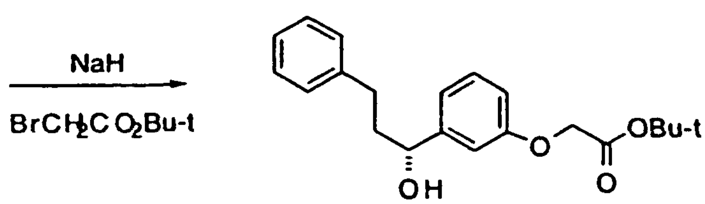


**3**

Chiral alcohols containing left-phenyl ring substitutions were prepared using a chalcone chemistry as shown in the following scheme.

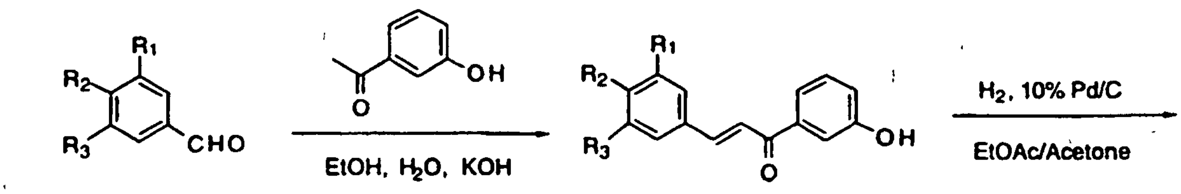


4: $R_1 = R_2 = OCH_3$, $R_3 = H$
5: $R_1 = R_2 = R_3 = OCH_3$
6: $R_1 = R_2 = OCH_2O$, $R_3 = H$

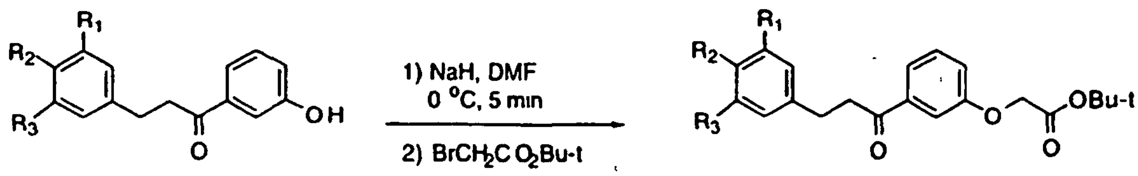


7: $R_1 = R_2 = OCH_3$, $R_3 = H$
8. $R_1 = R_2 = R_3 = OCH_3$
9: $R_1 = R_2 = OCH_2O$, $R_3 = H$

10 $R_1 = R_2 = OCH_3$, $R_3 = H$
11 $R_1 = R_2 = R_3 = OCH_3$
12. $R_1 = R_2 = OCH_2O$, $R_3 = H$

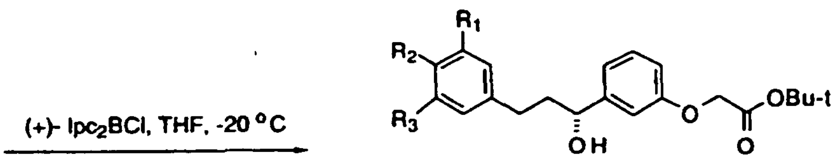


13: $R_1 = R_2 = OCH_3$, $R_3 = H$
14 $R_1 = R_2 = R_3 = OCH_3$
15. $R_1 = R_2 = OCH_2O$, $R_3 = H$

Pyridine and indole containing chiral alcohols were prepared using a similar chalcone chemistry but with some minor modifications as shown below:

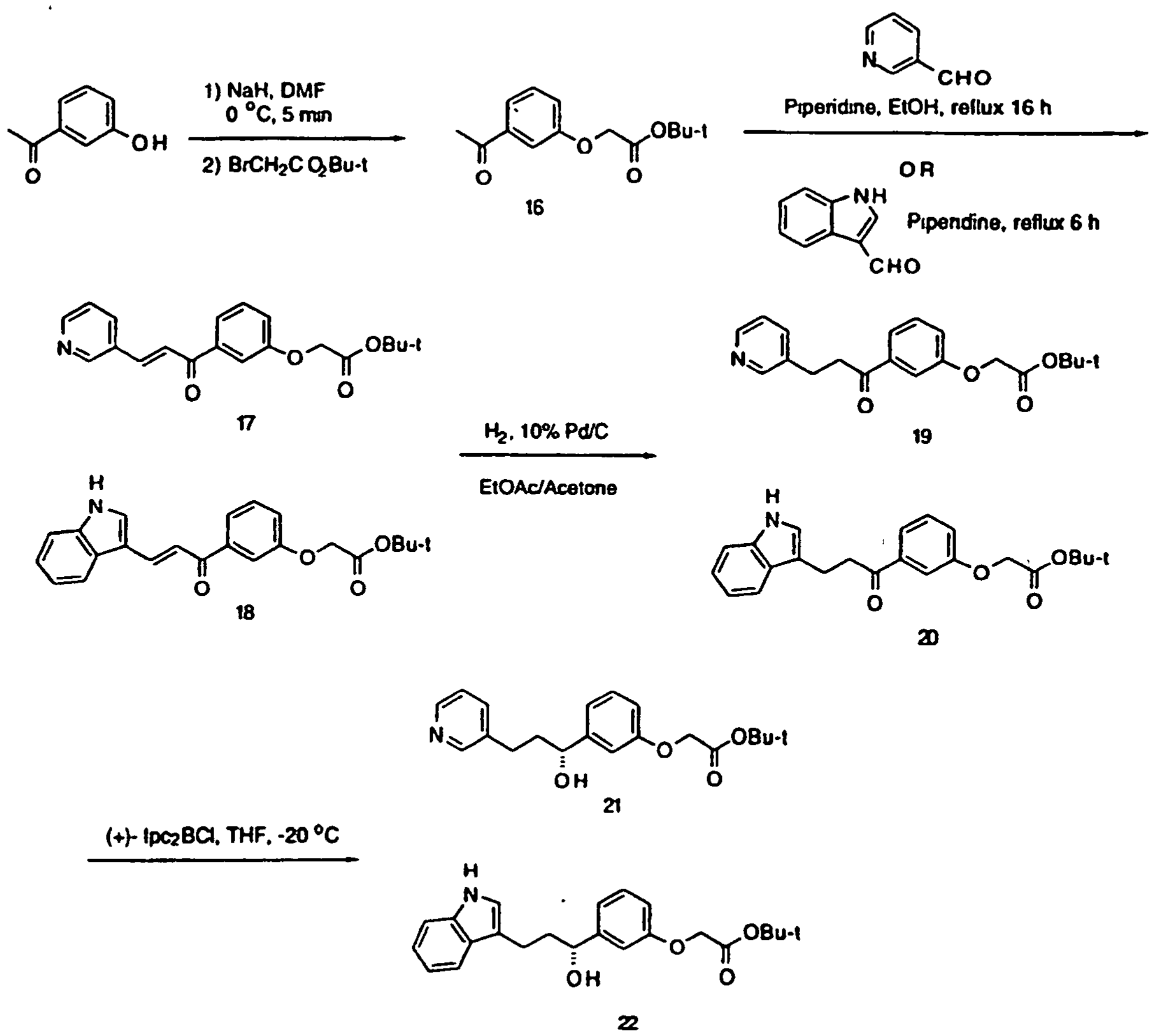


16 17 18 19 20 21 22

The carboxylic acid **23** was prepared from L-pipecolic acid in four steps following literature procedures by Holt et al. *J. Amer. Chem. Soc.*, **1993**, *115*, 9925-9938.

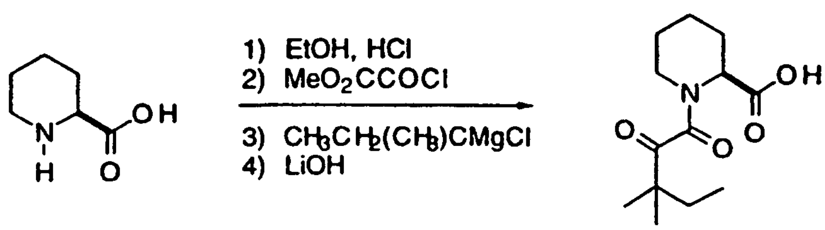


23

Coupling of **23** with **2** using DCC/DMAP and then removal of Boc-group with trifluoroacetic acid give the amine monomer **24** in good yield. The carboxylic acid monomers **25-30** were produced in a similar fashion.

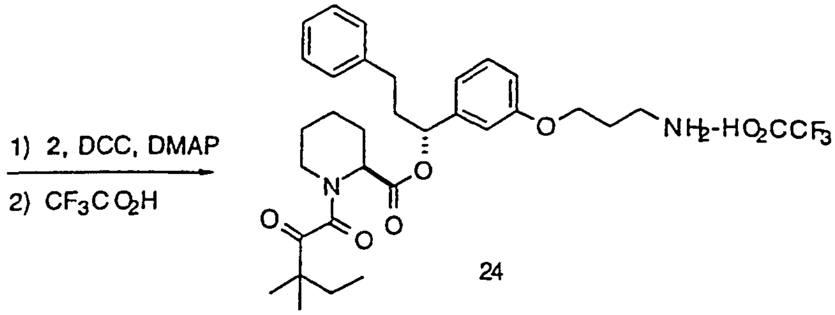


24

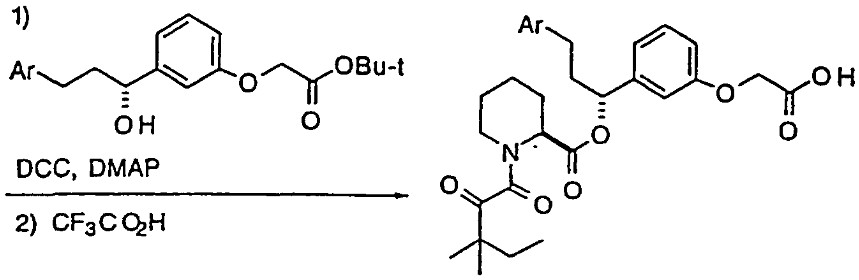


25: Ar = Ph
26: Ar = 3,4,-$(OCH_3)_2$Ph
27: Ar = 3,4,5-$(OCH_3)_3$Ph
28: Ar = 3,4-$(OCH_2O)$Ph
29: Ar = 3-pyridyl
30: Ar = 3-indolyl

With monomers **24** and **25-30** in hand, various dimers were then synthesized. The amine **24** was treated with disuccinimidyl dicarboxylates to produce dimers **31-34** and **37**, and **38**. Reaction of **24** with benzene-1,3-disulfonyl chloride yielded **36**. Coupling of **24** with triethylene glycol bis(chloroformate) yielded **39.** Treatment of compound 34 with methyl iodide afforded **35** in quantitative yield.

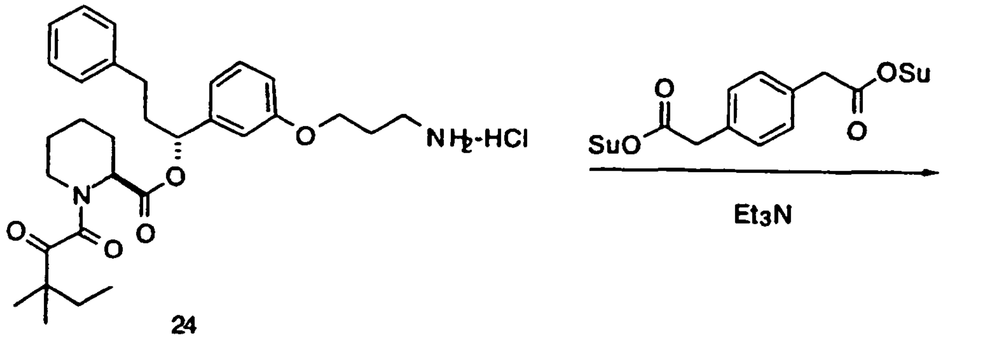


24

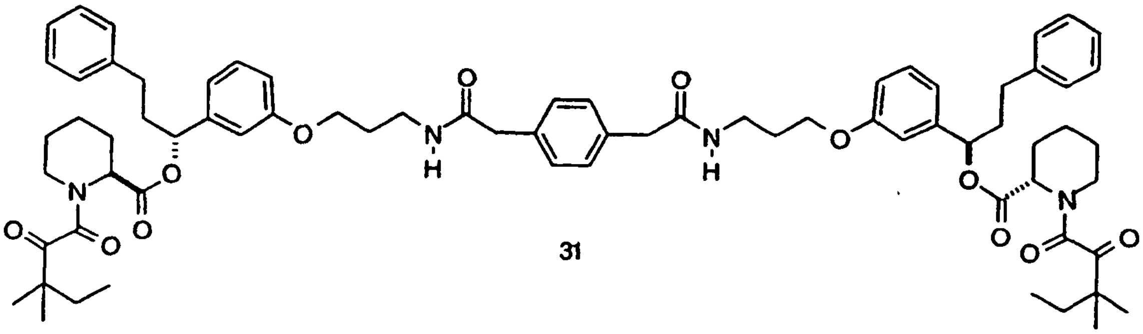

31

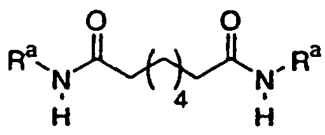

32

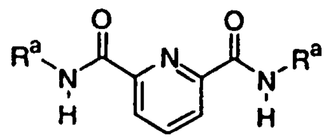

33

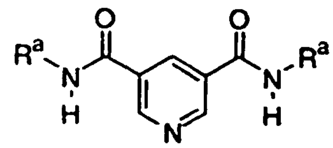

34

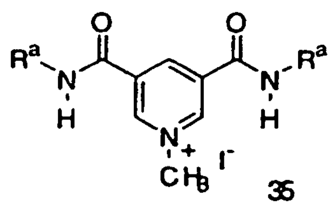

35

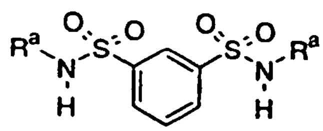

36

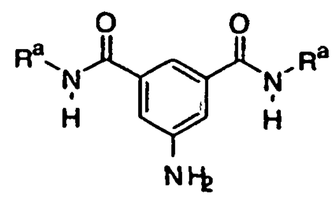

37

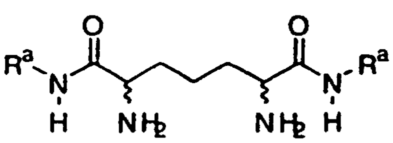

38

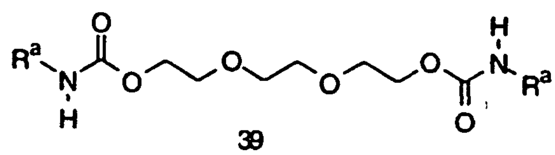

39

The acids **25-30** were converted to their activated succinimidyl esters and then coupled with various diamines to give dimers **40-63.** ($R^a$ and $R^b$ groups represent the vanous monomers, M).

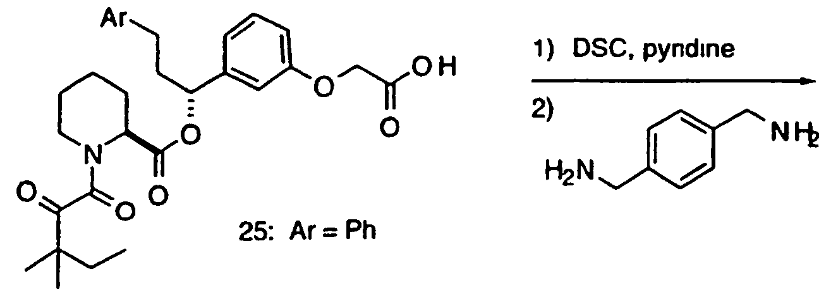


25: Ar = Ph

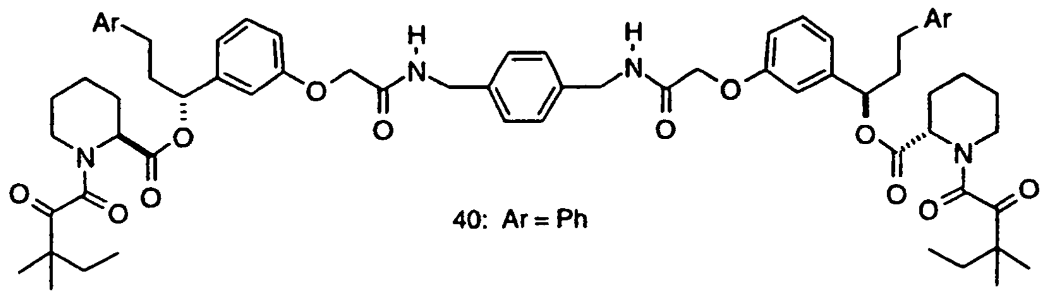

40: Ar = Ph

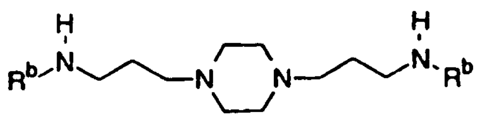

41: Ar = Ph

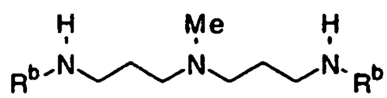

42: Ar = Ph

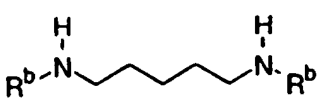

43: Ar = Ph
44: Ar = 3,4-$(OCH_3)_2$Ph
45: Ar = 3,4,5-$(OCH_3)_3$Ph
46: Ar = 3,4-$(OCH_2O)$Ph
47: Ar = 3-pyridyl

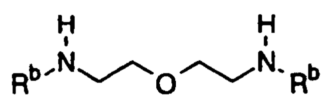

48: Ar = Ph
49: Ar = 3,4-$(OCH_3)_2$Ph
50: Ar = 3,4,5-$(OCH_3)_3$Ph
51: Ar = 3,4-$(OCH_2O)$Ph
52: Ar = 3-pyridyl

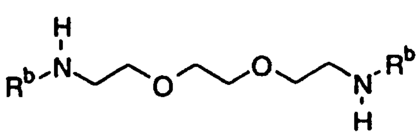

53: Ar = Ph
54: Ar = 3,4-$(OCH_3)_2$Ph
55: Ar = 3,4,5-$(OCH_3)_3$Ph
56: Ar = 3,4-$(OCH_2O)$Ph
57: Ar = 3-pyridyl
58: Ar = 3-indolyl

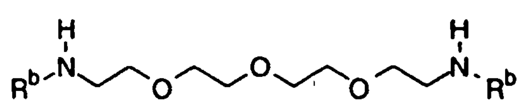

59: Ar = Ph
60: Ar = 3,4-$(OCH_3)_2$Ph
61: Ar = 3,4,5-$(OCH_3)_3$Ph
62: Ar = 3,4-$(OCH_2O)$Ph
63: Ar = 3-pyridyl

Compounds 64-67, based on the parent structure of 40 but containing the specified linkers and Ar moieties,

were made by adaptation of methods described herein. The structure of the four compounds was confirmed by NMR and MS spectroscopy. All four were found to be active in cell-based transcription assays such as described infra.

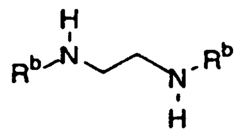

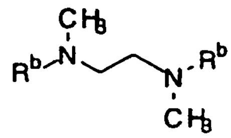

**64: Ar = 3,4-$(OCH_3)_2$Ph**
**65: Ar = 3-pyridyl**

**66: Ar = 3,4-$(OCH_3)_2$Ph**
**67: Ar = 3-pyridyl**

## Synthetic Details

### General Methods

Proton and carbon magnetic resonance spectra ($^1$H, $^{13}$C NMR) were recorded on Bruker ARX-300 spectrometer. Chemical shifts are reported in parts per million (δ) relative to $Me_4Si$ (δ 0.0). All reagents were analytical grade and were used as received. Anhydrous solvents were purchased from Aldrich in sure-seal bottles. Chromatography refers to short column chromatography using TLC grade silica gel 60 G (Merck) and the indicated solvents as the mobile phase. HPLC was conducted using a 4.6 mm x 250 mm Daicel *Chiracel OD* column and (unless otherwise noted) a mobile phase of 85:15 hexane-propanol, flow rate of 1 mL/min, and UV detection at 210 nm. Melting points are uncorrected.

### Preparation of Functionalized Chiral Alcohols

(1*R*)-3-Phenyl-1-(3-(3-*tert*-butyloxycarbamylpropyl)oxyphenyl)propan-1-ol (**2**)

(1*R*)-3-Phenyl-1-(3-hydroxyphenyl)propan-1-ol (**1**, 98% ee, 1.47 g, 6.45 mmol, prepared in five steps from 3-hydroxybenzaldehyde following reported procedures by Holt et al. *J. Amer. Chem. Soc.*, **1993,** *115*, 9925-9938) was added to a suspension of NaH (60% dispersion in mineral oil, 310 mg, 7.74 mmol) in DMF (30 mL). 3-*tert*-Butyloxycarbamylpropyl bromide (3.07 g, 12.9 mmol) was then added and the resulting mixture was stirred at 40 °C under $N_2$ overnight. The reaction was quenched with water (50 mL) and the mixture was extracted with EtOAc (250 mL). The organic layer was washed with saturated brine, dried ($Na_2SO_4$), and concentrated *in vacuo*. The mixture was redissolved in $Et_2O$ (150 mL) and washed with 2 N NaOH (2 x 100 mL) to remove any unreacted **1** (which has the same Rf as the product **2**). The organic layer was then washed with saturated brine, dried ($Na_2SO_4$), and concentrated *in vacuo*. Chromatography (silica gel, 30% EtOAc/hexanes) afforded 2 (1.9 g, 77% yield, 96% ee by Chiracel HPLC: retention time 19.0 min for the (1*R*)-enantiomer and 15.7 min for the (1*S*)-enantiomer) as a colorless oil: $^1$H NMR ($CDCl_3$, 300 MHz) 7.40-6.85 (m, 9 H), 4.76 (t, J = 5.3 Hz, 1 H), 4.12 (t, J = 5.9 Hz, 2 H), 3.42 (t, J = 6.3 Hz, 2 H), 2.80 (m, 2 H), 2.10-1.85 (m, 6 H), 1.53 (s, 9 H), ; $^{13}$C NMR ($CDCl_3$, 75MHz) 159.4, 156.4, 146.8, 142.1, 129.9, 128.83, 128.78, 126.2, 118.8, 114.0, 112.4, 74.2, 66.1, 40.8, 32.4, 30.0, 28.8. MS(FAB): $(M+Na)^+$ 408.

(1*R*)-3-Phenyl-1-(3-(2-*tert*-butyloxy-2-oxoethyl)oxyphenyl)propan-1-ol (**3**)

(1*R*)-3-Phenyl-1-(3-hydroxyphenyl)propan-1-ol (1, 98% ee, 1.7 g, 7.46 mmol, prepared in five steps from 3-hydroxybenzaldehyde following reported procedures by Holt et al. *J. Amer. Chem. Soc.*, **1993**, *115*, 9925-9938) was added to a suspension of NaH (60% dispersion in mineral oil, 358 mg, 8.95 mmol) in DMF (50 mL). *tert*-Butyl bromoacetate (2.4 mL, 14.9 mmol) was then added and the resulting mixture was stirred at 40 °C under $N_2$ overnight. The reaction was quenched with water (50 mL) and the mixture was extracted with EtOAc (250 mL). The organic layer was washed with saturated brine, dried ($Na_2SO_4$), and concentrated *in vacuo*. Chromatography (silica gel, 20% EtOAc/hexanes) afforded 3 (1.64 g, 64% yield, 98% ee by Chiracel HPLC: retention time 42.2 min for the (1*R*)-enantiomer and 30.6 min for the (1*S*)-enantiomer) as a colorless oil: $^1$H NMR ($CDCl_3$, 300 MHz) 7.22-6.71 (m, 9 H), 4.58 (t, 1 H), 4.44 (s, 2 H), 2.68-2.59 (m, 2 H), 2.05-1.93 (m, 2 H), 1.41 (s, 9 H); $^{13}$C NMR ($CDCl_3$, 75MHz) 168.4, 158.6, 146.8, 142.1, 130.0, 128.8, 128.7, 126.2, 119.5, 114.1, 112.6, 82.7, 74.1, 66.1, 40.8, 32.4, 28.4. HRMS(FAB): $(M+Na)^+$ calcd 365.1729, found 365.1721.

3,4- Dimethoxy-3'- hydroxy chalcone (**4**)

A solution of 3,4-Dimethoxybenzaldehyde (16.6 g, 100 mmol) in EtOH (75 mL) was treated with 3-Hydroxyacetaphenone (13.6 g, 100 mmol) and the resulting solution cooled to 0 °C in an ice bath. A 200 mL solution of aqueous KOH (28 g, 500 mmol) was added slowly and the resulting bright red solution was allowed to stir overnight (16 h) at room temperature. The mixture was then acidified to pH 5 by the dropwise addition of concentrated HCl and the resulting suspension extracted with EtOAc (2 x 200 mL). The combined organic extract was washed with a saturated NaCl solution (2 x 100 mL), dried over $MgSO_4$, filtered, evaporated, and flash chromatographed (silica gel, 30% → 50% EtOAc/hexanes) to give crude material. The crude solid was crystallized from EtOAc to afford 13.9 g (49%) of a yellow colored solids: IR (neat) 3420, 1650, 1575, 1510, 1265, 1140 $cm^{-1}$; $^1H$ NMR ($CDCl_3$, 300 MHz) 7.80 (d, J = 15.6 Hz, 1H), 7.68 (s, 1H), 7.59, (d, J= 7.7 Hz, 1H), 7.42-7.36 (m, 2H), 7.24 (dd, J = 8.3, 1.8 Hz, 1H), 7.16-7.13 (m, 2H), 6.90 (d, J = 8.3 Hz, 1H), 6.82 (s, 1H), 3.95 (s, 3H), 3.94 (s, 3H); $^{13}C$ NMR ($CDCl_3$, 75 MHz) 191.3. 157.0, 152.0, 149.7, 146.1, 140.1, 130.2, 128.2, 123.8, 121.2, 120.7, 120.3, 115.7, 111.6, 110.7, 56.4.

3,4,5- Trimethoxy-3'- hydroxy chalcone (**5**)

Prepared in a similar manner as (**4**) from 3,4,5- trimethoxybenzaldehyde. Flash chromatography (silica gel, 30% → 50% EtOAc/hexanes) afforded 2.61 g (17%) of yellow colored solids: $^1H$ NMR ($CDCl_3$, 300 MHz) 9.80 (s, 1H), 7.82 (d, J = 15.6 Hz, 1H), 7.70-7.63 (m, 2H), 7.48 (s, 1H), 7.39 (app t, J = 7.9 Hz, 1H) 7.23 (s, 2H) 7.08 (d, J = 7.6 Hz, 1H), 3.87 (s, 6H), 3.73 (s, 3H); $^{13}C$ NMR ($CDCl_3$, 75 MHz) 189.5, 158.1, 153.5, 144.7, 140.1, 139.5, 130.6, 130.1, 121.8, 120.5, 119.9, 115.0, 106.9, 60.5, 56.5.

3'- Hydroxy- 3,4-methylenedioxy chalcone (**6**)

Prepared in a similar manner as (**4**) from piperonal. Crude solids (26.7 g, 100%) were carried on directly to the next reaction step without chromatographic purification or characterization.

3-(3,4- Dimethoxyphenyl)-1- (3-hydroxyphenyl)propan-1-one (**7**)

A solution of 3,4- Dimethoxy-3'- hydroxy chalcone (**4**) (10 g, 35.2 mmol) in a 1:1 mixture of EtOAc:Acetone (40mL) was treated with 10% Pd on Carbon (500 mg) and the mixture hydrogenated at 40-50 psi pressure of $H_2$ for 3 h. The reaction mixture was filtered through a pad of Celite with the aid of acetone and the filtrate concentrated to afford a crude solid. The crude solid was triturated with EtOAc and filtered to afford 7.83 g (78%) of white solids which proved to be of -90% purity by $^1H$ NMR analysis: $^1H$ NMR ($CDCl_3$, 300 MHz) 7.56 (s, 1H), 7.55, (d, J= 2.2 Hz, 1H), 7.53-7.33 (m, 1H), 7.10 (dd, J = 7.9, 2.4 Hz, 1H), 6.80-7.79 (m, 3H), 6.61 (s, 1H), 3.86 (s, 3H), 3.86 (s, 3H), 3.28 (t, J = 7.9 Hz, 2H), 3.02 (t, J = 7.9 Hz, 2H); $^{13}C$ NMR ($CDCl_3$, 75 MHz) 200.6, 156.9, 149.3, 147.8, 138.6, 134.2, 130.3, 121.1, 120.6, 115.0, 112.4, 111.8, 56.3, 41.2, 30.3.

1-(3-Hydroxyphenyl)-3- (3,4,5-trimethoxyphenyl)propan-1-one (**8**)

Prepared in a similar manner as (**7**) from 3,4,5- Trimethoxy-3'- hydroxy chalcone (5). Flash chromatography (silica gel, 40% → 50% EtOAc/hexanes) of crude material afforded 1.37 g (68%) of white solids: IR (neat) 3395, 2940, 1680, 1590, 1505, 1455, 1240, 1125 $cm^{-1}$; $^1H$ NMR ($CDCl_3$, 300 MHz) 7.54-7.52 (m, 2H), 7.34 (app t, J = 8.1 Hz, 1H), 7.10 (dd, J= 7.9, 2.2 Hz, 1H), 6.48 (s, 2H), 6.08 (s, 1H), 3.85 (s, 9H), 3.30 (t, J = 7.3 Hz, 2H), 3.02 (t, J = 7.7 Hz, 2H); $^{13}C$ NMR ($CDCl_3$, 75 MHz) 200.0, 156.7, 153.6, 138.7, 137.4, 136.7, 130.3, 120.9, 115.0, 105.8, 61.3, 56.5, 41.0, 31.0.

1-(3-Hydroxyphenyl)-3- (3,4-methylenedioxyphenyl)propan-1-one (**9**)

Prepared in a similar manner as (7) from 3'- Hydroxy- 3,4-methylenedioxy chalcone (**6**). Crystallization of crude material from EtOAc/hexanes afforded 4.10 g (41%) of white solids: $^1H$ NMR ($CDCl_3$, 300 MHz) 9.73 (s, 1H), 7.43 (d, J= 7.8 Hz, 1H), 7.34-7.29 (m, 2H), 7.02 (dd, J = 8.0 Hz, 1H), 6.88 (m, 1H), 6.80 (d, J = 7.9 Hz, 1H), 6.71 (d, J = 7.9 Hz, 1H), 5.96 (s, 2H), 3.26 (t, J = 7.6 Hz, 2H), 2.84 (t, J = 7.5 Hz, 2H); $^{13}C$ NMR ($CDCl_3$, 75 MHz) 199.4 158.0, 147.5, 145.7, 138.4, 135.4, 130.1, 121.5, 120.5, 119.3, 114.4, 109.2, 108.4, 101.0, 40.2, 29.7.

1-(3-(*tert*-Butoxycarbonylmethoxy)phenyl)-3-(3,4-dimethoxyphenyl)propan-1-one (**10**)

A 60% mineral oil suspension of NaH (279 mg, 6.98 mmol) in anhydrous DMF (10 mL) was cooled to 0 °C in an ice bath and solid 3-(3,4- Dimethoxyphenyl)-1- (3-hydroxyphenyl)propan-1-one (**7**) (2 g, 6.98 mmol) added in one

portion. The resulting yellow solution was stirred for 5 min after which time *tert*-butylbromoacetate (1.18 mL, 7.33 mmol) was added. Stirring was continued at 0 °C for 15 mm after which time the reaction mixture was warmed to room temperature and partitioned between diethyl ether (50 mL) and water (50 mL). The organic layer was washed with a saturated NaCl solution (2 x 50 mL), dried over $MgSO_4$, filtered, evaporated, and flash chromatographed (silica gel, 30% EtOAc/hexanes) to afford 2.30g (82%) of a clear colorless oil: IR (neat) 2980, 1750, 1685, 1590, 1515, 1260, 1155 $cm^{-1}$; $^1$H NMR ($CDCl_3$, 300 MHz) 7.59 (d, J = 7.7 Hz, 1H), 7.49 (s, 1H), 7.39 (app t, J = 8.0 Hz, 1H), 7.14 (dd, J = 8.2, 2.6 Hz, 1H), 6.81-6.79 (m, 3H), 4.58 (s, 2H), 3.89 (s, 3H), 3.88 (s, 3H), 3.28 (t, J = 7.3 Hz, 2H), 3.02 (t, J = 7.8 Hz, 2H), 1.51 (s, 9H); $^{13}$C NMR ($CDCl_3$, 75 MHz) 199.2, 168.0, 158.6, 149.3, 147.8, 138.7, 134.2, 130.1, 121.8, 120.6, 113.5, 112.2, 111.8, 108.1, 83.0, 66.1, 56.2, 41.1, 30.2, 28.4.

1-(3-(*tert*-Butoxycarbonylmethoxy)phenyl)-3-(3,4,5-trimethoxyphenyl)propan-1-one (**11**)

Prepared in a similar manner as (**10**) from 1-(3-Hydroxyphenyl)-3- (3,4,5-trimethoxyphenyl)propan-1-one (**8**). Flash chromatography (silica gel, 30% → 40% EtOAc/hexanes) of crude material afforded 1.30 g (96%) of a clear colorless oil: IR (neat) 2955, 1750, 1684, 1590, 1455, 1230, 1150, 1125 $cm^{-1}$; $^1$H NMR ($CDCl_3$, 300 MHz) 7.59 (d, J = 7.7 Hz, 1H), 7.49 (s, 1H), 7.39 (app t, J = 7.9 Hz, 1H), 7.14 (dd, J = 8.2, 2.6 Hz, 1H), 6.47 (s, 2H), 4.58 (s, 2H), 3.86 (s, 6H), 3.84 (s, 3H), 3.28 (t, J = 7.3 Hz, 2H), 3.01 (t, J = 7.8 Hz, 2H), 1.50 (s, 9H); $^{13}$C NMR ($CDCl_3$, 75MHz) 199.1 168.0, 158.5, 153.6, 138.6, 137.4 136.8, 130.1, 121.8, 120.4, 113.6, 105.8, 83.0, 66.1, 61.2, 56.5, 41.0, 31.0, 28.4.

1-(3-(*tert*-Butoxycarbonylmethoxy)phenyl)-3-(3,4-methylenedioxyphenyl)propan-1-one (**12**)

Prepared in a similar manner as (**10**) from 1-(3-Hydroxyphenyl)-3- (3,4-methylenedioxyphenyl)propan-1-one (**9**). Flash chromatography (silica gel, 20% → 30% EtOAc/hexanes) of crude material afforded 5.04 g (89%) of a clear colorless oil: IR (neat) 2980, 1750, 1685, 1490, 1445, 1245, 1155, 1040 $cm^{-1}$; $^1$H NMR ($CDCl_3$, 300 MHz) 7.58 (dd, J = 6.7, 1.1 Hz, 1H), 7.48 (s, 1H), 7.39 (app t, J = 8.0 Hz, 1H), 7.17-7.13 (m, 1H), 6.89-6.69 (m, 4H), 5.94 (s, 2H), 4.58 (s, 2H), 3.25 (t, J = 7.8 Hz, 2H), 2.99 (t, J = 7.8 Hz, 2H), 1.51 (s, 9H); $^{13}$C NMR ($CDCl_3$, 75MHz) 199.0 168.0, 158.5, 148.1, 146.3, 138.6, 135.4, 130.1, 121.8, 121.5, 120.6, 113.4, 109.3, 108.7, 101.2, 83.0, 66.1, 41.1, 20.3, 28.4.

(R) 1-(3-(*tert*-Butoxycarbonylmethoxy)phenyl)-3-(3,4-dimethoxyphenyl)propan-1-ol (**13**)

A solution of 1-(3-(*tert*-Butoxycarbonylmethoxy)phenyl)-3-(3,4-dimethoxyphenyl)propan-1-one (**10**) (3.0 g, 7.49 mmol) in THF (5 mL) at -20 °C was treated with a solution of (+)-B-chlorodiisopinocamphenylborane (2.9 g, 8.99 mmol) in THF (10 mL) at -20 °C. The resulting mixture was allowed to stand in a -20 °C freezer for 48 h after which time the mixture was evaporated and treated with diethyl ether (25 mL) followed by diethanolamine (8 mL). The viscous mixture was allowed to stir at room temperature for 3 h, after which time, was filtered through a pad of Celite with the aid of diethyl ether. The cloudy filtrate was evaporated and flash chromatographed (silica gel, 30% → 40% EtOAc/hexanes) to afford 2.72 g (90%) of a clear colorless oil. (95% ee by Chiracel HPLC, 25% i-PrOH/hexanes, retention time 44.4 min for the *R*-enantiomer and 35.7 min for the S-enantiomer): IR (neat) 3525, 2935, 1750, 1515, 1150 $cm^{-1}$; $^1$H NMR ($CDCl_3$, 300 MHz) 7.30 -7.25 (m, 2H), 6.99-6.73 (m, 5H), 4.68 (m, 1H), 4.53 (s, 2H), 3.88 (s, 3H), 3.87 (s, 3H), 2.72-2.63 (m, 2H), 2.12-1.97 (m, 2H), 1.50 (s, 9H); $^{13}$C NMR ($CDCl_3$, 75 MHz) 168.4, 158.5, 149.3, 147.6, 146.9, 134.8, 130.0, 120.6, 119.5, 114.0, 112.6, 112.2, 111.7, 82.7, 74.1, 66.1, 56.3, 56.2, 41.0, 32.0, 28.4.

(1*R*)-3-(3,4,5-Trimethoxyphenyl)-1-(3-(tert-butoxycarbonylmethoxy)phenyl)-propan-1-ol (**14**)

To a solution of **11** (1.30 g, 3.0 mmol) in THF (5 mL) at -23 °C under $N_2$ was added a cold (-23 °C) solution of (+)-*B*-chlorodiisopinocampheylborane (1.64 g, 5.1 mmol) in THF (10 mL). The mixture was placed in a freezer for 3 days. Then, the mixture was concentrated *in vacuo* and the residue was redissolved in diethyl ether (60 mL). The ether solution was treated with diethanolamine (0.86 mL, 9.0 mmol) with vigorous stirring at room temperature for 3 h. The white precipitates were filtered off and the filtrate was concentrated *in vacuo*. Chromatography on silica (50-100% EtOAc/hexanes) provided 1.3 g (99%) of a colorless oil (98.1% ee by Chiracel HPLC, 20% i-PrOH/hexanes, retention time 46.4 min for the *R*-enantiomer and 40.0 min for the *S*-enantiomer). $^1$H NMR ($CDCl_3$, 300 MHz) 7.28 (t, J = 7.8 Hz, 1 H), 6.96 (m, 2 H), 6.82 (m, 1 H), 6.41 (s, 2 H), 4.69 (t, J = 6.2 Hz, 1 H), 4.52 (s, 2 H), 3.85 (s, 6 H), 3.83 (s, 3 H), 2.65 (m, 2 H), 2.05 (m, 2 H), 1.50 (s, 9 H); $^{13}$C NMR ($CDCl_3$, 75MHz) 168.4, 158.6, 153.5, 146.8, 137.9, 136.6, 130.0, 119.5, 114.0, 112.7, 105.7, 82.8, 74.1, 66.0, 61.2, 56.5, 40.8, 32.8, 28.4.

(R) 1-(3-(*tert*-Butoxycarbonylmethoxy)phenyl)-3-(3,4-methylenedioxyphenyl)propan-1-ol (**15**)

Prepared in a similar manner as (**13**) from 1-(3-(*tert*--Butoxycarbonylmethoxy)phenyl)-3-(3,4-methylenedioxyphenyl)propan-1-one (**12**). Flash chromatography (silica gel, 20% → 25% EtOAc/hexanes) of crude material afforded 3.84g (96%) of a clear colorless oil: IR (neat) 3440, 1750, 1490, 1440, 1245, 1150, 1040 $cm^{-1}$; $^1H$ NMR ($CDCl_3$, 300 MHz) 7.30-7.24 (m, 1 H),6.98-6.93 (m, 2H), 6.82 (dd, J = 8.2, 2.5 Hz, 1H), 6.75-6.64 (m, 3H), 5.93 (s, 2H), 4.67-4.63 (m, 1H), 4.53 (s, 2H), 2.68-2.60 (m, 2H), 2.10-1.95 (m, 3H), 1.51 (s, 9H); $^{13}C$ NMR ($CDCl_3$, 75MHz) 168.4, 158.5, 148.0, 146.9, 146.0, 136.0, 130.0, 121.5, 119.5, 114.1, 112.5, 109.3, 108.5, 101.1, 82.7, 73.9, 66.1, 41.1, 32.1, 28.4.

3'-(*tert*-Butoxycarbonylmethoxy)acetophenone (**16**)

To a suspension of NaH (60% dispersion in mineral oil, 1.47 g, 36.7 mmol) in anhydrous DMF (50 mL) at 0 °C was added solid 3'-hydroxyacetophenone (5.0 g, 36.7 mmol). The mixture was stirred under $N_2$ for 10 min and a clear yellow solution was formed. Then, *tert*-butylbromoacetate (6.23 mL, 38.5 mmol) was added and the mixture stirred at 0 °C for 5 min and then at room temperature for 20 min. TLC showed no starting material remaining. The mixture was partitioned between EtOAc (250 mL) and water (100 mL). The organic layer was separated, washed with saturated brine, dried ($MgSO_4$) and concentrated *in vacuo*. Chromatography on sihca (20% EtOAc/hexanes) gave 7.6 g (83%) of a white crystal. $^1H$ NMR ($CDCl_3$, 300 MHz) 7.60-7.14 (m, 4 H), 4.59 (s, 2 H), 2.60 (s, 3 H), 1.51 (s, 9 H); $^{13}C$ NMR ($CDCl_3$, 75MHz) 198.0, 168.0, 158.6, 138.9, 130.1, 122.3, 120.6, 113.5, 83.0, 66.1, 28.4, 27.0.

3-(3-Pyridyl)-1-(3-(tert-butoxycarbonylmethoxy)phenyl)-2-propen-1-one (**17**)

A mixture of **16** (4.0 g, 16 mmol), nicotinaldehyde (1.89 mL, 20 mmol), and piperidine (4.0 mL, 40 mmol) in absolute EtOH (65 mL) was heated at reflux for **16** h. The mixture was cooled and concentrated *in vacuo*. Chromatography on silica gel (30-60% EtOAc/hexanes) gave a mixture of unreacted nicotinaldehyde and **17** (both have the same $R_f$ on TLC). Washing of the mixture with hexane in a filter funnel provide 1.73 g (32%) of pure **17** as a yellow crystal. $^1H$ NMR ($CDCl_3$, 300 MHz) 8.87 (d, J = 2.1 Hz, 1 H), 8.66 (dd, J = 4.8, 1.5 Hz, 1 H), 7.97 (d, J = 7.9 Hz, 1 H), 7.80 (d, J = 16.7 Hz, 1 H), 7.66 (d, J = 7.6 Hz, 1 H), 7.60 (d, J = 15.9 Hz, 1 H), 7.55 (s, 1 H), 7.46 (t, J = 7.8 Hz, 1 H), 7.38 (dd, J = 7.9, 4.8 Hz, I H), 7.20 (dd, J = 8.2, 2.6 Hz, I H), 4.62 (s, 2 H), 1.52 (s, 9 H); $^{13}C$ NMR ($CDCl_3$, 75MHz) 189.7, 175.0, 168.0, 158.7, 151.6, 150.5, 141.4, 139.5, 134.9, 131.0, 130.2, 124.2, 122.3, 120.6, 114.1, 83.1, 66.2, 28.4.

1-(3-(*tert*-Butoxycarbonylmethoxy)phenyl)-3-(3-indoyl)-2-propen-1-one (**18**)

A mixture of **16** (2.0 g, 8.0 mmol) and 3-indolecarboxaldehyde (967 mg, 6.66 mmol) m piperidine (4mL) was heated at reflux for 6 h. The reaction mixture was cooled and treated with pH 7 phosphate buffer (25 mL) and EtOAc (50 mL). The organic portion was washed with a saturated $NaHCO_3$ solution (2 x 50 mL) followed by a saturated NaCl solution (2 x 25 mL) solution. The organic layer was then dried over $MgSO_4$, filtered, evaporated, and flash chromatographed (silica gel, 50% EtOAc/hexanes) to afford 1.47 g (59%) of yellow solids. IR (neat) 1730, 1650, 1560, 1240, 1150 $cm^{-1}$; $^1H$ NMR (MeOH, 300 MHz) 8.06 (d, J = 15.5 Hz, 1 H), 7.94-7.91 (m, 1 H), 7.76 (s, 1H), 7.62 (dd, J = 6.7, 1.1 Hz, 1H), 7.52-7.42 (m, 4H), 7.40-7.17 (m, 3H), 4.61 (s, 2H), 1.42 (s, 9H); $^{13}C$ NMR (MeOH, 75 MHz) 192.9, 170.5, 160.2, 142.4, 142.1, 139.8, 134.2, 131.3, 127.2, 124.5, 123.0, 121.7, 120.7, 117.4, 115.3, 113.7, 84.0, 67.2, 28.7.

3-(3-Pyridyl)-1-(3-(tert-butoxycarbonylmethoxy)phenyl)-propan-1-one (**19**)

A mixture of **17** (1.70 g, 5.0 mmol) and 10% Pd/C (85 mg) in EtOAc (70 mL) was hydrogenated in a Parr under $H_2$ at 42 psi for 15 h. The mixture was filtered through Celite and the filtrate was concentrated *in vacuo*. Chromatography on silica (50-60% EtOAc/hexanes) gave 1.70 g (100%) of a colorless oil. $^1H$ NMR ($CDCl_3$, 300 MHz) 8.54 (d, J = 2.0 Hz, 1 H), 8.48 (dd, J = 4.8, 1.5 Hz, 1 H), 7.70-7.10 (m, 6 H) 4.58 (s, 2 H), 3.31 (t, J = 7.3 Hz, 2 H), 3.09 (t, J = 7.4 Hz, 2 H), 1.50 (s, 9 H); $^{13}C$ NMR ($CDCl_3$, 75MHz) 198.3, 168.0, 158.6, 150.4, 148.1, 138.4, 136.9, 136.4, 130.2, 123.7, 121.8, 120.7, 113.5, 83.0, 66.1, 40.2, 28.4, 27.5.

1-(3-(*tert*-Butoxycarbonylmethoxy)phenyl)-3-(3-indoyl)propan-1-one (**20**)

Prepared in a similar manner as (**19**) from 1-(3-(*tert*-Butoxycarbonylmethoxy)phenyl)-3-(3-indoyl)-2-propen-1-one (**18**). Flash chromatography (silica gel, 20% → 30% EtOAc/hexanes) afforded 468 mg (80%) of a white solid: IR (neat) 1735, 1680, 1230, 1150 $cm^{-1}$; $^1H$ NMR (MeOH, 300 MHz) 7.60-7.55 (m, 2 H), 7.43-7.32 (m, 3H), 7.16-6.99 (m, 4H), 4.57 (s, 2H), 3.39-3.32 (obs t, 2H), 3.16 (t, J = 7.2 Hz, 2H), 1.47 (s, 9H); $^{13}C$ NMR (MeOH, 75 MHz) 202.5, 170.4, 160.0, 140.2, 138.6, 131.3, 129.0, 123.5, 122.9, 122.7, 121.5, 120.0, 119.7, 115.6, 114.6, 112.6, 84.0, 67.1, 41.0, 28.7, 21.6.

(1*R*)-3-(3-Pyridyl)-1-(3-(tert-butoxycarbonylmethoxy)phenyl)-propan-1-ol (**21**)

To a solution of **19** (1.70 g, 4.98 mmol) in THF (10 mL) at -23 °C under $N_2$ was added a cold (-23 °C) solution of (+)-B-chlorodiisopinocamphenylborane (3.2 g, 9.97 mmol) in THF (20 mL). The mixture was placed in a freezer for 3 days. Then, the mixture was concentrated *in vacuo* and the residue was redissolved in diethyl ether (100 mL). The ether solution was treated with diethanolamine (1.44 mL, 15.0 mmol) with vigorous stirring at room temperature for 3 h. The white precipitates were filtered off and the filtrate was concentrated *in vacuo*. Chromatography on silica (50-100% EtOAc/hexanes) provided 1.41 g (82%) of a colorless oil (97.5% ee by Chiracel HPLC, 25% i-PrOH/hexanes, retention time 78.5 min for the *R*-enantiomer and 52.1 min for the *S*-enantiomer). $^1$H NMR ($CDCl_3$, 300 MHz) 8.42 (m, 2 H), 7.55-6.80 (m, 6 H), 4.65 (dd, J = 7.8, 5.1 Hz,1 H), 4.52 (s, 2 H), 2.75 (m, 2 H), 2.05 (m, 2 H), 1.49 (s, 9 H); $^{13}$C NMR ($CDCl_3$, 75MHz) 175.1, 168.4, 158.6, 150.3, 147.7, 146.8, 137.5, 136.3, 130.0, 123.7, 119.4, 114.1, 112.5, 108.0, 82.8, 73.5, 66.0, 40.5, 29.5, 28.4.

(R) 1-(3-(tert-Butoxycarbonylmethoxy)phenyl)-3-(3-indoyl)propan-1-ol (**22**)

Prepared in a similar manner as (**21**) from 1-(3-(*tert*-Butoxycarbonylmethoxy)phenyl)-3-(3-indoyl)propan-1-one (**20**). Flash chromatography (silica gel, 20% → 30% EtOAc/hexanes) afforded 258 mg (55%) of yellowish oil (+95% ee by Chiracel HPLC, 20% i-PrOH/hexanes, retention time 54.2 mm for the R-enantiomer and 50.7 min for the *S*-enantiomer): IR (neat) 3410, 2930, 1735, 1455, 1230, 1150, 1080 cm$^{-1}$; $^1$H NMR (MeOH, 300 MHz) 7.51 (d, J = 7.8 Hz, I H), 7.33 (d, J = 8.1, 1H), 7.25 (app t, J = 7.9, 1H), 7.11-6.92 (m, 5H), 6.82-6.78 (m, 1H), 4.67 (t, J = 5.8 Hz, 1H), 4.54 (s, 2H), 2.85-2.77 (m, 2H), 2.18-2.06 (m, 2H), 1.47 (s, 9H); $^{13}$C NMR (MeOH, 75 MHz) 170.8, 159.9, 148.9, 138.6, 130.8, 129.2, 123.2, 122.6, 120.8, 119.9, 116.4, 114.9, 113.7, 112.6, 83.8, 75.0, 67.0, 41.3, 28.7, 22.9.

<u>Preparation of Functionalized Monomers</u>

(1*R*)-3-Phenyl-1-[3-((3-aminopropyl)oxy)phenyl]-1-propyl (2*S*)-1-(3,3-dimethyl-1,2-dioxopentyl)-2-piperidinecarboxylate trifluroacetic acid salt (**24**)

A solution of alcohol **2** (385 mg, 1.0 mmol) in $CH_2Cl_2$ (3 mL) was treated with (2*S*)-1-(1,2-dioxo-3,3-dimethylpentyl)-2-piperidinecarboxylic acid (**23**, 255 mg, 1.0 mmol, prepared from L-pipercolic acid in 4 steps following literature procedures by Holt <u>et al</u>. J. *Amer. Chem. Soc.,* **1993**, *115*, 9925-9938), followed by 1,3-dicyclohexylcarbodiimide (DCC, 247 mg, 1.2 mmol), and 4-(dimethylamino)-pyridine (DMAP, 85 mg, 0.70 mmol) under a nitrogen atmosphere. The resulting bright yellow suspension was allowed to stir overnight. The mixture was then filtered through glass wool and chromatographed (silica gel, 20% EtOAc/hexanes) to give (1*R*)-3-Phenyl-1-[3-9(3-*tert*-butyloxycarbamylpropyl) oxy)phenyl]-1-propyl (2*S*)-1-(3,3-dimethyl-1,2-dioxopentyl)-2-piperidinecarboxylate (524 mg, 84%) as a colorless oil: $^1$H NMR ($CDCl_3$, 300 MHz) (single diastereomer, mixture of rotamers) 7.35-6.90 (m, 9 H), 5.80 (t, J = 5.9 Hz, 1 H), 5.32 (d, J = 5.0 Hz, 0.82 H, pipercolate α-H of rotamer A), 4.80 (br. s, 1 H), 4.02 (t, J = 6.1 Hz, 2 H), 3.40-3.25 (m, 3 H), 3.12 (td, J = 13.0, 3.3 Hz, 1 H), 2.60 (m, 2 H), 2.35 (d, J = 14 Hz, 1 H), 2.28 (m, 1 H), 2.07 (m, 1 H), 1.96 (t, J = 6.3 Hz, 2 H), 1.80-1.60 (m, 5 H), 1.43 (s, 9 H), 1.22 (s, 3 H), 1.20 (s, 3 H), 0.88 (t, J = 7.4 Hz, 3 H); $^{13}$C NMR ($CDCl_3$, 75MHz) (single diastereomer, mixture of rotamers) 208.2, 170.1, 167.6, 159.4, 156.4, 141.7, 141.3, 130.1, 128.9, 128.7, 126.5, 119.4, 114.7, 113.2, 113.0, 66.1, 57.1, 51.7, 47.1, 44.5, 38.3, 32.9, 32.1, 30.0, 28.8, 26.8, 25.4, 24.9, 24.0, 23.8, 23.5, 21.6, 9.1. MS(FAB): $(M+Na)^+$ 645, $(M+H)^+$ 623.

A solution of the above compound (200 mg, 0.32 mmol) in $CH_2Cl_2$ (5.0 mL) was treated with trifluoroacetic acid (1.0 mL) and the mixture was stirred at room temperature for 1 h. The reaction mixture was diluted with toluene (150 mL) and concentrated *in vacuo* to give **24** (203 mg, 100%) as a colorless gum: $^1$H NMR ($CDCl_3$, 300 MHz) (single diastereomer, mixture of rotamers) 7.90 (br. s, 3 H), 7.30-6.70 (m, 9 H), 5.70 (t, J= 5.4 Hz, 1 H), 5.23 (d, J = 4.8 Hz, 1 H), 4.01 (m, 2 H), 3.30 (d, J = 12.8 Hz, 1 H), 3.13 (m, 3 H), 2.58 (m, 2 H), 2.40-2.00 (m, 4 H), 1.75-1.50 (m, 5 H), 1.35 (m, 2 H), 1.13 (s, 3 H), 1.12 (s, 3 H), 0.80 (t, J = 7.4 Hz, 3 H); $^{13}$C NMR ($CDCl_3$, 75MHz) (single diastereomer, mixture of rotamers) 208.4, 175.3, 175.2, 170.1, 167.8, 158.8, 142.0; 141.2, 130.2, 128.9, 128.7, 126.5, 119.8, 114.6, 113.0, 108.0, 66.1, 51.8, 47.1, 44.6, 38.6, 38.3, 32.8, 32.1, 27.3, 26.8, 25.3, 23.8, 23.4, 21.5, 9.0. HRMS(FAB): $(M+Na)^+$ calcd: 523.3172 found: 523.3162.

(1*R*)-3-Phenyl-1-(3-(hydroxycarbonylmethoxy)phenyl)-1-propyl (2*S*)-1-(3,3-dimethyl-1,2-dioxopentyl)-2-piperidinecarboxylate (**25**)

A solution of alcohol **3** (342 mg, 1.0 mmol) in $CH_2Cl_2$ (3 mL) was treated with (2*S*)-1-(1,2-dioxo-3,3-dimethylpentyl)-2-piperidinecarboxylic acid (23, 255 mg, 1.0 mmol, prepared from L-pipecolic acid in 4 steps following literature procedures by Holt <u>et al</u>. *J. Amer. Chem. Soc*., **1993,** *115*, 9925-9938), followed by 1,3-dicyclohexylcarbodiimide (DCC,

247 mg, 1.2 mmol), and 4-(dimethylamino)-pyridine (DMAP, 85 mg, 0.70 mmol) under a nitrogen atmosphere. The resulting bright yellow suspension was allowed to stir overnight. The mixture was then filtered through glass wool and chromatographed (silica gel, 20% EtOAc/hexanes) to give (1*R*)-3-Phenyl-1-(3-(*tert*-butoxycarbonylmethoxy)phenyl]-1-propyl (2*S*)-1-(3,3-dimethyl-1,2-dioxopentyl)-2-piperidinecarboxylate (470 mg, 82%) as a colorless oil: $^1$H NMR ($CDCl_3$, 300 MHz) (single diastereomer, mixture of rotamers) 7.50-6.90 (m, 9 H), 5.93 (t, J = 6.0 Hz, 1 H), 5.46 (d, J = 3.4 Hz, 0.83 H, pipercolate $\alpha$-H of rotamer A), 4.67 (s, 2 H), 3.50 (d, J = 12.9 Hz, 1 H), 3.32 (td, J = 12.5, 3.0 Hz, I H), 2.75 (m, 2 H), 2.53 (d, J = 13.6 Hz, 1 H), 2.41 (m, 1 H), 2.22 (m, 1 H), 2.97-2.71 (m, 6 H), 1.62 (s, 9 H), 1.38 (s, 3 H), 1.35 (s, 3 H), 1.03 (t, J = 7.4 Hz); $^{13}$C NMR ($CDCl_3$, 75MHz) (single diastereomer, mixture of rotamers) 208.3, 175.0, 170.0, 168.0, 167.5, 158.5, 141.7, 141.2, 130.2, 128.9, 128.7, 126.5, 120.2, 114.7, 113.6, 82.8, 66.1, 51.6, 47.1, 44.5, 38.2, 32.9, 32.0, 28.4, 26.8, 25.3, 24.0, 23.4, 21.6, 9.2. HRMS(FAB): $(M+Na)^+$ calcd: 602.3094, found: 602.3090.

A solution of the above *tert*-butyl ester (200 mg, 0.34 mmol) in $CH_2Cl_2$ (5.0 mL) was treated with trifluoroacetic acid (1.0 mL) and the mixture was stirred at room temperature for 1 h. The reaction mixture was diluted with toluene (150 mL) and concentrated *in vacuo* to give **25** (177 mg, 99%) as a colorless gum: $^1$H NMR ($CDCl_3$, 300 MHz) (single diastereomer, mixture of rotamers) 7.30-6.80 (m, 9 H), 5.75 (m, 1 H), 5.30 (d, J = 4.8 Hz, 1 H), 4.66 (s, 2 H), 3.35 (d, J = 9.27 Hz, 1 H), 3.19 (td, J = 12.4, 2.9 Hz, 1 H), 2.69 (m, 2 H), 2.39 (d, J = 16.2 Hz. 1 H), 2.30 (m, 1 H), 2.10 (m, 1 H), 1.90-1.60 (m, 6 H), 1.50 (m, 1 H), 1.19 (s, 3 H), 1.17 (s, 3 H), 0.85 (t, J = 7.4 Hz, 3 H); $^{13}$C NMR ($CDCl_3$, 75MHz) (single diastereomer, mixture of rotamers) 208.0, 172.3, 169.8, 167.9, 158.2, 142.2, 141.1, 130.2, 128.9, 128.7, 126.5, 120.3, 115.5, 111.8, 65.5, 57.2, 52.0, 47.2, 44.6, 38.3, 33.0, 32.9, 32.1, 27.0, 25.3, 25.2, 23.9, 23.4, 21.5, 9.1. HRMS (FAB): $(M+Na)^+$ calcd: 546.2468, found: 546.2461.

(1*R*)-3-(3,4-Dimethoxyphenyl)-1-[3-(hydroxycarbonylmethoxy)phenyl]-1-propyl (2*S*)-1-(3,3-dimethyl-1,2-dioxopentyl)-2-piperidinecarboxylate (**26**)

A solution of (R) 1-(3-(*tert*-Butoxycarbonylmethoxy)phenyl)-3-(3,4-dimethoxyphenyl) propan-1-ol (**13**) (805 mg, 2.0 mmol) in $CH_2Cl_2$ (4 mL) at 0°C was treated with (2*S*)-1-(1,2-dioxo-3,3-dimethylpentyl)-2-piperidinecarboxylic acid (**23**, 511mg, 2.0 mmol, prepared from L-pipecolic acid in 4 steps following literature procedures by Holt et al. *J. Amer. Chem. Soc.,* **1993,** *115*, 9925-9938) followed by 4-(dimethylamino)pyridine (DMAP 1 mg) and 1,3-dicyclohexyl carbodiimide (DCC, 413 mg, 2 mmol) under a nitrogen atmosphere. The resulting bright yellow suspension was allowed to stir for 2 h then diluted with diethyl ether (20 mL). The reaction mixture was then filtered, evaporated, and flash chromatographed (silica gel, 25% → 30% EtOAc/hexanes) to afford 993 mg (78%) of a clear colorless viscous oil: IR (neat) 2940, 1735, 1645, 1515, 1455, 1225, 1150 $cm^{-1}$; $^1$H NMR ($CDCl_3$, 300 MHz) 7.20-7.17 (m, 2H), 6.91-6.69 (m, 5H), 5.73-5.68 (m, 1H). 5.24 (br s, 1H), 4.46 (s, 2H), 3.79 (s, 3H), 3.78 (s, 3H), 3.29 (br d, J = 13.2 Hz, 1H), 3.07 (td, J = 12.7, 3.0 Hz, 1H), 2.52-2.44 (m, 2H), 2.29 (br d, J = 13.6 Hz, 1H), 2.20-2.13 (m, 1H), 2.04-1.95 (m, 1H), 1.71-1.51 (m, 7H), 1.41 (s, 9H), 1.16, (s, 3H), 1.14 (s, 3H), 0.82 (t, J = 7.4 Hz, 3H); $^{13}$C NMR ($CDCl_3$, 75 MHz) 208.2, 170.1, 168.3, 167.6, 158.5, 149.3, 147.8, 141.8, 133.9, 130.1, 120.5, 120.3, 114.7, 113.7, 112.2, 111.7, 82.7, 66.2, 56.2, 51.7, 47.1, 44.6, 38.3, 32.9, 31.6, 28.8, 26.8, 25.3, 23.8, 23.5, 21.6, 9.1. HRMS(FAB): $(M+Na)^+$ calcd: 662.3305, found 662.3301.

A solution of the above *tert*-butyl ester (460 mg, 0.72 mmol) in $CH_2Cl_2$ (5.0 mL) was treated with trifluoroacetic acid (1.0 mL) and the mixture was stirred at room temperature for 1 h. The reaction mixture was diluted with toluene (150 mL) and concentrated *in vacuo* to give **26** (420 mg, 100%) as a yellowish foam: $^1$H NMR ($CDCl_3$, 300 MHz) (single diastereomer, mixture of rotamers) 8.00 (br. s, 1 H), 7.35-6.70 (m, 7H), 5.82 (m, 1 H), 5.33 (d, J = 4.5 Hz, 1 H), 4.71 (m, 2 H), 3.89 (s, 3 H), 3.87 (s, 3 H), 3.38 (d, J = 12.6 Hz, I H), 3.24 (td, J = 12.3. 2.7 Hz, 1 H), 2.60 (m, 2 H), 2.45-2.05 (m, 3 H), 1.70 (m, 6 H), 1.45 (m, 2 H), 1.23 (s, 3 H), 1.21 (s, 3 H), 0.89 (t, J = 7.4 Hz, 3 H); $^{13}$C NMR ($CDCl_3$, 75MHz) (single diastereomer, mixture of rotamers) 208.0, 172.0, 169.8, 167.8, 158.2, 149.4, 147.8, 142.2, 133.7, 130.2, 129.4, 128.6, 125.7, 120.6, 120.3, 115.5, 112.2, 111.8, 111.7, 108.2, 65.5, 56.3, 51.9, 47.2, 44.6, 38.5, 32.9, 31.7, 28.4, 27.0, 25.3, 23.9, 23.4, 21.8, 21.5, 9.1. HRMS(FAB): $(M+Na)^+$ calcd: 606.2679, found: 606.2692.

(1*R*)-3-(3,4,5-Trimethoxyphenyl)-1-[3-(hydroxycarbonylmethoxy)phenyl]-1-propyl (2*S*)-1-(3,3-dimethyl-1,2-dioxopentyl)-2-piperidinecarboxylate (**27**)

A solution of alcohol **14** (650 mg, 1.5 mmol) in $CH_2Cl_2$ (5 mL) was treated with (2*S*)-1-(1,2-dioxo-3,3-dimethylpentyl)-2-piperidinecarboxylic acid (23, 382 mg, 1.5 mmol, followed by 1,3-dicyclohexylcarbodiimide (370 mg, 1.8 mmol), and 4-(dimethylamino)-pyridine (128 mg, 1.0 mmol) under a nitrogen atmosphere. The resulting bright yellow suspension was allowed to stir overnight. The mixture was then filtered through glass wool and chromatographed (silica gel, 20-30% EtOAc/hexanes) to give (1*R*)-3-(3,4,5-trimethoxyphenyl)-1-[3-(*tert*-butoxycarbonylmethoxy)phenyl]-1-propyl (2S)-1-(3,3-dimethyl-1,2-dioxopentyl)-2-piperidinecarboxylate (776 mg, 78%) as a colorless oil: $^1$H NMR ($CDCl_3$, 300 MHz) (single diastereomer, mixture of rotamers) 7.30-6.80 (m, 4 H), 6.37 (s, 2 H), 5.82 (t, J = 6.1 Hz, 1 H), 5.33 (d, J = 5.2 Hz, 1 H), 4.54 (s, 2 H), 3.85 (s, 6 H), 3.83 (s, 3 H), 3.38 (d, J = 12.6 Hz, 1 H), 3.16 (td, J = 12.8,

3.1 Hz, 1 H), 2.60 (m, 2 H), 2.45-2.05 (m, 3 H), 1.70 (m, 6 H), 1.50 (s, 9 H), 1.45 (m, 2 H), 1.25 (s, 3 H), 1.23 (s, 3 H), 0.90 (t, J = 7.4 Hz, 3 H); $^{13}$C NMR ($CDCl_3$, 75MHz) (single diastereomer, mixture of rotamers) 208.2, 175.1, 170.1, 168.2, 167.6, 158.5, 153.6, 141.7, 137.0, 130.1, 120.9, 120.2, 114.6, 113.7, 105.7, 82.7, 66.2, 61.2, 56.5, 51.7, 47.1, 44.6, 38.2, 32.9, 32.4, 28.4, 26.8, 25.3, 23.9, 23.5, 21.6, 9.1.

A solution of the above *tert*-butyl ester (400 mg, 0.60 mmol) m $CH_2Cl_2$ (5.0 mL) was treated with trifluoroacetic acid (1.0 mL) and the mixture was stirred at room temperature for 1 h. The reaction mixture was diluted with toluene (150 mL) and concentrated *in vacuo* to give **27** (358 mg, 98%) as a white foam: $^1$H NMR ($CDCl_3$, 300 MHz) (single diastereomer, mixture of rotamers) 7.30-6.80 (m, 4 H), 6.39 (s, 2 H), 5.82 (m, 1 H), 5.33 (d, J = 4.6 Hz, 1 H), 4.70 (m, 2 H), 3.86 (s, 6 H), 3.84 (s, 3 H), 3.38 (d, J = 12.6 Hz, 1 H), 3.22 (td, J = 12.8, 3.1 Hz, I H), 2.60 (m, 2 H), 2.45-2.05 (m, 3 H), 1.70 (m, 6 H), 1.45 (m, 2 H), 1.23 (s, 3 H), 1.21 (s, 3 H), 0.89 (t, J = 7.4 Hz, 3 H); $^{13}$C NMR ($CDCl_3$, 75MHz) (single diastereomer, mixture of rotamers) 208.0, 175.0, 171.7, 169.8, 167.8, 158.2, 153.6, 142.1, 136.9, 130.2, 129.4, 128.6, 125.7, 120.3, 115.5, 111.8, 107.9, 105.8, 65.6, 61.2, 56.5, 52.0, 47.2, 44.6, 38.3, 32.9, 32.5, 27.0, 25.3, 23.8, 23.4, 21.5, 9.1. MS(FAB): $(M+Na)^+$ calcd: 636.2785, found: 636.2756.

(R) 1-(3-(Hydroxycarbonylmethoxy)phenyl)-3-(3,4-methylendioxyphenyl)-1-propyl (2S)-1-(3,3'-dimethyl-1,2-dioxopentyl)-2-piperdinecarboxylate (**28**)

A solution of (R) 1-(3-(*tert*-Butoxycarbonylmethoxy)phenyl)-3-(3,4-methylenedioxyphenyl) propan-1-ol (**15**) (500 mg, 1.29 mmol) in $CH_2Cl_2$ (4 mL) at 0 °C was treated with (2*S*)-1-(1,2-dioxo-3,3-dimethylpentyl)-2-piperidinecarboxylic acid (23, 330 mg, 1.29 mmol, prepared from L-pipecolic acid in 4 steps following literature procedures by Holt et al. *J. Amer. Chem. Soc.*, **1993,** *115*, 9925-9938) followed by 4-(dimethylamino)pyridine (DMAP 1 mg) and 1.3-dicyclohexyl carbodiimide (DCC, 267 mg, 1.29 mmol) under a nitrogen atmosphere. The resulting bright yellow suspension was allowed to stir for 2 h then diluted with diethyl ether (20 mL). The reaction mixture was filtered, evaporated, and flash chromatographed . Flash chromatography (silica gel, 20% → 30%% EtOAc/hexanes) of crude material afforded 556 mg (69%) of a clear colorless oil: IR (neat) 2970, 1745, 1700, 1640, 1490, 1440, 1245, 1150 cm$^{-1}$; $^1$H NMR ($CDCl_3$, 300 MHz) (single diastereomer, mixture of rotamers) 7.32-7.26 (m, 1H), 6.99-6.84 (m, 6H), 5.93 (s, 2H). 5.80-76 (m, 1H), 5.33 (d, J = 4.9 Hz, 1H), 4.55 (s, 2H), 3.38 (br d, J = 12.9 Hz, 1H), 3.16 (td, J = 12.3, 3.1 Hz, 1H), 2.63-2.50 (m, 2H), 2.38 (br d, J = 13.7 Hz, 1H), 2.26-2.16 (m, 1H), 2.09-2.04 (m, 1H), 1.81-1.57 (m, 7H), 1.51 (s, 9H), 1.26, (s, 3H), 1.23 (s, 3H), 0.91(t, J = 7.4 Hz, 3H); $^{13}$C NMR ($CDCl_3$, 75 MHz) (single diastereomer, mixture of rotamers) 208.2, 170.0, 168.3, 167.6, 158.5, 148.1, 146.2, 141.7, 135.0, 130.1, 121.5, 120.2, 114.9, 113.6, 109.2, 108.6, 101.2, 82.7, 66.2, 51.7, 47.1, 44.5, 38.3, 32.9, 31.6, 28.4, 26.8, 25.3, 23.8, 23.5, 21.6, 9.1. HRMS(FAB): $(M+Na)^+$ calcd: 646.2992, found 646.3021.

A solution of the above *tert*-butyl ester (625 mg, 1.00 mmol) in $CH_2Cl_2$ (4.0 mL) was treated with trifluoroacetic acid (1.5 mL) and the mixture was stirred at room temperature for 1 h. The reaction mixture was diluted with toluene (150 mL) and concentrated *in vacuo* to give **28** (483 mg, 85%) as a clear colorless oil: IR (neat) 3420, 2940, 1735, 1700, 1640, 1490, 1440, 1245, 1040 cm$^{-1}$; $^1$H NMR ($CDCl_3$, 300 MHz) (single diastereomer, mixture of rotamers) 7.12 (t, J = 6.7 Hz, 1H), 6.92-6.81 (m, 3H), 6.68-6.52 (m, 3H), 5.86 (s, 2H), 5.73 (t, J = 7.2 Hz, 1H), 5.33 (s, 1H), 4.40 (s, 2H), 3.34 (d, J = 12.2 Hz, 1H), 3.19 (t, J = 12.0 Hz, 1H), 2.54-2.46 (m, 2H), 2.34 (d, J = 12.6 Hz, 1H), 2.24-2.00 (m, 2H), 1.73- 1.32 (m, 7H), 1.18 (s, 3H), 1.16 (s, 3H), 0.84 (t, J = 7.3 Hz, 3H); $^{13}$C NMR ($CDCl_3$, 75 MHz) (single diastereomer, mixture of rotamers) 208.0, 169.9, 167.79, 158.3, 148.0, 146.2, 141.9, 135.0, 130.1, 121.5, 109.1, 108.6, 107.2, 101.2, 77.0, 51.9, 47.0, 44.6, 38.6, 32.9, 31.8, 26.9, 25.3, 23.9, 23.3, 21.6, 9.1.

(1*R*)-3-(3-Pyridyl)-1-(3-(hydroxycarbonylmethoxy)phenyl)-1-propyl (2*S*)-1-(3,3-dimethyl-1,2-dioxopentyl)-2-piperidinecarboxylate (**29**)

A solution of alcohol **21** (530 mg, 1.54 mmol) in $CH_2Cl_2$ (5 mL) was treated with (2*S*)-1-(1,2-dioxo-3,3-dimethylpentyl)-2-piperidinecarboxylic acid (**23**, 393 mg, 1.54 mmol, followed by 1,3-dicyclohexylcarbodiimide (381 mg, 1.85 mmol), and 4-(dimethylamino)-pyridine (132 mg, 1.08 mmol) under a nitrogen atmosphere. The resulting bright yellow suspension was allowed to stir overnight. The mixture was then filtered through glass wool and chromatographed (silica gel, 20-60% EtOAc/hexanes) to give (1*R*)-3-(3-Pyridyl)-1-[3-(*tert*-butoxycarbonylmethoxy)phenyl]-1-propyl (2*S*)-1-(3,3-dimethyl-1,2-dioxopentyl)-2-piperidinecarboxylate (860 mg, 96%) as a colorless oil: $^1$H NMR ($CDCl_3$, 300 MHz) (single diastereomer, mixture of rotamers) 8.46 (m, 2 H), 7.50-6.80 (m, 6 H), 5.80 (t, J = 6.1 Hz, 1 H), 5.32 (d, J = 5.0 Hz, 1 H), 4.54 (s, 2 H), 3.38 (d, J = 12.8 Hz, 1 H), 3.14 (td, J = 12.6, 3.0 Hz, 1 H) 2.60 (m, 2 H), 2.36 (d, J = 13.7 Hz, 1 H), 2.25 (m, 1 H), 2.10 (m, 1 H), 1.75 (m, 4 H), 1.49 (s, 9 H), 1.45 (m, 2 H), 1.24 (s, 3 H), 1.22 (s, 3 H), 0.90 (t, J = 7.4 Hz, 3 H); $^{13}$C NMR ($CDCl_3$, 75MHz) (single diastereomer, mixture of rotamers) 208.2, 175.1, 170.0, 168.2, 167.7, 158.6, 150.2, 148.1, 141.3, 136.6, 130.2, 123.8, 120.1, 115.0, 113.6, 107.9, 82.8, 66.1, 51.7, 47.1, 44.6, 39.2, 37.8, 34.4, 33.0, 29.2, 28.4, 26.7, 25.3, 23.9, 23.5, 21.6, 21.4, 9.1.

A solution of the above *tert*-butyl ester (400 mg, 0.69 mmol) in $CH_2Cl_2$ (5.0 mL) was treated with trifluoroacetic

acid (1.0 mL) and the mixture was stirred at room temperature for 1 h. The reaction mixture was diluted with toluene (150 mL) and concentrated *in vacuo* to give **29** (424 mg, 96%, trifluoroacetic acid salt) as a white foam: $^1$H NMR ($CDCl_3$, 300 MHz) (single diastereomer, mixture of rotamers) 8.75 (s, 1 H), 8.67 (d, J = 10.4 Hz, 1 H), 8.23 (t, J = 5.6 Hz, 1 H), 7.79 (dd, J = 7.9, 5.6 Hz, 1 H), 7.35-6.75 (m, 4 H), 5.80 (t, J = 6.1 Hz, 1 H), 5.25 (d, J = 5.0 Hz, 1 H), 4.75 (m, 2 H), 3.35 (d, J = 13.2 Hz, 1 H), 3.14 (td, J = 12.6, 3.0 Hz., 1 H) 2.75 (m, 2 H), 2.30 (m, 3 H), 1.70 (m. 6 H), 1.40 (m, 2 H), 1.22 (s, 6 H), 0.92 (t, J = 7.4 Hz, 3 H); $^{13}$C NMR ($CDCl_3$, 75MHz) (single diastereomer, mixture of rotamers) 208.3, 172.3, 169.9, 167.8, 158.6, 145.5, 142.5, 142.0, 139.8, 139.5, 130.6, 129.4, 128.6, 120.2, 117.1, 111.8, 65.2, 51.8, 47.1, 44.8, 36.7, 32.8, 28.4, 26.6, 25.2, 23.7, 21.4, 9.1. HRMS(FAB): $(M+Na)^+$ calcd: 547.2420, found: 547.2415.

(1*R*)-3-(3-Indolyl)-1-(3-(hydroxycarbonylmethoxy)phenyl)-1-propyl (2*S*)-1-(3,3-dimethyl-1,2-dioxopentyl)-2-piperidinecarboxylate (**30**)

The *tert*-butyl ester was prepared in a similar manner as the ester of **28** from (R) 1-(3-(*tert*-Butoxycarbonylmethoxy) phenyl)-3-(3-indoyl)propan-1-ol (**22**). Flash chromatography (silica gel, 30% EtOAc/hexanes) afforded 492 mg (76%) of clear colorless oil: IR (neat) 3410, 2970, 1735, 1700, 1635, 1455, 1225, 1150 $cm^{-1}$; $^1$H NMR ($CDCl_3$, 300 MHz) 8.04 (br s, 1H), 7.53 (d, J = 7.8 Hz, 1 H), 7.37 (d, J = 8.0 Hz, 1H), 7.30-7.11 (m, 3H), 7.01-6.84 (m, 4H), 5.91-5.86 (m, 1H), 5.35 (d, J = 4.8 Hz, 1H), 4.54 (s, 2H), 3.39 (d, J = 13.3 Hz, 1H), 3.18 (td, J = 12.6, 3.0 Hz, 1H), 2.87-2.74 (m, 2H), 2.41-2.18 (m, 3H), 1.82-1.57 (m, 7H), 1.50 (s, 9H), 1.27 (s, 3H), 1.24 (s, 3H), 0.92 (t, J = 7.4 Hz, 3H); $^{13}$C NMR ($CDCl_3$, 75 MHz) 208.3, 170.1, 168.3, 167.7, 158.5, 141.9, 136.8, 130.1, 127.7, 122.3, 121.8, 120.3, 119.6, 119.1, 115.4, 114.7, 113.7, 111.5, 82.7, 66.2, 51.7, 47.1, 44.5, 36.8, 32.9, 28.4, 26.9, 25.4, 24.0, 23.4, 21.6, 9.1. HRMS(FAB): $(M+Na)^+$ calcd: 641.3203, found: 641.3193.

A solution of the above *tert*-butyl ester (112 mg, 0.18 mmol) in $CH_2Cl_2$ (5.0 mL) was treated with trifluoroacetic acid (1.0 mL) and the mixture was stirred at room temperature for 1 h. The reaction mixture was diluted with toluene (150 mL) and concentrated *in vacuo* to give **30** (102 mg, 100%) as a brown foam: $^1$H NMR ($CDCl_3$, 300 MHz) (single diastereomer, mixture of rotamers) 7.90-6.70 (m, 10 H), 5.85 (m, 1 H), 5.35 (m, 1 H), 4.62 (m, 2 H), 3.40 (m, 1 H), 3.25 (m, 1 H), 2.80 (m, 2 H), 2.40-2.05 (m, 3 H), 1.85-1.45 (m, 12 H), 1.23 (s, 3 H), 1.21 (s, 3 H), 0.88 (t, J = 7.4 Hz, 3 H); $^{13}$C NMR ($CDCl_3$, 75MHz) (single diastereomer, mixture of rotamers) 208.0, 175.0, 169.8, 167.9, 158.2, 142.3, 130.2, 129.4, 128.6, 125.7, 122.5, 119.7, 119.1, 115.3, 111.6, 108.0, 65.5, 52.0, 47.2, 44.6, 32.9, 27.0, 25.3, 23.9, 23.4, 21.6, 9.1. HRMS(FAB): $(M+Na)^+$ calcd: 585.2577, found: 585.2561.

<u>Preparation of Dimerizers</u>

Example 1. *N,N'-Bis*[3-(3-((1*R*)-1-((2*S*)-(3,3-dimethyl-1,2-dioxopentyl)piperidtne-2-carbonyloxo)-3-phenyl) propylphenoxy)propyl] 1,4-phenylenediacetamide (31)

A mixture of 1.4-phenylenediacetic acid (194 mg, 1.0 mmol) and disuccinimidyl carbonate (512 mg, 2.0 mg) in anhydrous acetonitrile (5.0 mL) was treated with pyridine (243 μL, 3.0 mmol). The mixture was stirred at room temperature under nitrogen overnight. The resulting suspension was partitioned between EtOAc (70 mL) and water (50 mL). The organic layer was separated, washed with 1 M $Na_2CO_3$, water, 0.5 N HCl, saturated brine, dried ($Na_2SO_4$), and concentrated *in vacuo* to give disuccinimidyl 1,4-phenylenediacetate (144 mg, 37%) as a white solid. $^1$H NMR (DMSO-$d_6$, 300 MHz) 7.34 (s, 4 H), 4.10 (s, 4 H), 2.80 (s, 8 H).

A solution of **24** (102 mg, 0.16 mmol) in $CH_2Cl_2$ (2.0 mL) was treated with the above activated diester (31 mg, 0.080 mmol) and $Et_3N$ (67 μL, 0.48 mmol). The mixture was stirred at room temperature overnight. The resulting clear solution was impregnated on silica gel and evaporated to dryness. Chromatography (silica gel, 50 - 100% EtOAc/hexanes) provided **31** (60 mg, 62%) as a white solid: mp 55-57 °C; $^1$H NMR ($CDCl_3$, 300 MHz) (single diastereomer, mixture of rotamers) 7.32-7.15 (m, 16 H), 6.95 (d, J = 7.7 Hz, 2 H), 6.83 (s, 2 H), 6.74 (m, 2 H), 6.01 (br. s, 2 H), 5.80 (t, J = 5.8 Hz, 2 H), 5.32 (d, J = 4.9 Hz, 2 H), 3.98 (t, J = 5.7 Hz, 4 H), 3.52 (s, 4 H), 3.50-3.30 (m, 6 H), 3.22 (td, J = 12.4, 2.6 Hz, 2 H), 2.67 (m, 4 H), 2.38 (d, J = 13.6 Hz, 2 H), 2.30 (m, 2 H), 2.12 (m, 2 H), 1.95 (t, J = 6.1 Hz, 4 H), 1.85-1.60 (m, 10 H), 1.50 (m, 4 H), 1.23 (s, 6 H), 1.21 (s, 6 H), 0.89 (t, J = 7.4 Hz, 6 H); $^{13}$C NMR ($CDCl_3$, 75MHz) (single diastereomer, mixture of rotamers) 208.2, 208.0, 171.3, 170.1, 169.9, 167.8, 159.2, 141.8, 141.6, 141.2, 134.4, 130.3, 130.1, 128.9, 128.7, 126.5, 119.3, 115.0, 114.7, 113.1, 112.8, 57.1, 51.7, 47.1, 44.5, 43.7, 39.3, 38.3, 38.2, 37.8, 33.0, 32.8, 32.1, 29.3, 26.9, 25.3, 23.9, 23.5, 21.6, 21.4, 9.17, 9.13. MS(FAB): $(M+Na)^+$ 1225, $(M+H)^+$ 1203.

Example 2. *N,N'-Bis*[3-(3-((1*R*)-1-((2*S*)-(3,3-dimethyl-1,2-dioxopentyl)piperidine-2-carbonyloxo)-3-phenyl) propylphenoxy)propyl] suberamide (**32**)

Following the same procedure as in Example 1 except replacing suberic acid for 1,4-phenylenediacetic acid, obtained **32** (54 mg, 56%) as a white solid. mp 44-46 °C; $^1$H NMR ($CDCl_3$, 300 MHz) (single diastereomer, mixture of

rotamers) 7.35-6.85 (m, 18 H), 6.18 (br, s, 2 H), 5.86 (t, J = 5.9 Hz, 2 H), 5.39 (d, J = 4.9 Hz, 2 H), 4.12 (t, J = 5.9 Hz, 4 H), 3.60-3.40 (m, 6 H), 3.28 (td, J = 12.6, 2.8 Hz, 2 H), 2.70 (m, 4 H), 2.47 (d, J = 13.8 Hz, 2 H), 2.35 (m, 2 H), 2.30-2.00 (m, 12 H), 1.95-1.70 (m, 14 H), 1.55-1.35 (m, 6 H), 1.30 (s, 6 H), 1.28 (s, 6 H), 0.96 (t, J = 7.5 Hz, 6 H); $^{13}C$ NMR ($CDCl_3$, 75MHz) (single diastereomer, mixture of rotamers) 208.3, 170.0, 167.8, 159.3, 141.8, 141.2, 130.2, 128.9, 128.7, 126.5, 119.4, 114.7, 113.0, 66.4, 51.7, 47.1, 44.5, 38.3, 37.7, 32.8, 32.1, 29.3, 28.7, 26.9, 25.8, 25.3, 23.9, 21.6, 9.1. MS(FAB): $(M+Na)^+$ 1205.

Example 3. *N,N'-Bis*[3-(3-((1*R*)-1-((2*S*)-(3,3-dimethyl-1,2-dioxopentyl)piperidine-2-carbonyloxo)-3-phenyl) propylphenoxy)propyl] pyridine-2,6-dicarboxamide (**33**)

Following the same procedure as in Example 1 except replacing pyridine-2,6-dicarboxylic acid for 1,4-phenylenediacetic acid, obtained 33 (44 mg, 54%) as a white solid. mp 60-62 °C; $^1H$ NMR ($CDCl_3$, 300 MHz) (single diastereomer, mixture of rotamers) 8.34 (d, J = 7.7 Hz, 2 H), 8.00 (t, J = 7.7 Hz, 1 H), 7.99 (br. s, 2 H, NHs), 7.30-6.75 (m, 18 H), 5.77 (t, J = 5.7 Hz, 2 H), 5.30 (d, J = 4.8 Hz, 2 H), 4.02 (m, 4 H), 3.63 (m, 4 H), 3.35 (d, J = 12.7 Hz, 2 H), 3.20 (td, J = 12.7, 2.8 Hz, 2 H), 2.60 (m, 4 H), 2.36 (d, J = 13.3 Hz, 2 H), 2.24 (m, 2 H), 2.05 (m, 6 H), 1.80-1.65 (m, 10 H), 1.50 (m, 4 H), 1.20 (s, 6 H), 1.18 (s, 6 H), 0.85 (t, J = 7.4 Hz, 6 H); $^{13}C$ NMR ($CDCl_3$, 75MHz) (single diastereomer, mixture of rotamers) 208.2, 170.1, 167.7, 164.0, 159.3, 149.1, 142.0, 141.2, 139.3, 130.2, 128.9, 128.7, 126.5, 125.4, 119.4, 115.1, 113.2, 107.9, 67.0, 57.1, 51.6, 47.1, 44.5, 38.3, 37.9, 32.8, 32.1, 29.6, 28.0, 26.9, 25.4, 23.9, 23.5, 21.6, 9.1. MS(FAB): $(M+Na)^+$ 1198, $(M+H)^+$ 1176.

Example 4. *N,N'-Bis*[3-(3-((1*R*)-1-((2*S*)-(3,3-dimethyl-1,2-dioxopentyl)piperidine-2-carbonyloxo)-3-phenyl) propylphenoxy)propyl] pyridine-3,5-dicarboxamide (**34**)

Following the same procedure as in Example I except replacing pyridine-3,5-dicarboxylic acid for 1,4-phenylenediacetic acid, obtained 34 (32 mg, 39%) as a white solid. mp 62-64 °C; $^1H$ NMR ($CDCl_3$, 300 MHz) (single diastereomer, mixture of rotamers) 9.09 (d, J = 1.8 Hz, 2 H), 8.42 (d, J = 1.9 Hz, 1 H), 7.30-6.80 (m, 20 H), 5.78 (t, J = 5.6 Hz, 2 H), 5.28 (d, J = 4.7 Hz, 2 H), 4.12 (t, J = 5.6 Hz, 4 H), 3.68 (m, 4 H), 3.36 (d, J = 13.0 Hz, 2 H), 3.18 (td, J = 13.4, 3.4 Hz, 2 H), 2.60 (m, 4 H), 2.35 (d, J = 13.2 Hz, 2 H), 2.25 (m, 2 H), 2.05 (m, 6 H), 1.80-1.65 (m, 10 H), 1.50 (m, 4 H), 1.18 (s, 6 H), 1.16 (s, 6 H), 0.84 (t, J = 7.4 Hz, 6 H); $^{13}C$ NMR ($CDCl_3$, 75MHz) (single diastereomer, mixture of rotamers) 208.3, 170.1, 167.8, 165.2, 159.1, 150.9, 141.9, 141.3, 130.3, 130.2, 128.9, 128.7, 126.5, 119.5, 115.0, 112.7, 107.9, 67.0, 51.7, 47.1, 44.5, 38.7, 38.2, 32.8, 32.1, 26.8, 25.3, 23.9, 23.5, 21.5, 9.1. MS(FAB): $(M+Na)^+$ 1198, $(M+H)^+$ 1176.

Example 5. *N,N'-Bis*[3-(3-((1*R*)-1-((2*S*)-(3,3-dimethyl-1,2-dioxopentyl)piperidine-2-carbonyloxo)-3-phenyl) propylphenoxy)propyl] N-methyl-pyridinium-3,5-dicarboxamide iodide (35)

A solution of **34** (10 mg, 8.5 μmol) in acetone (1.0 mL) was treated with MeI (60 μL, 10.2 mmol). The mixture was left to stand at room temperature under dark for 3 d and TLC (100% EtOAc) showed all starting material converted to a baseline compound. The resulting deep yellow solution was concentrated *in vacuo* to afford **35** (11 mg, 100%) as a yellow solid. $^1H$ NMR (Acetone-$d_6$, 300 MHz) (single diastereomer, mixture of rotamers) 9.98 (s, 1 H), 9.62 (s, 2 H), 9.14 (br. s, 2 H), 7.20-6.70 (m, 20 H), 5.83 (t, J = 5.2 Hz, 2 H), 5.27 (d, J = 4.5 Hz, 2 H), 4.75 (s, 3 H), 4.18 (t, J = 6.4 Hz, 4 H), 3.67 (q, J = 6.1 Hz, 4 H), 3.45 (d, J = 13.4 Hz, 2 H), 3.25 (m, 2 H), 2.75 (m, 4 H), 2.20-1.90 (m, 10 H), 1.75 (m, 10 H), 1.50 (m, 4 H), 1.21 (s, 6 H), 1.19 (s, 6 H), 0.85 (t, J = 7.5 Hz, 6 H); $^{13}C$ NMR (Acetone-$d_6$, 75MHz) (single diastereomer, mixture of rotamers) 208.7, 170.8, 168.2, 162.1, 160.6, 148.7, 143.3, 142.6, 130.8, 129.6, 127 2, 119.8, 115.6, 113.9, 104.0, 77.8, 67.2, 52.5, 47.6, 45.3, 39.4, 38.1, 33.6, 32.8, 27.6, 26.1, 24.2, 23.8, 22.4, 9.5. MS(FAB): $M^+$ 1190.

Example 6. *N,N'-Bis*[3-(3-((1*R*)-1-((2*S*)-(3,3-dimethyl-1,2-dioxopentyl)piperidine-2-carbonyloxo)-3-phenyl) propylphenoxy)propyl] benzene-1,3-disulfonamide (**36**)

A solution of **24** (106 mg, 0.17 mmol) in $CH_2Cl_2$ (2.0 mL) was treated with $Et_3N$ (71 μL, 0.51 mmol) and benzene-1,3-disulfonyl chloride (23 mg, 0.085 mmol). The mixture was stirred at room temperature overnight. The resulting yellow solution was then impregnated on silica gel and evaporated to dryness. Chromatography (silica gel, 50% EtOAc/hexanes) afforded **36** (64 mg, 61 %) as a white solid. mp 58-60 °C; $^1H$ NMR ($CDCl_3$, 300 MHz) (single diastereomer, mixture of rotamers) 8.39 (d, J = 6.3 Hz, 1 H), 8.03 (dd, J = 7.8, 1.6 Hz, 2 H), 7.57 (td, J = 7.9, 4.4 Hz, 1 H), 7.35-6.80 (m, 18 H), 5.81 (m, 2 H), 5.50 (m, 2 H), 5.36 (d, J = 4.4 Hz, 2 H), 3.95 (m, 4 H), 3.43 (d, J = 12.6 Hz, 2 H), 3.22 (m, 6 H), 2.65 (m, 4 H), 2.43 (d, J = 13.6 Hz, 2 H), 2.30 (m, 2 H), 2.15 (m, 2 H), 1.95 (m, 4 H), 1.90-1.65 (m, 12 H), 1.50 (m, 4 H), 1.25 (s, 6 H), 1.23 (s, 6 H), 0.90 (t, J = 7.4 Hz, 6 H); $^{13}C$ NMR ($CDCl_3$, 75MHz) (single diastereomer,

mixture of rotamers) 208.5, 170.1, 167.8, 159.0, 142.1, 141.9, 141.3, 131.1, 130.5, 130.1, 128.9, 128.7, 126.5, 125.8, 119.5, 114.8, 112.7, 65.7, 57.2, 51.8, 47.1, 44.6, 41.2, 38.4, 32.9, 32.8, 32.1, 29.5, 26.8, 25.3, 23.9, 23.4, 21.6, 9.2, 9.1. MS(FAB): $(M+Na)^+$ 1269.

Example 7. *N,N'-Bis*[3-(3-((1*R*)-1-((2*S*)-(3,3-dimethyl-1,2-dioxopentyl)piperidine-2-carbonyloxo)-3-phenyl) propylphenoxy)propyl] 5-aminobenzene-1,3-dicarboxamide (**37**)

A mixture of 5-aminoisophthalic acid (1.81 g, 10 mmol) and dioxane (60 mL) was treated with a solution of $Na_2CO_3$ (4.24 g, 40 mmol) in water (60 mL) and then with $(Boc)_2O$ (3.5 mL, 15 mmol). The mixture was stirred at room temperature for 16 h. EtOAc (100 mL) was added to the mixture and 10% $KHSO_4$ (*ca*. 100 mL) added to bring the pH to 2. The organic layer was separated and the aqueous layer was extracted with EtOAc (2 x 100 mL). The combined EtOAc solution was washed with saturated brine, dried ($Na_2SO_4$), and concentrated *in vacuo* to give 5-*tert*-butyloxycarbamyl-benzene-1,3-dicarboxylic acid (2.8 g, 100%).

A mixture of the above diacid (422 mg, 1.5 mmol) and disuccinimidyl carbonate (768 mg, 3.0 mmol) in acetonitrile (20 mL) was treated with pyridine (364 μL, 4.5 mmol). The mixture was stirred vigorously at room temperature for 20 h. The resulting suspension was partitioned between EtOAc (150 mL) and 0.5 N HCl (50 mL). The organic layer was separated and then washed with water (50 mL), 10% $NaHCO_3$ (2 x 50 mL), saturated brine, dried ($Na_2SO_4$), and concentrated *in vacuo*. Chromatography (silica gel, 70%EtOAc/hexanes) afforded disuccinimidyl (5-*tert*-butyloxycarbamyl)benzene-1,3-dicarboxylate (193 mg, 27%) as a white solid. $^1H$ NMR ($CDCl_3$, 300 MHz) 8.50 (s, 1 H), 8.44 (s, 2 H), 6.91 (s, 1 H), 2.89 (s, 8 H), 1.54 (s, 9 H).

To a solution of **24** (81 mg, 0.127 mmol) in $CH_2Cl_2$ (2.0 mL) was added the above activated diester (30 mg, 0.064 mmol), followed by dropwise addition of $Et_3N$ (53 μL, 0.38 mmol). The mixture was stirred at room temperature for 4 h. The resulting clear solution was impregnated on silica gel and evaporated to dryness. Chromatography (silica gel, 50 - 70% EtOAc/hexanes) provided *N*-Boc-**37** (56 mg, 68%) as a white solid. $^1H$ NMR ($CDCl_3$, 300 MHz) (single diastereomer, mixture of rotamers) 8.01 (s, 2 H), 7.93 (s, 1 H), 7.35-6.85 (m, 21 H), 5.83 (t, J = 6.0 Hz, 2 H), 5.34 (d, J = 4.6 Hz, 2 H), 4.14 (t, J = 5.2 Hz, 4 H), 3.70 (m, 4 H), 3.42 (d, J = 12.8 Hz, 2 H), 3.22 (t, J = 10.2 Hz, 2 H), 2.65 (m, 4 H), 2.40 (d, J = 13.0 Hz, 2 H), 2.30 (m, 2 H), 2.15 (m, 6 H), 1.85-1.65 (m, 10 H), 1.57 (s, 9 H), 1.50 (m, 4 H), 1.25 (s, 6 H), 1.23 (s, 6 H), 0.91 (t, J = 7.4 Hz, 6 H); $^{13}C$ NMR ($CDCl_3$, 75MHz) (single diastereomer, mixture of rotamers) 208.4, 170.1, 167.8, 166.8, 159.3, 153.1, 141.7, 141.3, 139.9, 136.1, 130.1, 128.8, 128.7, 126.5, 119.7, 119.4, 115.0, 112.8, 66.7, 51.7, 47.1, 44.5, 38.4, 38.2, 32.8, 32.1, 29.4, 28.7, 26.8, 25.3, 23.4, 21.5, 9.1.

A solution of *N*-Boc-**37** (20 mg, 0.016 mmol) in $CH_2Cl_2$ (4.0 mL) was treated with trifluoroacetic acid (0.8 mL) and the mixture was stirred at room temperature for 1 h. The mixture was diluted with toluene (150 mL) and concentrated *in vacuo* to give **37** trifluoroacetic acid salt (20 mg, 96%) as a colorless gum. $^1H$ NMR ($CDCl_3$, 300 MHz) (single diastereomer, mixture of rotamers) 7.67 (s, 1 H), 7.45-6.90 (m, 22 H), 5.88 (m, 2 H), 5.40 (d, J = 4.6 Hz, 2 H), 4.80 (br. s, 4 H), 4.20 (m, 4 H), 3.75 (m, 4 H), 3.45 (d, J = 12.7 Hz, 2 H), 3.32 (m, 2 H), 2.75 (m, 4 H), 2.50-2.30 (m, 4 H), 2.20 (m, 6 H), 1.78 (m, 10 H), 1.50 (m, 4 H), 1.32 (s, 6 H), 1.30 (s, 6 H), 0.98 (t, J = 7.4 Hz, 6 H). MS(FAB): $(M+H)^+$ 1190.

Example 8. *N,N'-Bis*[3-(3-((1*R*)-1-((2*S*)-(3,3-dimethyl-1,2-dioxopentyl)piperidine-2-carbonyloxo)-3-phenyl) propylphenoxy)propyl] (±)-2,6-diaminopimelamide (**38**)

Following the same procedures as in Example 7 except replacing (±)-2,6-diaminopimelic acid for 5-aminoisophthalic acid, obtained di-Boc-**38** (51 mg, 54%) as a white solid. $^1H$ NMR ($CDCl_3$, 300 MHz) (single diastereomer, mixture of rotamers) 7.35-6.50 (m, 20 H), 5.84 (t, J = 5.8 Hz, 2 H), 5.45-5.20 (m, 4 H), 4.08 (t, J = 5.4 Hz, 4 H), 3.55-3.30 (m, 6 H), 3.24 (t, J = 12.5 Hz, 2 H), 2.70 (m, 4 H), 2.42 (d, J = 13.0 Hz, 2 H), 2.35 (m, 2 H), 2.20 (m, 2 H), 2.05 (m, 4 H), 2.00-1.65 (m, 14 H), 1.50 (m, 4 H), 1.47 (s, 18 H), 1.28 (s, 6 H), 1.25 (s, 6 H), 0.94 (t, J = 7.4 Hz, 6 H); $^{13}C$ NMR ($CDCl_3$, 75MHz) (single diastereomer, mixture of rotamers) 208.3, 170.1, 159.3, 141.8, 141.3, 130.1, 128.9, 128.7, 126.5, 119.3, 113.1, 80.4, 66.7, 51.7, 47.1, 44.5, 38.3, 32.8, 32.1, 29.5, 28.74, 28.72, 26.9, 25.3, 24.0, 23.4, 21.6, 9.1.

A solution of di-Boc-**38** (20 mg, 0.014 mmol) in $CH_2Cl_2$ (4.0 mL) was treated with trifluoroacetic acid (0.8 mL) and the mixture was stirred at room temperature for 1 h. The mixture was diluted with toluene (150 mL) and concentrated *in vacuo* to give **38** di-(trifluoroacetic acid) salt (18.9 mg, 94%) as a colorless gum. $^1H$ NMR ($CDCl_3$, 300 MHz) (single diastereomer, mixture of rotamers) 7.40-6.85 (m, 20 H), 5.85 (m, 2 H), 5.38 (m, 2 H), 4.05 (m, 6 H), 3.45-3.25 (m, 8 H), 2.70 (m, 4 H), 2.45 (m, 2 H), 2.40 (m, 2 H), 2.20 (m, 2 H), 2.05 (m, 4 H), 1.95-1.60 (m, 14 H), 1.50 (m, 4 H), 1.28 (s, 6 H), 1.27 (s, 6 H), 0.95 (t, J = 7.4 Hz, 6 H). MS(FAB): $(M+H)^+$ 1199.

Example 9. *N,N'-Bis*[3-(3-((1*R*)-1-((2*S*)-(3,3-dimethyl-1,2-dioxopentyl)piperidine-2-carbonyloxo)-3-phenyl) propylphenoxy)propyl] triethyleneglycol-1,10-biscarbamate (**39**)

To a solution of **24** (85 mg, 0.13 mmol) in $CH_2Cl_2$ at 0 °C was added $Et_3N$, followed by triethylene glycol bis(chloroformate). The mixture was stirred at 0 °C for 1 h, and TLC showed no starting material left. The mixture was concentrated *in vacuo* and the residue was chromatographed on silica (70-80% EtOAc/hexanes) to give **39** as a colorless gum, 40 mg (48%). $^1H$ NMR ($CDCl_3$, 300 MHz) (single diastereomer, mixture of rotamers) 7.25-6.70 (m, 18 H), 5.71 (t, J = 5.8 Hz, 2 H), 5.25 (d, J = 4.7 Hz, 2 H), 5.12 (br. s., 2 H), 4.15 (t, J = 4.4 Hz, 4 H), 3.95 (t, J = 5.9 Hz, 4 H), 3.60 (t, J = 4.8 Hz, 4 H), 3.57 (s, 4 H), 3.30 (m, 6 H), 3.10 (td, J = 12.7, 3.0 Hz, 2 H), 2.50 (m, 4 H), 2.30 (d, J = 13.7 Hz, 2 H), 2.20 (m, 2 H), 2.05 (m, 2 H), 1.92 (t, J = 6.2 Hz, 4 H), 1.75-1.50 (m, 10 H), 1.35 (m, 4H), 1.16 (s, 6 H), 1.13 (s, 6 H), 0.81 (t, J = 7.4 Hz, 6 H); $^{13}C$ NMR ($CDCl_3$, 75MHz) (single diastereomer, mixture of rotamers) 208.2, 170.1, 167.6, 159.3, 159.9, 156.9, 141.7, 141.3, 130.1, 128.9, 128.7, 126.5, 119.4, 114.8, 113.2, 71.0, 70.1, 66.0, 64.3, 51.7, 47.1, 44.5, 39.2, 38.8, 38.3, 33.0, 32.9, 32.1, 29.9, 26.8, 25.4, 23.9, 23.8, 23.5, 21.6, 9.1. MS(FAB): $(M+Na)^+$ 1269.

Example 10. 1,4-Xylyldiamine *N,N'-bis*[2-(3-((1*R*)-1-((2*S*)-(3,3-dimethyl-1,2-dioxopentyl)piperidine-2-carbonyloxo)-3-phenyl)propy)phenoxyacetamide] (**40**)

A solution of carboxylic acid **25** (104 mg, 0.20 mmol) in acetonitrile (2.0 mL) was treated with disuccinimidyl carbonate (56 mg, 0.22 mmol) and pyridine (48 µL, 0.60 mmol). The mixture was stirred at room temperature overnight. The mixture was then partitioned between EtOAc (70 mL) and water (50 mL). The organic phase was separated, washed with saturated brine, dried ($Na_2$ $SO_4$), and concentrated *in vacuo* to give a white foam (115 mg, 93%). The activated succinimidyl ester was redissolved in anhydrous acetonitrile (2.0 mL). The solution was then treated with triethylamine (75 µL, 0.54 mmol) followed by a solution of 1,4-xylyldiamine in DMF (0.32 M, 288 µL, 0.092 mmol). The resulting suspension was stirred at room temperature for 1 h and TLC showed no starting material left. The mixture was partitioned between EtOAc (50 mL) and water (20 mL). The organic layer was separated, washed with 0.5 N HCl (aq.), saturated brine, dried ($Na_2SO_4$) and concentrated *in vacuo*. Chromatography (silica gel, 70% EtOAc/hexanes) afforded **40** (42 mg, 40%) as a white solid: mp 59-61 °C; $^1H$ NMR ($CDCl_3$, 300 MHz) (single diastereomer, mixture of rotamers) 7.25-6.70 (m, 22 H), 5.69 (m, 2 H), 5.22 (d, J = 4.8 Hz, 2 H), 4.46 (s, 4 H), 4.43 (d, J = 3.9 Hz, 4 H), 3.27 (d, J = 13.2 Hz, 2 H), 3.06 (td, J = 12.6, 2.6 Hz, 2 H), 2.50 (m, 4 H), 2.27 (d, J = 13.4 Hz, 2 H), 2.16 (m, 2 H), 2.00 (m, 2 H), 1.75-1.50 (m, 10 H), 1.35 (m, 4 H), 1.12 (s, 6 H), 1.10 (s, 6 H), 0.78 (t, J = 7.4 Hz, 6 H); $^{13}C$ NMR ($CDCl_3$, 75MHz) (single diastereomer, mixture of rotamers) 208.3, 170.0, 168.4, 168.3, 167.6, 157.7, 142.2, 142.1, 141.1, 137.6, 130.4, 128.9, 128.7, 128.5, 126.6, 120.6, 114.3, 113.8, 67.7, 57.0, 51.6, 47.1, 44.5, 43.0, 39.2, 38.4, 38.1, 32.9, 32.1, 32.0, 26.8, 25.3, 23.9, 23.5, 21.6, 9.2. MS(FAB): $(M+Na)^+$ 1169, $(M+H)^+$ 1147.

Example 11. 1,4-*Bis*(3-aminopropyl)piperazine *N,N'-bis*[2-(3-((1*R*)-1-((2*S*)-(3,3-dimethyl-1,2-dioxopentyl)piperidine-2-carbonyloxo)-3-phenyl)propyl)phenoxyacetamide] (**41**)

Following the same method as in Example 10 except replacing 1,4-bis(3-aminopropyl)piperazine for 1,4-xylyldiamine, obtained **41** (35 mg, 53%) as a white solid. $^1H$ NMR ($CDCl_3$, 300 MHz) (single diastereomer, mixture of rotamers) 7.55-6.90 (m, 20 H), 5.93 (t, J = 5.6 Hz, 2 H), 5.46 (d, J = 4.8 Hz, 2 H), 4.63 (s, 4 H), 3.70-3.50 (m, 6 H), 3.37 (m, 2 H), 2.95 -2.20 (m, 24 H), 1.90 (m, 16 H), 1.60 (m, 4 H), 1.37 (s, 6 H), 1.35 (s, 6 H), 1.03 (t, J = 7.5 Hz, 6 H); $^{13}C$ NMR ($CDCl_3$, 75MHz) (single diastereomer, mixture of rotamers) 208.2, 170.0, 168.4, 167.7, 158.0, 142.3, 141.1, 130.4, 128.9, 128.7, 126.6, 120.5, 115.0, 113.8, 107.9, 68.2, 51.6, 47.1, 44.5, 38.5, 32.9, 26.8, 25.3, 23.9, 23.5, 21.6, 9.1. MS(FAB): $(M+H)^+$ 1211.

Example 12. 3,3'-Diamino-*N*-methyldipropylamine *N,N'-bis*[2-(3-((1*R*)-1-((2*S*)-(3,3-dimethyl-1,2-dioxopentyl) piperidine-2-carbonyloxo)-3-phenyl)propy)phenoxyacetamide] (**42**)

Following the same method as in Example 10 except replacing 3,3'-Diamino-*N*-methyldipropylamine for 1,4-xylyldiamine, obtained **42** (28 mg, 48%) as a colorless gum. $^1H$ NMR ($CDCl_3$, 300 MHz) (single diastereomer, mixture of rotamers) 7.50-6.75 (m, 20 H), 5.76 (t, J = 5.8 Hz, 2 H), 5.29 (d, J = 4.8 Hz, 2 H), 4.45 (s, 4 H), 3.35 (m, 6 H), 3.17 (td, J = 12.6, 2.7 Hz, 2 H), 2.60 (m, 4 H), 2.35 (m, 6 H), 2.25 (m, 2 H), 2.05 (m, 5 H), 1.70 (m, 14 H), 1.40 (m, 4 H), 1.20 (s, 6 H), 1.18 (s, 6 H), 0.86 (t, J = 7.5 Hz, 6 H); $^{13}C$ NMR ($CDCl_3$, 75MHz) (single diastereomer, mixture of rotamers) 208.2, 170.0, 168.4, 167.7, 158.0, 142.2, 141.1, 130.4, 128.9, 128.7, 126.5, 120.3, 115.0, 114.6, 113.7, 108.0, 67.9, 56.4, 51.6, 47.1, 44.5, 38.4, 33.0, 32.9, 32.1, 26.8, 25.3, 23.9, 23.5, 21.6, 21.4, 9.1. MS(FAB): $(M+H)^+$ 1156, $(M+Na)^+$ 1178.

Example 13. 1,5-Diaminopentane *N,N'-bis*[2-(3-((1*R*)-1-((2*S*)-(3,3-dimethyl-1,2-dioxopentyl)piperidine-2-carbonyloxo)-3-phenyl)propyl)phenoxyacetamide] (**43**)

Following the same method as in Example 10 except replacing 1,5-diaminopentane for 1,4-xylyldiamine, obtained **43** (18 mg, 30%) as a colorless gum. $^1H$ NMR ($CDCl_3$, 300 MHz) (single diastereomer, mixture of rotamers)

7.40-7.00 (m, 18 H), 6.80 (br. s., NHs, 2 H), 5.85 (m, 2 H), 5.33 (d, J = 4.7 Hz, 2 H), 4.50 (s, 4 H), 3.37 (m, 6 H), 3.20 (td, J = 12.7, 2.7 Hz, 2 H), 2.65 (m, 4 H), 2.38 (d, J = 13.4 Hz, 2 H), 2.28 (m, 2 H), 2.14 (m, 2 H), 1.90-1.40 (m, 20 H), 1.24 (s, 6 H), 1.22 (s, 6 H), 0.90 (t, J = 7.4 Hz, 6 H); $^{13}$C NMR ($CDCl_3$, 75MHz) (single diastereomer, mixture of rotamers) 208.2, 170.0, 168.3, 167.7, 157.8, 142.2, 141.2, 130.4, 128.9, 128.7, 126.5, 120.5, 114.2, 113.9, 67.8, 51.6, 47.1, 44.5, 39.2, 38.4, 32.9, 32.0, 29.6, 26.8, 25.4, 24 4, 23.9, 23.5, 21.6, 9.1. MS(FAB): $(M+Na)^+$ 1135.

Example 14. 1,5-Diamino-3-oxapetane *N,N'*-*bis*[2-(3-((1*R*)-1-((2*S*)-(3,3-dimethyl-1,2-dioxopentyl)piperidine-2-carbonyloxo)-3-phenyl)propyl)phenoxyacetamide] (**48**)

Following the same method as in Example 10 except replacing 1,5-diamino-3-oxapentane dihydrochloride (Aldrich) for 1,4-xylyldiamine, obtained **48** (23 mg, 39%) as a colorless gum. $^1$H NMR ($CDCl_3$, 300 MHz) (single diastereomer, mixture of rotamers) 7.30-6.80 (m, 20 H), 5.80 (m, 2 H), 5.30 (d, J = 4.9 Hz, 2 H), 4.48 (s, 4 H), 3.50 (br. s, 8 H), 3.36 (d, J = 13.6 Hz, 2 H), 3.16 (td, J = 12.6, 2.7 Hz, 2 H), 2.60 (m, 4 H), 2.36 (d, J = 13.8 Hz, 2 H), 2.26 (m, 2 H), 2.10 (m, 2 H), 1.80-1.60 (m, 10 H), 1.50 (m, 4 H), 1.20 (s, 6 H), 1.19 (s, 6 H), 0.87 (t, J = 7.5 Hz, 6 H); $^{13}$C NMR ($CDCl_3$, 75MHz) (single diastereomer, mixture of rotamers) 208.2, 170.0, 168.5, 167.7, 157.8, 142.2, 141.1, 130.4, 128.9, 128.7, 126.5, 120.5, 114.6, 113.7, 108.1, 69.9, 67.8, 51.6, 47.1, 44.5, 39.1, 38.4, 32.9, 32.0, 26.8, 25.3, 23.9, 23.5, 21.6, 21.4, 9.1. MS(FAB): $(M+Na)^+$ 1137.

Example 15. 1,8-Diamino-3,6-dioxaoctane *N,N'*-*bis*[2-(3-((1*R*)-1-((2*S*)-(3,3-dimethyl-1,2-dioxopentyl)piperidine-2-carbonyloxo)-3-phenyl)propyl)phenoxyacetamide] (**53**)

Following the same method as in Example 10 except replacing 1,8-diamino-3,6-dioxaoctane (Fluka) for 1,4-xylyldiamine, obtained 53 (23 mg, 32%) as a colorless gum. $^1$H NMR ($CDCl_3$, 300 MHz) (single diastereomer, mixture of rotamers) 7.35-6.85 (m, 20 H), 5.80 (t, J = 5.7 Hz, 2 H), 5.33 (d, J = 4.9 Hz, 2 H), 4.51 (s, 4 H), 3.60 (br. s, 12 H), 3.40 (d, J = 12.3 Hz, 2 H), 3.20 (td, J = 12.6, 2.8 Hz, 2 H), 2.65 (m, 4 H), 2.40 (d, J = 13.4 Hz, 2 H), 2.26 (m, 2 H), 2.10 (m, 2 H), 1.90-1.60 (m, 10 H), 1.50 (m, 4 H), 1.25 (s, 6 H), 1.22 (s, 6 H), 0.90 (t, J = 7.4 Hz, 6 H); $^{13}$C NMR ($CDCl_3$, 75MHz) (single diastereomer, mixture of rotamers) 208.2, 170.0, 168.4, 167.7, 157.8, 142.2, 141.1, 130.4, 128.9, 128.7, 126.5, 120.5, 114.7, 113.6, 70.7, 70.1, 67.8, 51.6, 47.1, 44.5, 39.2, 38.4, 32.9, 32.0, 26.8, 25.3, 23.9, 23.5, 21.6, 21.4, 9.1. MS(FAB): $(M+Na)^+$ 1181.

Example 16. 1,11-Diamino-3,6,9-trioxaundecane *N,N'*-*bis*[2-(3-((1*R*)-1-((2*S*)-(3,3-dimethyl-1,2-dioxopentyl)piperidine-2-carbonyloxo)-3-phenyl)propyl)phenoxyacetamide] (**59**)

Following the same method as in Example 10 except replacing 1,11-Diamino-3,6,9-trioxaundecane (prepared using literature procedure of Dietrich, B.; Lehn, J.-M.; Sauvage, J.P.; Blanzat, J. *Tetrahedron,* **1973**, *29*, 1628) for 1,4-xylyldiamine, obtained 59 (18 mg, 24%) as a colorless gum. $^1$H NMR ($CDCl_3$, 300 MHz) (single diastereomer, mixture of rotamers) 7.35-6.80 (m, 20 H), 5.77 (t, J = 6.0 Hz, 2 H), 5.30 (d, J = 4.9 Hz, 2 H), 4.48 (s, 4 H), 3.60 (m, 16 H), 3.35 (d, J = 13.5 Hz, 2 H), 3.16 (td, J = 12.6, 2.9 Hz, 2 H), 2.65 (m, 4 H), 2.37 (d, J = 13.6 Hz, 2 H), 2.25 (m, 2 H), 2.05 (m, 2 H), 1.80-1.60 (m, 12 H), 1.50 (m, 4 H), 1.21 (s, 6 H), 1.19 (s, 6 H), 0.87 (t, J = 7.4 Hz, 6 H); $^{13}$C NMR ($CDCl_3$, 75MHz) (single diastereomer, mixture of rotamers) 208.2, 170.0, 168.4, 167.7, 157.8, 142.2, 141.1, 130.4, 128.9, 128.7, 126.5, 120.5, 114.7, 113.6, 108.0, 70.9, 70.7, 70.1, 67.8, 51.6, 47.1, 44.5, 39.2, 38.4, 32.9, 32.0, 26.8, 25.3, 23.9, 23.5, 21.6, 9.1. MS(FAB): $(M+Na)^+$ 1125.

Example 17. 1,5-Diaminopentane *N,N'*-*bis*[2-(3-((1*R*)-1-((2*S*)-(3,3-dimethyl-1,2-dioxopentyl)piperidine-2-carbonyloxo)-3-(3,4-dimethoxyphenyl))propyl)phenoxyacetamide] (**44**)

Following the same method as in Example 10 except replacing the acid monomer **26** for **25** and replacing 1,5-diaminopentane for 1,4-xylyldiamine, obtained **44** (58 mg, 49%) as a white foam. $^1$H NMR ($CDCl_3$, 300 MHz) (single diastereomer, mixture of rotamers) 7.35-6.65 (m, 16 H), 5.79 (m, 2 H), 5.33 (d, J = 4.8 Hz, 2 H), 4.49 (s, 4 H), 3.87 (s, 6 H), 3.86 (s, 6 H), 3.35 (m, 6 H), 3.20 (m, 2 H), 2.95 (m, 2 H), 2.60 (m, 4 H), 2.38 (d, J = 13.4 Hz, 2 H), 2.28 (m, 2 H), 2.10 (m, 2 H), 1.90-1.40 (m, 20 H), 1.23 (s, 6 H), 1.22 (s, 6 H), 0.90 (t, J = 7.4 Hz, 6 H); $^{13}$C NMR ($CDCl_3$, 75MHz) (single diastereomer, mixture of rotamers) 208.2, 170.0, 168.3, 168.2, 167.7, 157.8, 149.3, 147.8, 142.3, 133.8, 130.4, 120.6, 114.2, 113.9, 112.2, 111.8, 108.0, 67.8, 56.3, 56.2, 51.6, 47.1, 44.5, 39.2, 38.6, 38.3, 32.9, 31.6, 29.6, 26.8, 25.3, 24.4, 23.8, 23.6, 9.1. MS(FAB): $(M+Na)^+$ 1255.

Example 18. 1,5-Diamino-3-oxapetane *N,N'-bis*[2-(3-((1*R*)-1-((2*S*)-(3,3-dimethyl-1,2-dioxopentyl)piperidine-2-carbonyloxo)-3-(3,4-dimethoxyphenyl))propyl)phenoxyacetamide] (**49**)

Following the same method as in Example 10 except replacing the acid monomer **26** for **25** and replacing 1,5-diamino-3-oxapentane dihydrochloride (Aldrich) for 1,4-xylyldiamine, obtained **49** (73 mg, 62%) as a white foam. $^1H$ NMR ($CDCl_3$, 300 MHz) (single diastereomer, mixture of rotamers) 7.35-6.65 (m, 16 H), 5.79 (m, 2 H), 5.33 (d, J = 4.8 Hz, 2 H), 4.51 (s, 4 H), 3.87 (s, 6 H), 3.86 (s, 6 H), 3.55 (br.s, 8 H), 3.35 (m, 2 H), 3.20 (m, 2 H), 2.60 (m, 4 H), 2.38 (d, J = 13.4 Hz, 2 H), 2.28 (m, 2 H), 2.10 (m, 2 H), 1.90-1.40 (m, 20 H), 1.23 (s, 6 H), 1.22 (s, 6 H), 0.90 (t, J = 7.4 Hz, 6 H); $^{13}C$ NMR ($CDCl_3$, 75MHz) (single diastereomer, mixture of rotamers) 208.2, 170.0, 168.5, 168.4, 167.7, 157.8, 149.3, 147.8, 142.3, 133.7, 130.4, 120.5, 114.6, 113.7, 112.2, 111.8, 69.9, 67.8, 56.3, 56.2, 51.6, 47.1, 44.5, 39.1, 38.6, 32.9, 31.6, 26.8, 25.3, 23.8, 23.6, 21.6, 9.1. MS(FAB): $(M+Na)^+$ 1257.

Example 19. 1,8-Diamino-3,6-dioxaoctane *N,N'-bis*[2-(3-((1*R*)-1-((2*S*)-(3,3-dimethyl-1,2-dioxo-pentyl)piperidine-2-carbonyloxo)-3-(3,4-dimethoxyphenyl))propyl)phenoxyacetamide] (**54**)

Following the same method as in Example 10 except replacing the acid monomer **26** for **25** and replacing 1,8-diamino-3,6-dioxaoctane (Fluka) for 1,4-xylyldiamine, obtained **54** (54 mg, 49%) as a white foam. $^1H$ NMR ($CDCl_3$, 300 MHz) (single diastereomer, mixture of rotamers) 7.35-6.65 (m, 16 H), 5.79 (m, 2 H), 5.33 (d, J = 4.8 Hz, 2 H), 4.50 (s, 4 H), 3.87 (s, 6 H), 3.86 (s, 6 H), 3.59 (br.s, 12 H), 3.35 (m, 2 H), 3.20 (m, 2 H), 2.60 (m, 4 H), 2.38 (d, J = 13.4 Hz, 2 H), 2.28 (m, 2 H), 2.10 (m, 2 H), 1.90-1.40 (m, 20 H), 1.23 (s, 6 H), 1.22 (s, 6 H), 0.90 (t, J = 7.4 Hz, 6 H); $^{13}C$ NMR ($CDCl_3$, 75MHz) (single diastereomer, mixture of rotamers) 208.2, 170.1, 168.4, 167.7, 157.8, 149.3, 147.8, 142.3, 133.7, 130.4, 120.5, 114.7, 113.6, 112.2, 111.8, 70.6, 70.1, 67.8, 56.3, 56.2, 51.6, 47.1, 44.5, 39.2, 38.6, 32.9, 31.6, 26.8, 25.3, 23.8, 23.6, 21.6, 9.1. MS(FAB): $(M+Na)^+$ 1301.

Example 20. 1,11-Diamino-3,6,9-trioxaundecane *N,N'-bis*[2-(3-((1*R*)-1-((2*S*)-(3,3-dimethyl-1,2-dioxo-pentyl) piperidine-2-carbonyloxo)-3-(3,4-dimethoxyphenyl))propyl)phenoxy-acetamide] (**60**)

Following the same method as in Example 10 except replacing the acid monomer **26** for **25** and replacing 1,11-Diamino-3,6,9-trioxaundecane (prepared using literature procedure of Dietrich, B.; Lehn, J.-M.; Sauvage, J.P.; Blanzat, J. *Tetrahedron,* **1973**, *29*, 1628) for 1,4-xylyldiamine, obtained **60** (64 mg, 50%) as a colorless gum. $^1H$ NMR ($CDCl_3$, 300 MHz) (single diastereomer, mixture of rotamers) 7.35-6.65 (m, 16 H), 5.79 (m, 2 H), 5.33 (d, J = 4.8 Hz, 2 H), 4.50 (s, 4 H), 3.87 (s, 6 H), 3.86 (s, 6 H), 3.61 (m, 16 H), 3.38 (m, 2 H), 3.20 (m, 2 H), 2.60 (m, 4 H), 2.38 (m, 2 H), 2.28 (m, 2 H), 2.10 (m, 2 H), 1.90-1.40 (m, 20 H), 1.23 (s, 6 H), 1.22 (s, 6 H), 0.90 (t, J = 7.4 Hz, 6 H); $^{13}C$ NMR ($CDCl_3$, 75MHz) (single diastereomer, mixture of rotamers) 208.2, 170.1, 168.4, 167.7, 157.8, 149.3, 147.8, 142.3, 133.7, 130.4, 120.5, 114.7, 113.6, 112.2, 111.8, 108.0, 70.6, 70.1, 67.8, 56.3, 56.2, 51.6, 47.1, 44.5, 39.2, 38.6, 32.9, 31.6, 26.8, 25.3, 23.8, 23.5, 21.6, 9.1. MS(FAB): $(M+Na)^+$ 1345.

Example 21. 1,5-Diaminopentane *N,N'-bis*[2-(3-((1*R*)-1-((2*S*)-(3,3-dimethyl-1,2-dioxopentyl)piperidine-2-carbonyloxo)-3-(3,4,5-tnmethoxyphenyl))propyl)phenoxyacetamide] (**45**)

Following the same method as in Example 10 except replacing the acid monomer **27** for **25** and replacing 1,5-diaminopentane for 1,4-xylyldiamine, obtained **45** (33 mg, 34%) as a white foam. $^1H$ NMR ($CDCl_3$, 300 MHz) and $^{13}C$ NMR ($CDCl_3$, 75MHz) are correct. MS(FAB): $(M+Na)^+$ 1315.

Example 22. 1,5-Diamino-3-oxapetane *N,N'-bis*[2-(3-((1*R*)-1-((2*S*)-(3,3-dimethyl-1,2-dioxopentyl)piperidine-2-carbonyloxo)-3-(3,4,5-trimethoxyphenyl))propyl)phenoxyacetamide] (**50**)

Following the same method as in Example 10 except replacing the acid monomer **27** for **25** and replacing 1,5-diamino-3-oxapentane dihydrochloride (Aldrich) for 1,4-xylyldiamine, obtained **50** (41 mg, 46%) as a white foam. $^1H$ NMR ($CDCl_3$, 300 MHz) and $^{13}C$ NMR ($CDCl_3$, 75MHz) are correct. MS(FAB): $(M+Na)^+$ 1317.

Example 23. 1,8-Diamino-3,6-dioxaoctane *N,N'-bis*[2-(3-((1*R*)-1-((2*S*)-(3,3-dimethyl-1,2-dioxopentyl)piperidine-2-carbonyloxo)-3-(3,4,5-trimethoxyphenyl))propyl)phenoxyacetamide] (**55**)

Following the same method as in Example 10 except replacing the acid monomer **27** for **25** and replacing 1,8-diamino-3,6-dioxaoctane (Fluka) for 1,4-xylyldiamine for 1,4-xylyldiamine, obtained **55** (37 mg, 38%) as a white foam. $^1H$ NMR ($CDCl_3$, 300 MHz) and $^{13}C$ NMR ($CDCl_3$, 75MHz) are correct. MS(FAB): $(M+Na)^+$ 1361.

Example 24. 1,11-Diamino-3,6,9-trioxaundecane *N,N'-bis*[2-(3-((1*R*)-1-((2*S*)-(3,3-dimethyl-1,2-dioxopentyl)piperidine-2-carbonyloxo)-3-(3,4,5-trimethoxyphenyl))propyl)phenoxy-acetamide] (**61**)

Following the same method as in Example 10 except replacing the acid monomer **27** for **25** and replacing 1,11-Diamino-3,6,9-trioxaundecane for 1,4-xylyldiamine, obtained **61** (27 mg, 32%) as a white foam. $^{1}H$ NMR ($CDCl_3$, 300 MHz) and $^{13}C$ NMR ($CDCl_3$, 75MHz) are correct. MS(FAB): $(M+Na)^+$ 1405.

Example 25. 1,5-Diaminopentane *N,N'-bis*[2-(3-((1*R*)-1-((2*S*)-(3,3-dimethyl-1,2-dioxopentyl)piperidine-2-carbonyloxo)-3-(3,4-methylenedioxyphenyl))propyl)phenoxyacetamide] (**46**)

Following the same method as in Example 10 except replacing the acid monomer **28** for **25** and replacing 1,5-diaminopentane for 1,4-xylyldiamine, obtained **46** (42 mg, 42%) as a white foam. $^{1}H$ NMR ($CDCl_3$, 300 MHz) and $^{13}C$ NMR ($CDCl_3$, 75MHz) are correct. MS(FAB): $(M+Na)^+$ 1223.

Example 26. ,5-Diammo-3-oxapetane *N,N'-bis*[2-(3-((1*R*)-1-((2*S*)-(3,3-dimethyl-1,2-dioxopentyl)piperidine-2-carbonyloxo)-3-(3,4-methylenedioxyphenyl))propyl)phenoxyacetamide] (**51**)

Following the same method as in Example 10 except replacing the acid monomer **28** for **25** and replacing 1,5-diamino-3-oxapentane dihydrochloride (Aldrich) for 1,4-xylyldiamine, obtained **51** (39 mg, 34%) as a white foam. $^{1}H$ NMR ($CDCl_3$, 300 MHz) and $^{13}C$ NMR ($CDCl_3$, 75MHz) are correct. MS(FAB): $(M+Na)^+$ 1225.

Example 27. 1,8-Diamino-3,6-dioxaoctane *N,N'-bis*[2-(3-((1*R*)-1-((2*S*)-(3,3-dimethyl-1,2-dioxopentyl)piperidine-2-carbonyloxo)-3-(3,4-methylenedioxyphenyl))propyl)phenoxyacetamide] (**56**)

Following the same method as in Example 10 except replacing the acid monomer **28** for **25** and replacing 1,8-diamino-3,6-dioxaoctane (Fluka) for 1,4-xylyldiamine for 1,4-xylyldiamine, obtained 56 (55 mg, 47%) as a white foam. $^{1}H$ NMR ($CDCl_3$, 300 MHz) and $^{13}C$ NMR ($CDCl_3$, 75MHz) are correct. MS(FAB): $(M+Na)^+$ 1269.

Example 28. 1,11-Diamino-3,6,9-trioxaundecane *N,N'-bis*[2-(3-((1*R*)-1-((2*S*)-(3,3-dimethyl-1,2-dioxopentyl)piperidine-2-carbonyloxo)-3-(3,4-methylenedioxyphenyl))propyl)phenoxy-acetamide] (**62**)

Following the same method as in Example 10 except replacing the acid monomer **28** for **25** and replacing 1,11-Diamino-3,6,9-trioxaundecane for 1,4-xylyldiamine, obtained **62** (52 mg, 42%) as a colorless gum. $^{1}H$ NMR ($CDCl_3$, 300 MHz) and $^{13}C$ NMR ($CDCl_3$, 75MHz) are correct. MS(FAB): $(M+Na)^+$ 1313.

Example 29. 1,5-Diaminopentane *N,N'-bis*[2-(3-((1*R*)-1-((2*S*)-(3,3-dimethyl-1,2-dioxopentyl)piperidine-2-carbonyloxo)-3-(3-pyridyl))propyl)phenoxyacetamide] (**47**)

Following the same method as in Example 10 except replacing the acid monomer **29** for **25** and replacing 1,5-diaminopentane for 1,4-xylyldiamine, obtained **47** (64 mg, 58%) as a white foam. $^{1}H$ NMR ($CDCl_3$, 300 MHz) and $^{13}C$ NMR ($CDCl_3$, 75MHz) are correct. MS(FAB): $(M+Na)^+$ 1137.

Example 30. 1,5-Diamino-3-oxapetane *N,N'-bis*[2-(3-((1*R*)-1-((2*S*)-(3,3-dimethyl-1,2-dioxopentyl)piperidine-2-carbonyloxo)-3-(3-pyridyl))propyl)phenoxyacetamide] (**52**)

Following the same method as in Example 10 except replacing the acid monomer **29** for **25** and replacing 1,5-diamino-3-oxapentane dihydrochloride (Aldrich) for 1,4-xylyldiamine, obtained **52** (52 mg, 55%) as a white foam. $^{1}H$ NMR ($CDCl_3$, 300 MHz) and $^{13}C$ NMR ($CDCl_3$, 75MHz) are correct. MS(FAB): $(M+Na)^+$ 1139.

Example 31. 1,8-Diamino-3,6-dioxaoctane *N,N'-bis*[2-(3-((1*R*)-1-((2*S*)-(3,3-dimethyl-1,2-dioxopentyl)piperidine-2-carbonyloxo)-3-(3-pyridyl))propyl)phenoxyacetamide] (**57**)

Following the same method as in Example 10 except replacing the acid monomer **29** for **25** and replacing 1,8-diamino-3,6-dioxaoctane (Fluka) for 1,4-xylyldiamine for 1,4-xylyldiamine, obtained **57** (48 mg, 47%) as a white foam. $^{1}H$ NMR ($CDCl_3$, 300 MHz) and $^{13}C$ NMR ($CDCl_3$, 75MHz) are correct. MS(FAB): $(M+Na)^+$ 1183.

Example 32. 1,11-Diamino-3,6,9-trioxaundecane *N,N'-bis*[2-(3-((1*R*)-1-((2*S*)-(3,3-dimethyl-1,2-dioxopentyl)piperidine-2-carbonyloxo)-3-(3-pyridyl))propyl)phenoxyacetamide] (**63**)

Following the same method as in Example 10 except replacing the acid monomer **29** for **25** and replacing 1,11-Diamino-3,6,9-trioxaundecane for 1,4-xylyldiamine, obtained **63** (58 mg, 55%) as a colorless gum. $^1$H NMR ($CDCl_3$, 300 MHz) and $^{13}$C NMR ($CDCl_3$, 75MHz) are correct. MS(FAB): $(M+Na)^+$ 1227.

Example 33. 1,8-Diamino-3,6-dioxaoctane *N,N'-bis*[2-(3-((1*R*)-1-((2*S*)-(3,3-dimethyl-1,2-dioxopentyl)piperidine-2-carbonyloxo)-3-(3-indolyl))propyl)phenoxyacetamide] (**58**)

Following the same method as in Example 10 except replacing the acid monomer **30** for **25** and replacing 1,8-diamino-3,6-dioxaoctane (Fluka) for 1,4-xylyldiamine for 1,4-xylyldiamine, obtained **58** (20 mg, 20%) as a white foam. $^1$H NMR ($CDCl_3$, 300 MHz) and $^{13}$C NMR ($CDCl_3$, 75MHz) are correct. MS(FAB): $(M+Na)^+$ 1259.

Example 34. Ethylenediamine *N,N'-bis*[2-(3-((1*R*)-1-((2*S*)-(3,3-dimethyl-1,2-dioxopentyl)piperidine-2-carbonyloxo)-3-(3,4-dtmethoxyphenyl))propyl)phenoxyacetamide] (**64**)

Following the same method as in Example 10 except replacing the acid monomer **26** for **25** and replacing ethylenediamine dihydrochloride for 1,4-xylyldiamine, obtained **64** (126 mg, 59%) as a white solid. $^1$H NMR ($CDCl_3$, 300 MHz) and $^{13}$C NMR ($CDCl_3$, 75MHz) are correct. MS(FAB): $(M+Na)^+$ 1213.

Example 35. Ethylenediamine *N,N'-bis*[2-(3-((1*R*)-1-((2*S*)-(3,3-dimethyl-1,2-dioxopentyl)piperidine-2-carbonyloxo)-3-(3-pyridyl))propyl)phenokyacetamide] (**65**)

Following the same method as in Example 10 except replacing the acid monomer **29** for **25** and replacing ethylenediamine dihydrochloride for 1,4-xylyldiamine, obtained **65** (79 mg, 42%) as a white solid. $^1$H NMR ($CDCl_3$, 300 MHz) and $^{13}$C NMR ($CDCl_3$, 75MHz) are correct. MS(FAB): $(M+H)^+$ 1073.

Example 36. *N,N'*-Dimethylethylenediamine *N,N'-bis*[2-(3-((1*R*)-1-((2*S*)-(3,3-dimethyl-1,2-dioxopentyl)piperidine-2-carbonyloxo)-3-(3,4-dimethoxyphenyl))propyl)phenoxyacetamide] (**66**)

Following the same method as in Example 10 except replacing the acid monomer **26** for **25** and replacing *N, N'*-dimethylethylenediamine for 1,4-xylyldiamine, obtained **66** (118 mg, 55%) as a white solid. $^1$H NMR ($CDCl_3$, 300 MHz) and $^{13}$C NMR ($CDCl_3$, 75MHz) are correct. MS(FAB): $(M+Na)^+$ 1241.

Example 37. *N,N'*-Dimethylethylenediamine *N,N'-bis*[2-(3-((1*R*)-1-((2*S*)-(3,3-dimethyl-1,2-dioxopentyl)piperidine-2-carbonyloxo)-3-(3-pyridyl))propyl)phenoxyacetamide] (**67**)

Following the same method as in Example 10 except replacing the acid monomer **29** for **25** and replacing *N, N'*-dimethylethylenediamine for 1,4-xylyldiamine, obtained **67** (70 mg, 37%) as a white solid. $^1$H NMR ($CDCl_3$, 300 MHz) and $^{13}$C NMR ($CDCl_3$, 75MHz) are correct. MS(FAB): $(M+H)^+$ 1101.

**Synthetic Overview, part II:**

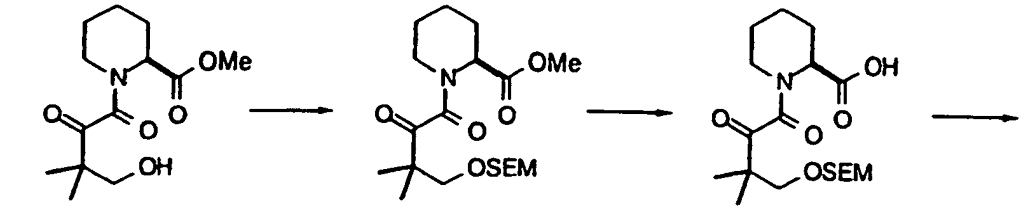

71 72 73

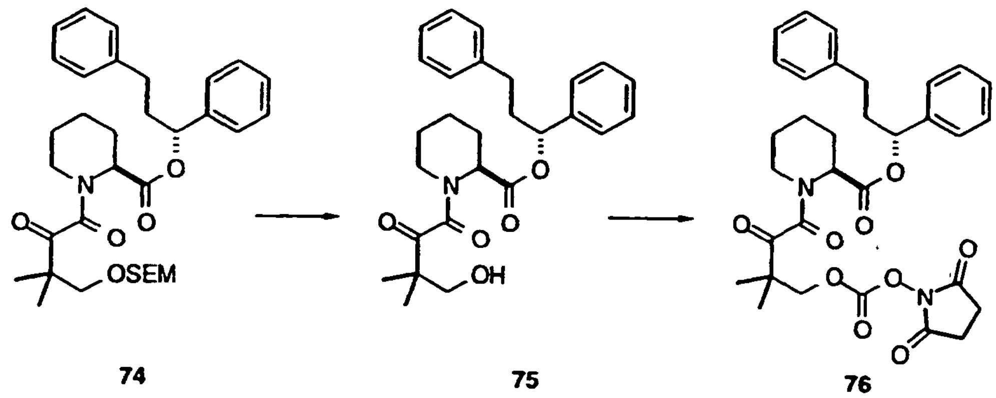

74 75 76

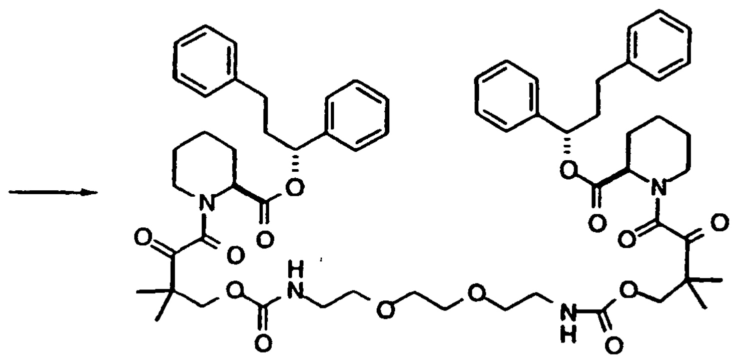

77

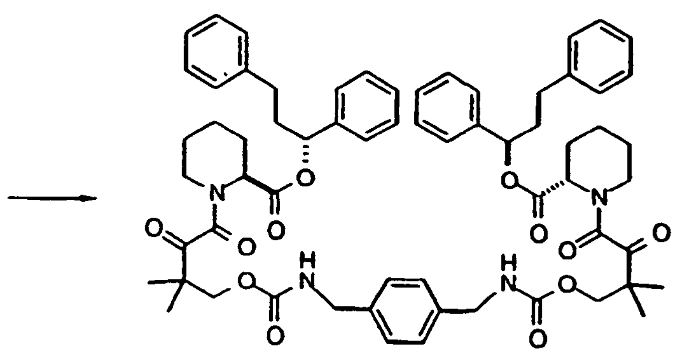

78

**Synthetic Details**

**Methyl (2S)-1-(3,3-dimethyl-1,2-dioxo-4-hydroxy)butyl-2-piperidinecarboxylate, (71)** Prepared according to the procedure reported by D. A. Holt et al., *J. Am, Chem. Soc.* **1993,** *115*, 9925-9938 for the ethyl ester analog.

$^1H$ NMR ($CDCl_3$) δ 5.25 (dist d, J=5.2 Hz, 1H), 3.78 (s, 3H), 3.59-3.71 (m, 2H), 3.49 (br d, J=13.8 Hz, 1H), 3.37 (t, J=6.4 Hz, 1H), 3.18 (td, J=12.9, 3.3 Hz, 1H), 2.32 (br d, J=14.0 Hz, 1H), 1.25-1.80 (m, 5H), 1.23 (s, 6H). MS (DCI/NaI) m/z 289 ($M+NH_4$), 272 (M+H).

**Methyl (2S)-1-{3,3-dimethyl-1,2-dioxo-4-[2-(trimethylsilyl)ethoxy] methoxy}butyl-2-piperidinecarboxylate, (72)**

A solution of methyl (2S)-1-(3,3-dimethyl-1,2-dioxo-4-hydroxy)butyl-2-piperidinecarboxylate, **71** (1.80 g, 6.6 mmol), N,N'-diisopropylethylamine (1.03 g, 8.0 mmol), and 2-(trimethylsilyl)ethoxymethyl chloride (1.33 g, 8.0 mmol) in dichloromethane (25 mL) was stirred at room temperature for 21.5 h. The solution was concentrated and the residue was chromatographed (silica-gel, hexanes-ethyl acetate 10:1 to 6:1 gradient) to give the title compound (2.60 g) as a colorless liquid.

$^1H$ NMR ($CDCl_3$) δ 5.22 (br d, J=5.1 Hz, 1H), 4.62 (s, 2H), 3.73 (s, 3H), 3.49-3.71 (m, 5H), 3.14 (td, J=13.3, 3.4 Hz, 1H), 2.28 (br d, J=14.0 Hz, 1H), 1.18-1.77 (m, 5H), 1.30 (s, 3H), 1.27 (s, 3H), 0.84-0.94 (m, 2H), 0.00 (s, 9H). MS ($FAB^+$/NaI) m/z

**(2S)-1-{3,3-Dimethyl-1,2-dioxo-4-[2-(trimethylsilyl)ethoxy]methoxy}butyl-2-piperidinecarboxylic acid, (73)**

A mixture of methyl (2S)-1-(3,3-dimethyl-1,2-dioxo-4-[2-(trimethylsilyl) ethoxy]methoxy)butyl-2-piperidinecarboxylate, **72** (2.50 g, 6.2 mmol), 1N lithium hydroxide (9.3 mL) and methanol (10 mL) was stirred at 0°C for 30 min and then at room temperature for 7 h. The mixture was acidified with 1N HCl, diluted with water, and extracted with dichloromethane. The organic extract was washed with water, dried over anhydrous sodium sulfate, and concentrated to give a colorless oil (2.11 g) which was used without further purification.

$^1H$ NMR ($CDCl_3$) δ 10.25 (br s, 1H), 5.27 (d, J=4.9 Hz, 1H), 4.61 (s, 2H), 3.68 (dist. t, J=9.4, 9.9 Hz, 1H), 3.49-3.60 (m, 4H), 3.11-3.20 (m, 1H), 2.31 (br d, J=13.7 Hz, 1H), 1.36-1.79 (m, 5H), 1.29 (s, 3H), 1.27 (s, 3H), 0.91 (td, J=8.4, 3.0 Hz, 2H), 0.00 (s, 9H). $^{13}C$ NMR ($CDCl_3$) δ 207.5, 176.9, 168.7, 96.4, 75.0, 66 6, 52.5, 48.8, 45.3, 27.7, 26.2, 23.9 (2 C), 22.6, 19.5, 0.00. MS ($FAB^-$) m/z 386 (M-H)

**(1R)-1,3-Diphenyl-1-propyl (2S)-1-{3,3-dimethyl-1,2-dioxo-4-[2-(trimethylsilyl)ethoxy]methoxy}butyl-2-piperidinecarboxylate, (74)**

A solution of (2S)-1-{3,3-dimethyl-1,2-dioxo-4-[2-(trimethylsilyl)ethoxy] methoxy}butyl-2-piperidinecarboxylic acid, **73** (1.00 g, 2.6 mmol) and (1R)-1,3-diphenyl-1-propanol (0.72 g, 3.4 mmol) in dichloromethane (10 mL) was treated with N,N-dicyclohexylcarbodiimide (0.70 g, 3.4 mmol) and 4-dimethylaminopyridine (0.22 g, 1.8 mmol). The resulting suspension was stirred at room temperature under a nitrogen atmosphere for 17h. The mixture was then diluted with a small amount of ethyl acetate, filtered, and concentrated, and the residue was subjected to column chromatography (silica-gel, hexanes-ethyl acetate 8:1) to afford the title compound (1.33 g) as a colorless oil

$^1H$ NMR ($CDCl_3$) δ 7.14-7.32 (m, 10H), 5.27-5.47 (m, 1H), 5.08 (br d, J=5.2 Hz, 1H), 4.59 (AB q, $J_{AB}$=6.8 Hz, 2H), 3.66 (dd, J=9.2, 8.6 Hz, 1H), 3.48-3.62 (m, 3H), 3.33 (br d, J=13.1 Hz, 1H), 2.70-2.92 (m, 5H), 2.00 (br d, J=11.5 Hz, 1H), 1.21-1.49 (m, 5H), 1.27 (s, 3H), 1.25 (s, 3H), 0.86-0.95 (m, 2H), 0.00 and -0.02 (2xs, 9H). MS ($FAB^+$/NaI) m/z 604 (M+Na). Exact Mass: Calc. (M+Na) for $C_{33}H_{47}NSiO_6$, 604.3070 ; found, 604.3073.

**(1R)-1,3-Diphenyl-1-propyl (2S)-1-(3,3-dimethyl-1,2-dioxo-4-hydroxy)butyl-2-piperidinecarboxylate, (75)**

A solution of (1R)-1,3-diphenyl-1-propyl (2S)-1-{3,3-dimethyl-1,2-dioxo-4-[2-(trimethylsilyl)ethoxy]methoxy} butyl-2-piperidinecarboxylate, **74** (0.75 g, 1.3 mmol) and 48 wt% HF (0.5 mL) in acetonitrile (25 mL) was stirred at room temperature for 4 h, and then partitioned between 10% aqueous sodium bicarbonate and ethyl acetate. The organic layer was decanted, washed with water, dried over anhydrous sodium sulfate, and concentrated, and the residue chromatographed (silica-gel, hexanes-ethyl,acetate 4:1 to 2:1 gradient) to afford **75** (0.45 g) as a colorless oil).

$^1H$ NMR ($CDCl_3$) δ 7.10-7.27 (m, 10H), 5.38-5.47 (m, 1H), 5.04 (br d, J=5.3 Hz, 1H), 3.47-3.621 (m, 3H), 3.29 (br d, J=13.9 Hz, 1H), 2.67-2.93 (m, 5H), 2.00 (br d, J=12.8 Hz, 1H), 1.17-1.57 (m, 5H), 1.15 (s, 3H), 1.14 (s, 3H). MS ($FAB^+$/NaI) m/z 474 (M+Na). Exact Mass: Calc. (M+Na) for $C_{25}H_{33}NO_5$, 474.2256; found, 474.2273.

**(1R)-1,3-Diphenyl-1-propyl (2S)-1-[3,3-dimethyl-1,2-dioxo-4-(1-succinimidyloxycarbonyl)oxy]butyl-2-piperidinecarboxylate, (76)**

A solution of (1R)-1,3-diphenyl-1-propyl (2S)-1-(3,3-dimethyl-1,2-dioxo-4-hydroxy)butyl-2-piperidinecarboxylate, **75** (223 mg, 0.49 mmol) in dichloromethane (13 mL) was treated with N,N-diisopropylethylamine (0.8 mL), and N,N'-disuccinimidyl carbonate (385 mg), and the mixture stirred at room temperature for 66 h. It was then washed with saturated sodium bicarbonate, dried over anhydrous sodium sulfate, and concentrated to afford a yellow oil (280 mg) which was used without further purification.

$^1H$ NMR ($C_6D_6$) δ 7.11-7.32 (m, 10H), 5.56-5.65 (m, 1H), 5.36 (br d, J=5.1 Hz, 1H), 4.51 (d, J=10.5 Hz, 1H), 4.17 (d, J=10.5 Hz, 1H), 3.52 (br d, J=13.4 Hz, 1H), 2.71-3.11 (m, 5H), 1.99 (br d, J=14.4 Hz, 1H), 1.70 (br s, 4H), 1.01-1.38 (m, 11H). $^{13}C$ NMR ($C_6D_6$) δ 205.4, 171.0, 169.7, 168.2, 153.5, 139.2 (139.0), 131.2, 130.2, 130.1, 129.8, 129.5, 129.2, 128.3 (128.2), 77.9, 76.9, 53.1, 48.4, 45.3, 41.9, 41.8, 27.7, 26.6 (2C), 26.3, 23.5, 22.5, 22.3. MS ($FAB^+$/NaI) m/z 615 (M+Na), 474, 434. Exact Mass: Calc. (M+Na) for $C_{32}H_{36}N_2O_9$, 615.2319; found, 615.2299.

**2,2-(Ethylenedioxy)diethylamine N,N'-{2,2-dimethyl-3,4-dioxo-4-{(2S)-2-[(1R)-1,3-diphenylpropyloxycarbonyl]-1-piperidinyl}butylcarbamate, (77)**

A solution of (1R)-1,3-diphenyl-1-propyl-(2S)-1-[3,3-dimethyl-1,2-dioxo-4-(1-succinimidyloxycarbonyl)oxy] butyl-2-piperidinecarboxylate, **76** (75 mg, 0.13 mmol) and N,N-diisopropylethylamine (66.3 μL) in acetonitrile (4 mL) was treated with 2,2'-(ethylenedioxy)diethylamine (9.3 μL), and the mixture stirred at room temperature for 21 h. The solvent was removed and the residue chromatographed (silica-gel, hexanes-ethyl acetate 1:3 to 1:2 gradient) to give the title compound (20 mg) as a colorless oil.

$^1H$ NMR ($CDCl_3$) δ 7.10-7.55 (m, 20H), 5.48 (br dd, J=12.2, 6.1 Hz, 2H), 5.20-5.35 (br s, 2H), 5.09-5.18 (m, 2H), 4.22 (AB q, $J_{AB}$=10.6 Hz, 4H), 3.48-3.80 (m, 8H), 3.20-3.45 (m, 6H), 2.80-3.15 (m, 10H), 1.98-2.08 (m, 2H), 1.17-1.68 (m, 22H). $^{13}C$ NMR ($CDCl_3$) δ 205.3, 170.1, 166.9, 156.6, 137.6, 137.3, 129.7 (2C), 128.9, 128.8, 127.1, 127.0, 76.9, 70.6, 70.4, (69.9), (60.8), (56.9), 51.6, 47.3, (47.2), 44.0, 41.2, 40.7, 40.5, (40.2), (39.0), (28.1), 26.8, 25.2, (24.7), 22.3, (22.2), 21.8, (21.4), 21.0, (20.8), 14.6. MS ($FAB^+$/NaI) m/z 1125 (M+Na).

***p*-Xylylenediamine N,N'-{2,2-dimethyl-3,4-dioxo-4-{(2S)-2-[(1R)-1,3-diphenylpropyloxycarbonyl]-1-piperidinyl} butylcarbamate, (78)**

A solution of *p*-xylylenediamine in dimethylformamide (0.1 mM, 0.5 mL) was added dropwise, over a 30 min-period, to a solution of **76** (66 mg, 0.11 mmol) and triethylamine (46 μL) in acetonitrile (1 mL). The mixture was then partitioned between ethyl acetate and water, and the organic layer was decanted, washed with water, dried over anhydrous sodium sulfate, and concentrated to a yellow oil. Column chromatography (silica-gel, hexanes-ethyl acetate 1:1) afforded the title compound (33 mg) as a colorless oil.

$^1H$ NMR ($C_6D_6$) δ 7.34-7.55 (m, 24H), 5.72-5.85 (m, 2H), 5.64-5.68 (m, 2H), 5.35-5.45 (m, 2H), 4.77 (AB q, $J_{AB}$=10.8 Hz, 4H), 4.46-4.57 (m, 4H), 3.64 (br d, J=12.2 Hz, 2H), 2.92-3.25 (m, 10H), 2.14 (br d, J=13.2 Hz, 2H), 1.20-1.75 (m, 22H). MS ($FAB^+$/NaI) m/z 1113 (M+Na).

<u>Cell-based transfection assay</u>

In general, dimerizer assays were carried out as follows. Human 293 cells were transiently transfected by calcium phosphate procedure with plasmids PCGNNGF3 and PCGNNF3VP16, expressing Gal4 DNA binding domain (aa 1-147) fused to 3 copies of FKBP12 and 3 copies of FKBP12 fused to the VP16 activation domain (aa 411-490), respectively. The reporter plasmid (G5IL2-SEAP) used in these assays contains a gene that encodes for secreted alkaline phosphatase (SEAP) under the control of the minimal IL2 promoter and 5XGAL4 binding sites placed upstream of the promoter. In all cases, a plasmid expressing growth hormone was used as an internal control to moniter transfection efficiency.

Approximately, 16 hrs after transfection, the media was removed and the cells were washed twice with PBS. Cells were refed with 2.5 ml of DMEM containing 10% serum and two hours later, synthetic dimerizers were added directly to the medium at appropriate concentrations in 5ul of ethanol carrier solution. Approximetely, 24 hrs after the addition of the drugs, 100 ul of the media was removed and assayed for SEAP activity and another 100 ul of the media was used to assay for growth hormone activity (to normalize for transfection efficiency).

Results for a sample of our multimerizers in that system are shown below at multimerizer concentrations from 0.1 to $10^4$ nM, as indicated, normalized for hGH expression, and expressed as a % of maximal transcriptional activity observed with the prototype multimerizer, FK1012 (see Spencer et al, Science, supra). It should be appreciated that multimerizers of this invention will vary somewhat in their observed activity, depending on the particular chimeric proteins and other components of such systems. We recommend that the practitioner select multimerizers based on their performance in the particular system of interest.

| Dimerizer Assay Worksheet | | | | | |
|---|---|---|---|---|---|
| Activation Domain: VP16 *Ratio VP16/Gal4:* 10:01 DNA Binding Domain: Gal4 *Reporter Gene:* G5IL2-SEAP | | | | | |
| Cmpd | ***% of FK1012 Max. Activity*** | | | | |
| | ***1*** | ***10*** | ***100*** | ***1,000*** | ***10,000*** |
| FK1012 | 17.38 | 17.24 | 100.00 | 70.87 | |
| 53 | | 3.49 | 6.17 | 12.62 | 55.12 |
| 35 | | 7.87 | 2.91 | 2.76 | 2.62 |
| Cmpd | ***% of FK1012 Max. Activity*** | | | | |
| | ***1*** | ***10*** | ***100*** | ***1,000*** | ***10,000*** |
| FK1012 | | 36.67 | 100.00 | 54.19 | |
| 53 | | 4.79 | 6.42 | 31.09 | 57.67 |
| 37 | | 4.05 | 2.98 | 5.88 | 10.49 |
| 48 | | 5.72 | 6.56 | 24.79 | 47.56 |
| 59 | | 5.56 | 5.28 | 40.72 | 36.02 |
| Cmpd | ***% of FK1012 Max. Activity*** | | | | |
| | ***0.1*** | ***1*** | ***10*** | ***100*** | ***1,000*** |
| FK1012 | 24.28 | 25.67 | 30.74 | 100.00 | |
| 60 | 24.24 | 27.84 | 49.66 | 21.28 | |
| 54 | 26.84 | 30.61 | 57.48 | 29.67 | |
| 49 | 19.74 | 19.26 | 54.90 | 76.59 | |
| 50 | 20.25 | 20.19 | 26.24 | 58.99 | |
| 52 | 23.40 | 25.12 | 31.51 | 56.54 | |
| 47 | 23.26 | 25.15 | 42.75 | 25.14 | |

## Claims

**1.** A multimerizing agent of the formula $M^1$-L-$M^2$, and pharmaceutically acceptable salts thereof, where $M^1$ and $M^2$ are moieties independently selected from the following:

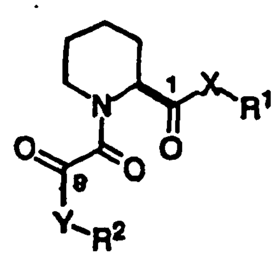

and

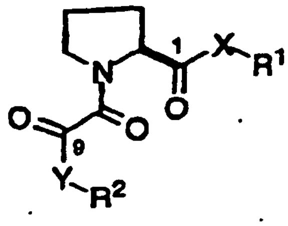

where

X = O, NH or $CH_2$;

Y = O, NH, $NR^3$, or represents a covalent bond from $R^2$ to atom 9; and,

$R^1$, $R^2$, and $R^3$ are independently $C_1$-$C_{20}$ alkyl or aryl;

wherein alkyl comprises saturated or unsaturated, straight chain, branched, cyclic, or polycyclic aliphatic hydrocarbons which may contain oxygen, sulfur, or nitrogen in place of one or more carbon atoms, and which are optionally substituted with one or more functional groups selected from the group consisting of hydroxy, $C_1$-$C_8$ alkoxy, acyloxy, carbamoyl amino, N-acylamino, ketone, halogen, cyano, carboxyl, and aryl;

aryl comprises stable cyclic, heterocyclic, polycyclic, or polyheterocyclic unsaturated $C_3$-$C_{14}$ moieties; which may further be substituted with one to five members selected from the group consisting of hydroxy, $C_1$-$C_8$ alkoxy, $C_1$-$C_8$ branched or straight-chain alkyl, acyloxy, carbamoyl, amino, N-acylamino, nitro, halogen, trifluoromethyl, cyano, and carboxyl;

L is a linker moiety joining monomers $M^1$ and $M^2$ through covalent bonds to either $R^1$ or $R^2$, not necessarily the same in each of $M^1$ and $M^2$; and wherein one of $M^1$ and $M^2$ may optionally be a macrocyclic structure formed by a covalent link between $R^1$ and $R^2$; provided that said multimerizing agent does not have the formula:

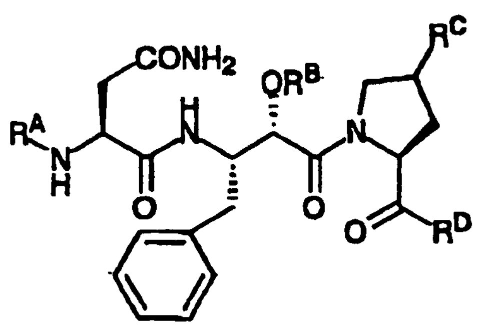

where $R^A$ is carbobenryloxy, $R^B$ and $R^C$ are each MeOCOCO-Pro-(D)Phe-, and $R^D$ is $-NHC(CH_3)_3$.

2. A multimerizing agent of claim 1 in which $M^1$ and $M^2$ are moieties independently selected from the following:

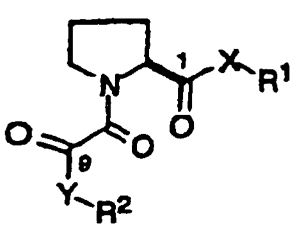

and

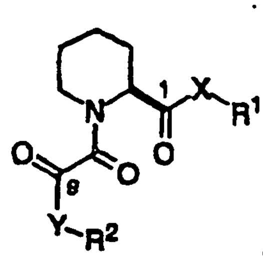

3. A multimerizing agent of claim 1 of the formula:

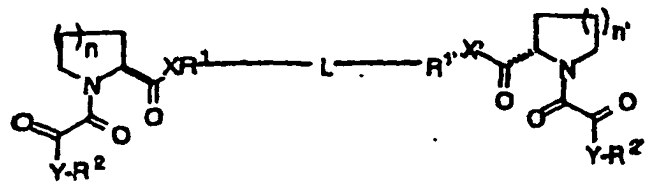

**4.** A multimerizing agent of claim 1 of the formula:

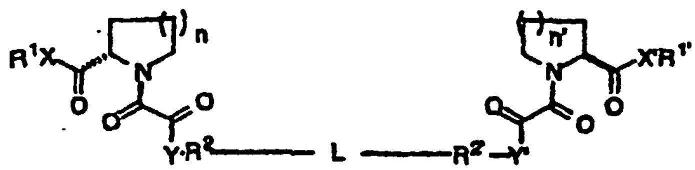

**5.** A multimerizing agent of claim 1 of the formula:

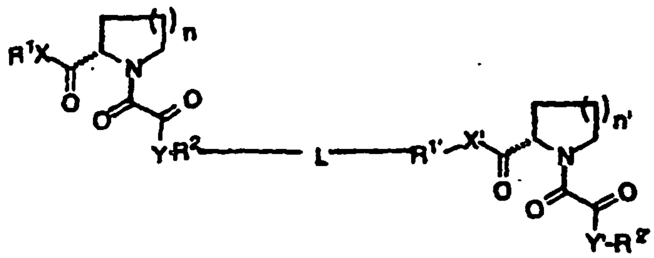

**6.** A multimerizing agent of any of claims 1 - 5 containing an $M^1$ or $M^2$ moiety in which each $-YR^2$ is a straight, branched or cyclic aliphatic moiety of 1 to 8 carbon atoms.

**7.** A multimerizing agent of claim 6 containing an $M^1$ or $M^2$ moiety in which $-YR^2$ is ethyl, isopropyl, tert-butyl, tert-amyl or cyclohexyl.

**8.** A multimerizing agent of any of claims 1 - 5 containing an $M^1$ or $M^2$ moiety in which $-YR^2$ comprises a stable cyclic, heterocyclic, polycyclic, or polyheterocyclic unsaturated $C_3$-$C_{14}$ moiety; which may further be substituted with one to five substituents selected from the group consisting of hydroxy, $C_1$-$C_8$ alkoxy, $C_1$-$C_8$ branched or straight-chain alkyl, acyloxy, carbamoyl, amino, N-acylamino, nitro, halogen, trifluoromethyl, cyano, and carboxyl.

**9.** A multimerizing agent of any of claims 1 - 5 containing an $M^1$ or $M^2$ moiety in which $-XR^1$ is a moiety of the formula

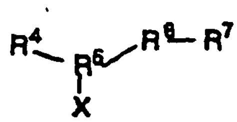

where $R^4$ is a phenyl, substituted phenyl or heteroaromatic group; $R^5$ is a straight, branched or cyclic aliphatic moiety of 2 to 8 carbon atoms, which may be optionally substituted; $R^6$ is an aromatic or heteroaromatic moiety bearing a reactive functional group, $R^7$, which may be -COOH, -CHO, -X'H or X'$R^8$, where X' is O,S or NH (substituted or unsubstituted) and $R^8$ is $-(CH_2)_z$-COOH where z is an integer from 1 through 4; and in which $-YR^2$ is a straight branched or cyclic aliphatic moiety of 1 to 8 carbon atoms.

**10.** A multimerizing agent of claim 9 containing an $M^1$ or $M^2$ moiety of the formula:

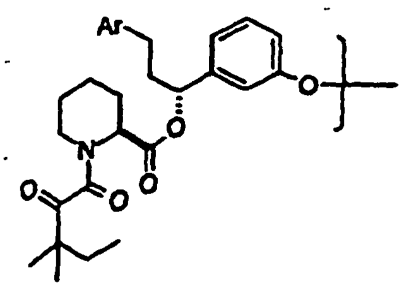

wherein Ar is phenyl, 3,4-dimethoxyphenyl, 3,4-($OCH_2O$)-phenyl, 3,4,5-trimethoxyphenyl, 3-pyridyl, or 3-indolyl.

**11.** A multimerising agent according to any one of claims 1 to 10 for use in therapy.

**12.** Use of a multimerising agent according to any one of claims 1 to 10 in the manufacture of a medicament for administration to an organism containing genetically engineered cells, wherein said cells contain FKBP-based recombinant proteins, in an amount effective to multimerize the recombinant proteins and activate a biological switch based on ligand-mediated multimerization of FKBP-based recombinant proteins to activate the transcription of a desired gene, actuate apoptosis, or trigger another biological event.

**13.** A method for activating a biological switch based on ligand-mediated multimerization of FKBP-based recombinant proteins to activate transcription of a desired gene, actuate apoptosis, or trigger another biological event in genetically engineered cells growing in culture, wherein said cells contain FKBP-based recombinant proteins, comprising administering to the cells a multimerising agent according to any one of claims 1 to 10 in an amount effective to multimerize the recombinant proteins.

**14.** A method for preparing a multimerizing agent of any of claims 1 to 10 which comprise covalently linking a compound of the formula M1 to a compound of the formal M2 through a linking moiety L.

**Patentansprüche**

**1.** Multimerisierendes Mittel der Formel $M^1$-L-$M^2$ und pharmazeutisch annehmbare Salze davon, in dem $M^1$ und $M^2$ Reste sind, die unabhängig voneinander ausgewählt sind aus dem Folgenden:

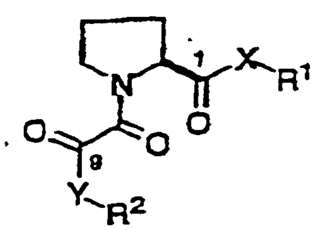

und

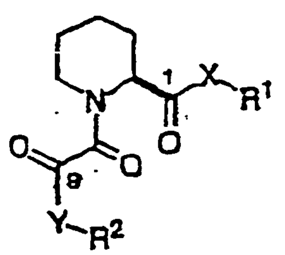

in denen

X = O, NH oder $CH_2$;

Y = O, NH, $NR^3$ oder eine kovalente Bindung von $R^2$ zu Atom 9 darstellt; und

in denen $R^1$, $R^2$ und $R^3$ unabhängig voneinander $C_1$-$C_{20}$ Alkyl oder Aryl sind;

wobei Alkyl gesättigte oder ungesättigte, geradkettige, verzweigte, cyclische oder polycyclische aliphatische Kohlenwasserstoffe aufweist, die anstatt eines oder mehrerer Kohlenstoffatome Sauerstoff, Schwefel oder Stickstoff enthalten können und die ggf. mit einer oder mehreren funktionellen Gruppen substituiert sind, die ausgewählt sind aus der Gruppe bestehend

aus Hydroxy, $C_1$-$C_8$ Alkoxy, Acyloxy, Carbamoyl, Amino, N-Acylamino, Keton, Halogen, Cyano, Carboxyl

und Aryl;

wobei Aryl stabile cyclische, heterocyclische, polycyclische oder polyheterocyclische ungesättigte $C_3$-$C_{14}$-Reste aufweist, die ferner mit ein bis fünf Gliedern substituiert sein können, die ausgewählt sind aus der Gruppe bestehend aus Hydroxy, $C_1$-$C_8$ Alkoxy, verzweigtem oder geradkettigem, $C_1$-$C_8$ Alkyl, Acyloxy, Carbamoyl, Amino, N-Acylamino, Nitro, Halogen, Trifluormethyl, Cyano und Carboxyl;

wobei L ein Bindeglied ist, das die $M^1$- und $M^2$-Monomere durch kovalente Bindungen entweder mit $R^1$ oder mit $R^2$ verbindet, was nicht notwendigerweise in jedem von $M^1$ und $M^2$ gleich ist; und wobei eines von $M^1$ und $M^2$ ggf. eine makrocyclische Struktur sein kann, die durch eine kovalente Verbindung zwischen $R^1$ und $R^2$ gebildet wird;

vorausgesetzt, dass das multimerisierende Mittel nicht die folgende Formel aufweist:

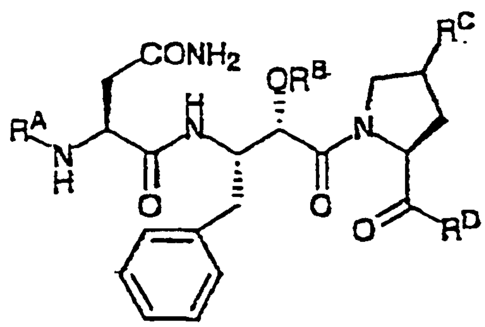

in der $R^A$ Carbobenzyloxy ist, in der $R^B$ und $R^C$ jeweils MeOCOCO-Pro-(D)Phe- sind und in der $R^D$ -NHC$(CH_3)_3$ ist.

2. Multimerisierendes Mittel nach Anspruch 1, in dem $M^1$ und $M^2$ Reste sind, die unabhängig voneinander ausgewählt sind aus dem Folgenden:

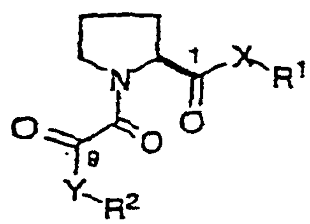

und

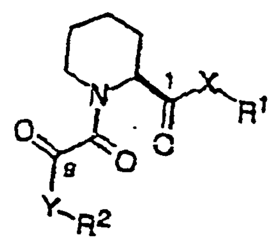

3. Multimerisierendes Mittel nach Anspruch 1 der Formel:

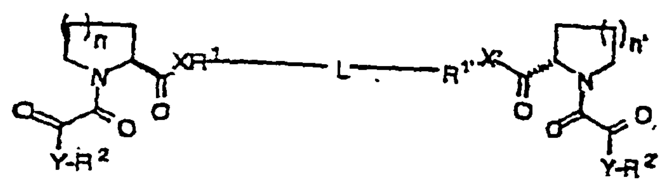

4. Multimerisierendes Mittel nach Anspruch 1 der Formel:

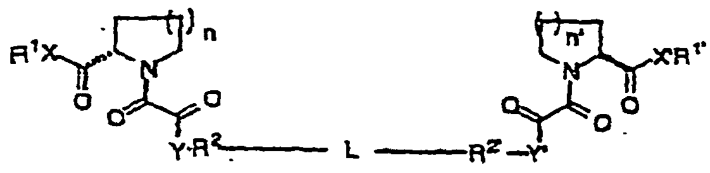

5. Multimerisierendes Mittel nach Anspruch 1 der Formel:

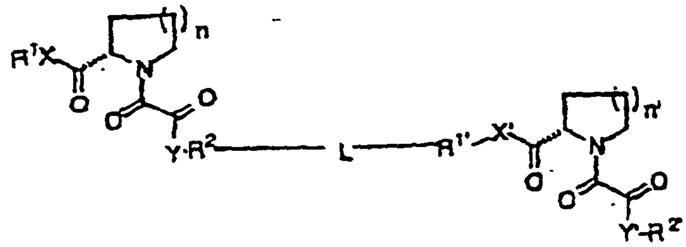

6. Multimerisierendes Mittel nach einem der Ansprüche 1 bis 5, das einen $M^1$- oder $M^2$-Rest aufweist, in dem jedes $-YR^2$ ein gerader, verzweigter oder cyclischer aliphatischer Rest mit 1 bis 8 Kohlenstoffatomen ist.

7. Multimerisierendes Mittel nach Anspruch 6, das einen $M^1$-oder $M^2$-Rest aufweist, in dem $-YR^2$ Ethyl, Isopropyl, tert-Butyl, tert-Amyl oder Cyclohexyl ist.

8. Multimerisierendes Mittel nach einem der Ansprüche 1 bis 5, das einen $M^1$- oder $M^2$-Rest aufweist, in dem $-YR^2$ einen stabilen cyclischen, heterocyclischen, polycyclischen oder polyheterocyclischen ungesättigten $C_3$-$C_{14}$-Rest aufweist, der ferner mit ein bis fünf Substituenten substituiert sein kann, die ausgewählt sind aus der Gruppe bestehend aus Hydroxy, $C_1$-$C_8$ Alkoxy, verzweigtem oder geradkettigem $C_1$-$C_8$ Alkyl, Acyloxy, Carbamoyl, Amino, N-Acylamino, Nitro, Halogen, Trifluormethyl, Cyano und Carboxyl.

9. Multimerisierendes Mittel nach einem der Ansprüche 1 bis 5, das einen $M^1$- oder $M^2$-Rest aufweist, in dem $-XR^1$ ein Rest der folgenden Formel ist

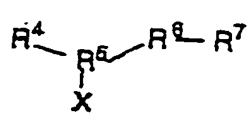

in der $R^4$ ein Phenyl, ein substituiertes Phenyl oder eine heteroaromatische Gruppe ist; in der $R^5$ ein gerader, verzweigter oder cyclischer aliphatischer Rest mit 2 bis 8 Kohlenstoffatomen ist, der ggf. substituiert sein kann; in der $R^6$ ein aromatischer oder heteroaromatischer Rest ist, der eine reaktive funktionelle Gruppe trägt, in der $R^7$ -COOH, -CHO, -X'H oder X'$R^8$ sein kann, wobei X' ein O, S oder NH (substituiert oder unsubstituiert) ist und wobei $R^8$ $-(CH_2)_z$-COOH ist, in dem z eine gerade Zahl von 1 bis 4 ist; und

in dem $-YR^2$ ein gerader, verzweigter oder cyclischer aliphatischer Rest mit 1 bis 8 Kohlenstoffatomen ist.

10. Multimerisierendes Mittel nach Anspruch 9, das einen $M^1$-oder $M^2$-Rest der folgenden Formel aufweist:

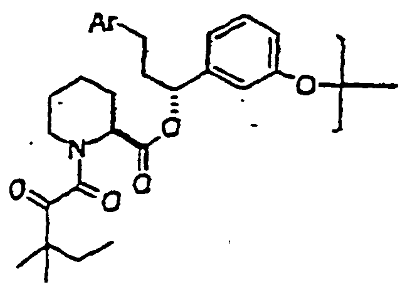

in der Ar ein Phenyl, 3,4-Dimethoxyphenyl, 3,4-($OCH_2O$)-Phenyl, 3,4,5-Trimethoxyphenyl, 3-Pyridyl oder 3-Indolyl ist.

**11.** Multimerisierendes Mittel nach einem der Ansprüche 1 bis 10 zur Verwendung in der Therapie.

**12.** Verwendung eines multimerisierenden Mittels nach einem der Ansprüche 1 bis 10 zur Herstellung eines Medikaments zur Verabreichung an einen Organismus, der genetisch manipulierte Zellen aufweist, wobei diese Zellen FKBP-basierte rekombinante Proteine enthalten, in einer Menge, die wirksam ist, die rekombinanten Proteine zu multimerisieren und einen biologischen Schalter zu aktivieren, der auf einer Liganden-vermittelten Multimerisation von FKBP-basierten rekombinanten Proteinen basiert, um die Transkription eines gewünschten Gens zu aktivieren, die Apoptose zu starten oder einen anderen biologischen Vorgang auszulösen.

**13.** Verfahren zum Aktivieren eines biologischen Schalters, der auf einer Liganden-vermittelten Multimerisation von FKBP-basierten rekombinanten Proteinen basiert, um in genetisch manipulierten Zellen, die in einer Kultur wachsen, die Transkription eines gewünschten Gens zu aktivieren, die Apoptose zu starten oder einen anderen biologischen Vorgang auszulösen, wobei diese Zellen FKBP-basierte rekombinante Proteine enthalten, das das Verabreichen eines multimerisierenden Mittels nach einem der Ansprüche 1 bis 10 in einer Menge aufweist, die wirksam ist, die rekombinanten Proteine zu multimerisieren.

**14.** Verfahren zum Herstellen eines multimerisierenden Mittels nach einem der Ansprüche 1 bis 10, das das kovalente Verbinden einer Verbindung der Formel $M^1$ mit einer Verbindung der Formel $M^2$ durch ein Bindeglied L aufweist.

## Revendications

**1.** Agent de multimérisation répondant à la formate $M^1$-L-$M^2$, et ses sels acceptables pharmaceutiquement dans laquelle $M^1$ et $M^2$ sont des radicaux choisis indépendamment parmi les suivants :

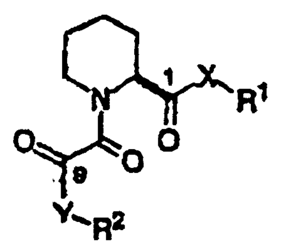

et

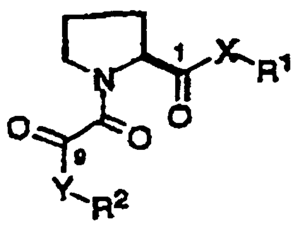

dans lesquels

X = O, NH ou $CH_2$ ;

Y = O, NH, $NR^3$ ou représente une liaison covalente allant de $R^2$ à l'atome 9 ; et ,

$R^1$, $R^2$ et $R^3$ sont indépendamment alcoyle ayant de 1 à 20 atomes de carbone ou aryle ;

dans laquelle alcoyle comprend des hydrocarbures aliphatiques, saturés ou insaturés, en chaîne linéaire, ramifiés, cycliques ou polycycliques, qui peuvent contenir de l'oxygène, du soufre ou de l'azote au lieu d'un ou de plusieurs atomes de carbone et qui sont substitués éventuellement par un ou par plusieurs groupes fonctionnels choisis dans le groupe consistant en hydroxy, alcoxy ayant de 1 à 8 atomes de carbone, acyloxy, carbamoyle, amino, N-acylamino, cétone, halogène, cyano, carboxyle et aryle ;

aryle comprend des radicaux ayant de 3 à 14 atomes de carbone insaturés, stables, cycliques, hétérocycliques ou polyhétérocycliques, qui peuvent, en outre, être substitués par de un à cinq éléments choisis dans le groupe consistant en hydroxy, alcoxy ayant de 1 à 8 atomes de carbone, alcoyle à chaîne ramifiée ou linéaire ayant de 1 à 8 atomes de carbone, carbamoyle, amino, N-acylamino, nitro, halogène, trifluorométhyle, cyano et carboxyle ;

L est un radical de pontage réunissant les monomères $M^1$ et $M^2$ par des liaisons de covalence à $R^1$ ou à $R^2$ qui n'est pas nécessairement le même dans chacun de $M^1$ et de $M^2$ ; et l'un de $M^1$ et de $M^2$ peut éventuellement être une structure macrocyclique formée par une liaison de covalence entre $R^1$ et $R^2$ ;

pourvu que l'agent de multimérisation n'ait pas la formule :

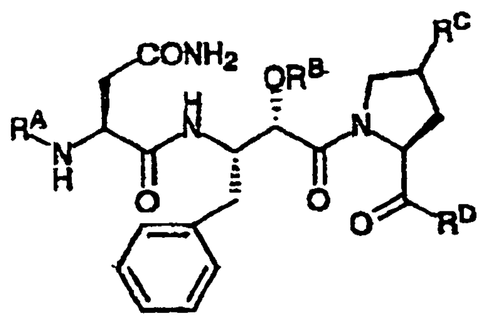

dans laquelle $R^4$ est carbobenzyloxy, $R^B$ et $R^C$ sont chacun MeOCOCO-Pro-(D)Phe-, et $R^D$ est $-NHC(CH_3)_3$.

2. Agent de multimérisation suivant la revendication 1, dans lequel $M^1$ et $M^2$ sont des radicaux choisis indépendamment parmi les suivants :

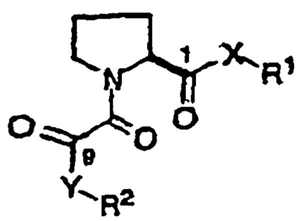

et

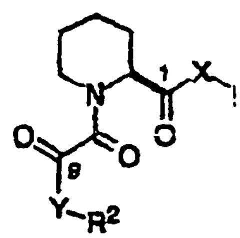

3. Agent de multimérisation suivant la revendication 1 de formule :

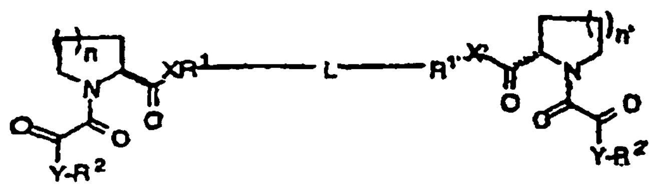

4. Agent de multimérisation suivant la revendication 1 de formule :

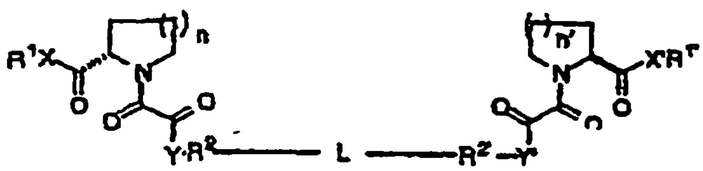

5. Agent de multimérisation suivant la revendication 1 de formule :

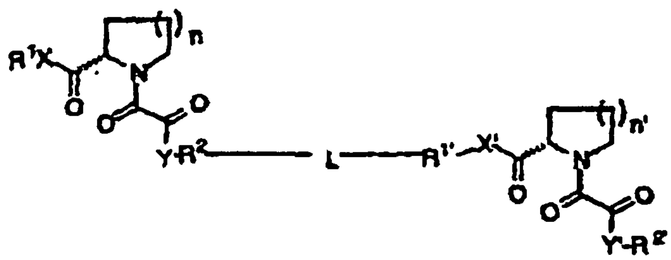

6. Agent de multimérisation suivant l'une quelconque des revendications 1 à 5 contenant un radical $M^1$ ou $M^2$ dans lequel chaque $-YR^2$ est un radical aliphatique, linéaire, ramifié ou cyclique ayant de 1 à 8 atomes de carbone.

7. Agent de multimérisation suivant la revendication 6 contenant un radical $M^1$ ou $M^2$ dans lequel $-YR^2$ est éthyle, isopropyle, tert-butyle, tert-amyle ou cyclohexyle.

8. Agent de multimérisation suivant l'une quelconque des revendications 1 à 5 contenant un radical $M^1$ ou $M^2$ dans lequel $-YR^2$ comprend un radical insaturé ayant de 3 à 14 atomes de carbone, stable, cyclique, hétérocyclique, polycyclique ou polyhétérocyclique ; qui peut être, en outre, substitué par de un à cinq substituants choisis dans le groupe consistant en hydroxy, alcoxy ayant de 1 à 8 atomes de carbone, alcoyle à chaîne ramifiée ou linéaire ayant de 1 à 8 atomes de carbone, carbamoyle, amino, N-acylamino, nitro, halogène, trifluorométhyle, cyano et carboxyle.,

9. Agent de multimérisation suivant l'une quelconque des revendications 1 à 5 contenant un radical $M^1$ ou $M^2$ dans lequel $-XR^1$ est un radical de formule :

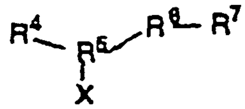

dans laquelle $R^4$ est un groupe phényle, phényle substitué ou hétéro-aromatique ; $R^5$ est un radical aliphatique, linéaire, ramifié ou cyclique ayant 2 à 8 atomes de carbone qui peut être éventuellement substitué ; $R^6$ est un radical aromatique ou hétéro-aromatique portant un groupe fonctionnel réactif qui peut être -COOH, -CHO, -X'H ou $X'R^8$, X' étant O, S ou NH (substitué ou non substitué) et $R^8$ est $-(CH_2)_z-COOH$, z étant un nombre entier allant

de 1 à 4 ; et
dans lequel -$YR^2$ est un radical aliphatique, linéaire, ramifié ou cyclique ayant de 1 à 8 atomes de carbone.

**10.** Agent de multimérisation contenant un radical $M^1$ ou $M^2$ de formule :

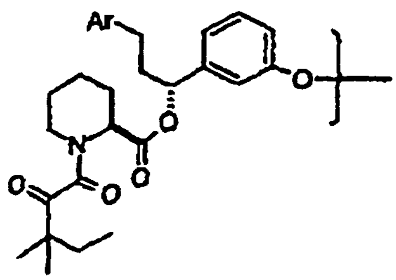

dans laquelle Ar est phényle, 3,4-diméthoxyphényle, 3,4-($OCH_2O$)-phényle, 3,4,5-triméthylphényl, 3-pyridyle ou 3-indolyle.

**11.** Agent de multimérisation suivant l'une quelconque des revendications 1 à 10, destiné à être utilisé en thérapeutique.

**12.** utilisation d'un agent de multimérisation suivant l'une quelconque des revendications 1 à 10 dans la fabrication d'un médicament destiné à être administré à un organisme contenant des cellules modifiées génétiquement, ces cellules contenant des protéines recombinantes à base de FKBP en une quantité efficace pour multimériser les protéines recombinantes et activer un commutateur biologique basé sur une multimérisation, à médiation par un ligand, de protéines recombinantes à base de FKBP pour activer la transcription d'un gène souhaité, effectuer une apoptose ou déclencher un autre événement biologique.

**13.** Procédé d'activation d'un commutateur biologique reposant sur une multimérisation, à médiation par un ligand, de protéines recombinantes à base de FKBP pour activer la transcription d'un gène souhaité, effectuer une apoptose ou déclencher un autre événement biologique dans des cellules modifiées génétiquement croissant dans une culture, les cellules contenant des protéines recombinantes à base de FKBP dans lequel on administre aux cellules un agent de multimérisation suivant l'une quelconque des revendications 1 à 10 en une quantité efficace pour multimériser les protéines recombinantes.

**14.** Procédé de préparation d'un agent de multimérisation suivant l'une quelconque des revendications 1 à 10, dans lequel on relie par covalence un composé de formule M1 à un composé de formule M2 par un radical L de liaison.